# EUROPEAN PATENT APPLICATION

(11) **EP 1 098 003 A2**
(43) Date of publication of application: **09.05.2001**
(21) Application number: 00106325.4
(22) Date of filing: 23.03.2000
(51) Int. Cl.: C12Q 1/68

(54) **Identification method and specific detection method of slow growing mycobacteria utilizing DNA gyrase gene**

(30) Priority: 02.11.1999 JP 31252599
(71) Applicant: MARINE BIOTECHNOLOGY INSTITUTE CO., LTD., Bunkyo-ku, Tokyo 113-0033 (JP)
(72) Inventor: Kasai, Hiroaki Co., Ltd c/o Marine Biotech.Inst.Co, Kamaishi-shi; Iwate 026-0001 (JP); Harayama, Shigeaki, Ltd c/o Marine Biotech.Inst.Co, Kamaishi-shi; Iwate 026-0001 (JP); Ezaki, Takayuki c/o Gifu University, Gifu 500-8705 (JP)
(74) Representative: VOSSIUS & PARTNER

(57) **Abstract**

A method for identification and detection of slow growing mycobacteria, especially tubercle bacilli group bacteria, utilizing characteristic nucleotide sequences which are present in the *gyrB* gene. It renders possible accurate identification and detection of slow growing mycobacteria which are difficult by the conventional methods.

SEQUENCE LISTING

<110> MARINE BIOTECHNOLOGY INSTITUTE CO., LTD.

<120> IDENTIFICATION METHOD AND SPECIFIC DETECTION METHOD OF
      SLOW GROWING MYCOBACTERIA UTILIZING DNA GYRASE GENE

<130> K4C-120036C/KI

<140>
<141>

<150> JP 11-312525
<151> 1999-11-02

<160> 62

<170> PatentIn Ver. 2.1

<210> 1
<211> 20
<212> DNA
<213> Artificial Sequence

<220>
<223> Description of Artificial Sequence: Synthetic DNA

<400> 1
ggtgtctcgg tggtcaacgc                                                20

<210> 2
<211> 6
<212> PRT
<213> Artificial Sequence

<220>
<223> Description of Artificial Sequence: Amino acid
      sequence corresponding to primer or probe

<400> 2
Gly Val Ser Val Val Asn

<210> 3
<211> 20
<212> DNA
<213> Artificial Sequence

<220>
<223> Description of Artificial Sequence: Synthetic DNA

<400> 3
gaccttgtgc ggggcggcgg                                                20

<210> 4
<211> 6
<212> PRT

<213> Artificial Sequence

<220>
<223> Description of Artificial Sequence: Amino acid
      sequence corresponding to primer or probe

<400> 4
Ala Ala Pro His Lys Val

<210> 5
<211> 20
<212> DNA
<213> Artificial Sequence

<220>
<223> Description of Artificial Sequence: Synthetic DNA

<400> 5
caccttgtgg ggggcggtga                                                20

<210> 6
<211> 6
<212> PRT
<213> Artificial Sequence

<220>
<223> Description of Artificial Sequence: Amino acid
      sequence corresponding to primer or probe

<400> 6
Thr Ala Pro His Lys Val

<210> 7
<211> 1263
<212> DNA
<213> Mycobacterium simiae

<220>
<221> CDS
<222> (1)..(1263)

<400> 1
ggg gag aac agt ggc tac acc gtc agc ggc ggg ttg cac ggg gtc gga    48
Gly Glu Asn Ser Gly Tyr Thr Val Ser Gly Gly Leu His Gly Val Gly
  1               5                  10                  15

gtg tcg gtg gtc aac gcc ctg tcc acc cgc ctg gaa gtc aac gtc aag    96
Val Ser Val Val Asn Ala Leu Ser Thr Arg Leu Glu Val Asn Val Lys
             20                  25                  30

cgt gac ggc tat gag tgg ttc cag tac tac gac cgg gcg gtg ccc ggc    144
Arg Asp Gly Tyr Glu Trp Phe Gln Tyr Tyr Asp Arg Ala Val Pro Gly
         35                  40                  45

acc ctc aag caa ggc gag gcg acc aag aag acc ggc acc acg atc cgg    192
Thr Leu Lys Gln Gly Glu Ala Thr Lys Lys Thr Gly Thr Thr Ile Arg

    50                  55                  60

ttc tgg gcc gat cct gag atc ttc gaa acc acc cag tac gac ttc gag 240
Phe Trp Ala Asp Pro Glu Ile Phe Glu Thr Thr Gln Tyr Asp Phe Glu
65                  70                  75                  80

acg gtg gcg cgc cgg ttg cag gaa atg gcg ttc ctc aac aag ggc ctg 288
Thr Val Ala Arg Arg Leu Gln Glu Met Ala Phe Leu Asn Lys Gly Leu
                85                  90                  95

acc atc aac ctc acc gac gaa cgt gtc gag cag gac gag gtg gtc gat 336
Thr Ile Asn Leu Thr Asp Glu Arg Val Glu Gln Asp Glu Val Val Asp
            100                 105                 110

gag gtg gtt agc gac acc gcc gag gcg ccg aag tca gcc gag gag cag 384
Glu Val Val Ser Asp Thr Ala Glu Ala Pro Lys Ser Ala Glu Glu Gln
        115                 120                 125

gcg gcc gaa tcg gcc aag ccg cac aag gtc aag cac cgc acg ttc cac 432
Ala Ala Glu Ser Ala Lys Pro His Lys Val Lys His Arg Thr Phe His
    130                 135                 140

tac ccg ggt ggg ttg gtg gat ttc gtc aag cac atc aat cgc acc aaa 480
Tyr Pro Gly Gly Leu Val Asp Phe Val Lys His Ile Asn Arg Thr Lys
145                 150                 155                 160

aac ccg atc cag cag agc gtc atc gac ttc gac ggc aaa gga acc ggg 528
Asn Pro Ile Gln Gln Ser Val Ile Asp Phe Asp Gly Lys Gly Thr Gly
                165                 170                 175

cac gaa gtc gag atc gcg atg cag tgg aac ggt ggt tat tcg gag tcg 576
His Glu Val Glu Ile Ala Met Gln Trp Asn Gly Gly Tyr Ser Glu Ser
            180                 185                 190

gtg cac acc ttc gcc aac acc atc aac acc cat gag ggc ggc acc cac 624
Val His Thr Phe Ala Asn Thr Ile Asn Thr His Glu Gly Gly Thr His
        195                 200                 205

gag gag ggc ttc cgc agc gcg ctg acc tcg gtg gtg aac aag tac gcc 672
Glu Glu Gly Phe Arg Ser Ala Leu Thr Ser Val Val Asn Lys Tyr Ala
    210                 215                 220

aaa gac aag aag ctg ctc aag gac aag gat ccc aac ctc acc ggc gac 720
Lys Asp Lys Lys Leu Leu Lys Asp Lys Asp Pro Asn Leu Thr Gly Asp
225                 230                 235                 240

gac atc cga gaa ggg ctg gcc gcg gtg atc tcc gtg aag gtc gcc gag 768
Asp Ile Arg Glu Gly Leu Ala Ala Val Ile Ser Val Lys Val Ala Glu
                245                 250                 255

ccg cag ttc gag ggc cag act aag acg aaa ctc ggc aac acc gag gtc 816
Pro Gln Phe Glu Gly Gln Thr Lys Thr Lys Leu Gly Asn Thr Glu Val
            260                 265                 270

aag tcg ttt gtc cag aaa gtc tgt aac gaa caa ctc act cac tgg ttc 864
Lys Ser Phe Val Gln Lys Val Cys Asn Glu Gln Leu Thr His Trp Phe

275                 280                 285

gag gcg aac ccg tcg gaa gct aaa acc gtt gta aac aag gcg gtt tcg     912
Glu Ala Asn Pro Ser Glu Ala Lys Thr Val Val Asn Lys Ala Val Ser
    290                 295                 300

tcg gcc cag gcc cgc att gcg gcg cgt aag gcg cgg gag ttg gtg cgg     960
Ser Ala Gln Ala Arg Ile Ala Ala Arg Lys Ala Arg Glu Leu Val Arg
305                 310                 315                 320

cgt aag agt gct acg gat ttg ggt ggg ttg ccg ggc aag ttg gct gat     1008
Arg Lys Ser Ala Thr Asp Leu Gly Gly Leu Pro Gly Lys Leu Ala Asp
                325                 330                 335

tgc cgc tcg acg gat ccg cgg aag tct gag ctg tat gtg gtg gaa ggt     1056
Cys Arg Ser Thr Asp Pro Arg Lys Ser Glu Leu Tyr Val Val Glu Gly
            340                 345                 350

gat tcc gcg ggt ggg tcg gcg aaa agt ggg cgt gat tcg atg ttc cag     1104
Asp Ser Ala Gly Gly Ser Ala Lys Ser Gly Arg Asp Ser Met Phe Gln
        355                 360                 365

gcg atc ttg ccg ctg cgc ggc aag atc atc aac gtc gaa aag gcc cgc     1152
Ala Ile Leu Pro Leu Arg Gly Lys Ile Ile Asn Val Glu Lys Ala Arg
    370                 375                 380

atc gat cgg gtg ctg aaa aac acc gaa gtc cag gcc atc atc acc gcg     1200
Ile Asp Arg Val Leu Lys Asn Thr Glu Val Gln Ala Ile Ile Thr Ala
385                 390                 395                 400

ctg ggc acc ggc atc cac gac gaa ttc gac atc acc aaa ctg cgt tac     1248
Leu Gly Thr Gly Ile His Asp Glu Phe Asp Ile Thr Lys Leu Arg Tyr
                405                 410                 415

cac aag atc gtg ttg                                                 1263
His Lys Ile Val Leu
            420

<210> 8
<211> 421
<212> PRT
<213> Mycobacterium simiae

<400> 8
Gly Glu Asn Ser Gly Tyr Thr Val Ser Gly Gly Leu His Gly Val Gly
1               5                   10                  15

Val Ser Val Val Asn Ala Leu Ser Thr Arg Leu Glu Val Asn Val Lys
            20                  25                  30

Arg Asp Gly Tyr Glu Trp Phe Gln Tyr Tyr Asp Arg Ala Val Pro Gly
        35                  40                  45

Thr Leu Lys Gln Gly Glu Ala Thr Lys Lys Thr Gly Thr Thr Ile Arg
    50                  55                  60

Phe Trp Ala Asp Pro Glu Ile Phe Glu Thr Thr Gln Tyr Asp Phe Glu
65                  70                  75                  80

Thr Val Ala Arg Arg Leu Gln Glu Met Ala Phe Leu Asn Lys Gly Leu
                85                  90                  95

Thr Ile Asn Leu Thr Asp Glu Arg Val Glu Gln Asp Glu Val Val Asp
            100                 105                 110

Glu Val Val Ser Asp Thr Ala Glu Ala Pro Lys Ser Ala Glu Glu Gln
        115                 120                 125

Ala Ala Glu Ser Ala Lys Pro His Lys Val Lys His Arg Thr Phe His
    130                 135                 140

Tyr Pro Gly Gly Leu Val Asp Phe Val Lys His Ile Asn Arg Thr Lys
145                 150                 155                 160

Asn Pro Ile Gln Gln Ser Val Ile Asp Phe Asp Gly Lys Gly Thr Gly
                165                 170                 175

His Glu Val Glu Ile Ala Met Gln Trp Asn Gly Gly Tyr Ser Glu Ser
            180                 185                 190

Val His Thr Phe Ala Asn Thr Ile Asn Thr His Glu Gly Gly Thr His
        195                 200                 205

Glu Glu Gly Phe Arg Ser Ala Leu Thr Ser Val Val Asn Lys Tyr Ala
    210                 215                 220

Lys Asp Lys Lys Leu Leu Lys Asp Lys Asp Pro Asn Leu Thr Gly Asp
225                 230                 235                 240

Asp Ile Arg Glu Gly Leu Ala Ala Val Ile Ser Val Lys Val Ala Glu
                245                 250                 255

Pro Gln Phe Glu Gly Gln Thr Lys Thr Lys Leu Gly Asn Thr Glu Val
            260                 265                 270

Lys Ser Phe Val Gln Lys Val Cys Asn Glu Gln Leu Thr His Trp Phe
        275                 280                 285

Glu Ala Asn Pro Ser Glu Ala Lys Thr Val Val Asn Lys Ala Val Ser
    290                 295                 300

Ser Ala Gln Ala Arg Ile Ala Ala Arg Lys Ala Arg Glu Leu Val Arg
305                 310                 315                 320

Arg Lys Ser Ala Thr Asp Leu Gly Gly Leu Pro Gly Lys Leu Ala Asp
                325                 330                 335

Cys Arg Ser Thr Asp Pro Arg Lys Ser Glu Leu Tyr Val Val Glu Gly
            340                 345                 350

Asp Ser Ala Gly Gly Ser Ala Lys Ser Gly Arg Asp Ser Met Phe Gln

355                 360                 365

Ala Ile Leu Pro Leu Arg Gly Lys Ile Ile Asn Val Glu Lys Ala Arg
    370                 375                 380

Ile Asp Arg Val Leu Lys Asn Thr Glu Val Gln Ala Ile Ile Thr Ala
385                 390                 395                 400

Leu Gly Thr Gly Ile His Asp Glu Phe Asp Ile Thr Lys Leu Arg Tyr
                405                 410                 415

His Lys Ile Val Leu
            420


<210> 9
<211> 1257
<212> DNA
<213> Mycobacterium bovis

<220>
<221> CDS
<222> (1)..(1257)

<400> 9
   tcg gac gcg tat gcg ata tct ggt ggt ctg cac ggc gtc ggc gtg tcg      48
   Ser Asp Ala Tyr Ala Ile Ser Gly Gly Leu His Gly Val Gly Val Ser
   1               5                   10                  15
   gtg gtt aac gcg cta tcc acc cgg ctc gaa gtc gag atc aag cgc gac      96
   Val Val Asn Ala Leu Ser Thr Arg Leu Glu Val Glu Ile Lys Arg Asp
               20                  25                  30
   ggg tac gag tgg tct cag gtt tat gag aag tcg gaa ccc ctg ggc ctc      144
   Gly Tyr Glu Trp Ser Gln Val Tyr Glu Lys Ser Glu Pro Leu Gly Leu
           35                  40                  45
   aag caa ggg gcg ccg acc aag aag acg ggg tca acg gta cgg ttc tgg      192
   Lys Gln Gly Ala Pro Thr Lys Lys Thr Gly Ser Thr Val Arg Phe Trp
       50                  55                  60
   gcc gac ccc gct gtt ttc gaa acc acg gaa tac gac ttc gaa acc gtc      240
   Ala Asp Pro Ala Val Phe Glu Thr Thr Glu Tyr Asp Phe Glu Thr Val
   65                  70                  75                  80
   gcc cgc cgg ctg caa gag atg gcg ttc ctc aac aag ggg ctg acc atc      288
   Ala Arg Arg Leu Gln Glu Met Ala Phe Leu Asn Lys Gly Leu Thr Ile
                   85                  90                  95
   aac ctg acc gac gag agg gtg acc caa gac gag gtc gtc gac gaa gtg      336
   Asn Leu Thr Asp Glu Arg Val Thr Gln Asp Glu Val Val Asp Glu Val
               100                 105                 110
   gtc agc gac gtc gcc gag gcg ccg aag tcg gca agt gaa cgc gca gcc      384
   Val Ser Asp Val Ala Glu Ala Pro Lys Ser Ala Ser Glu Arg Ala Ala
           115                 120                 125
   gaa tcc act gca ccg cac aaa gtt aag agc cgc acc ttt cac tat ccg     432
   Glu Ser Thr Ala Pro His Lys Val Lys Ser Arg Thr Phe His Tyr Pro
       130                 135                 140
   ggt ggc ctg gtg gac ttc gtg aaa cac atc aac cgc acc aag aac gcg     480
   Gly Gly Leu Val Asp Phe Val Lys His Ile Asn Arg Thr Lys Asn Ala
   145                 150                 155                 160
   att cat agc agc atc gtg gac ttt tcc ggc aag ggc acc ggg cac gag     528
   Ile His Ser Ser Ile Val Asp Phe Ser Gly Lys Gly Thr Gly His Glu
                   165                 170                 175
   gtg gag atc gcg atg caa tgg aac gcc ggg tat tcg gag tcg gtg cac     576
   Val Glu Ile Ala Met Gln Trp Asn Ala Gly Tyr Ser Glu Ser Val His
               180                 185                 190
   acc ttc gcc aac acc atc aac acc cac gag ggc ggc acc cac gaa gag     624
   Thr Phe Ala Asn Thr Ile Asn Thr His Glu Gly Gly Thr His Glu Glu
           195                 200                 205
   ggc ttc cgc agc gcg ctg acg tcg gtg gtg aac aag tac gcc aag gac     672
   Gly Phe Arg Ser Ala Leu Thr Ser Val Val Asn Lys Tyr Ala Lys Asp
       210                 215                 220
   cgc aag cta ctg aag gac aag gac ccc aac ctc acc ggt gac gat atc     720
   Arg Lys Leu Leu Lys Asp Lys Asp Pro Asn Leu Thr Gly Asp Asp Ile
   225                 230                 235                 240
   cgg gaa ggc ctg gcc gct gtg atc tcg gtg aag gtc agc gaa ccg cag     768
   Arg Glu Gly Leu Ala Ala Val Ile Ser Val Lys Val Ser Glu Pro Gln
                   245                 250                 255
   ttc gag ggc cag acc aag acc aag ttg ggc aac acc gag gtc aaa tcg     816
   Phe Glu Gly Gln Thr Lys Thr Lys Leu Gly Asn Thr Glu Val Lys Ser
               260                 265                 270
   ttt gtg cag aag gtc tgt aat gaa cag ctg acc cac tgg ttt gaa gcc     864
   Phe Val Gln Lys Val Cys Asn Glu Gln Leu Thr His Trp Phe Glu Ala
           275                 280                 285
   aac ccc acc gac tcg aaa gtc gtt gtg aac aag gct gtg tcc tcg gcg     912
   Asn Pro Thr Asp Ser Lys Val Val Val Asn Lys Ala Val Ser Ser Ala
       290                 295                 300
   caa gcc cgt atc gcg gca cgt aag gca cga gag ttg gtg cgg cgt aag    960
   Gln Ala Arg Ile Ala Ala Arg Lys Ala Arg Glu Leu Val Arg Arg Lys
   305                 310                 315                 320
   agc gcc acc gac atc ggt gga ttg ccc ggc aag ctg gcc gat tgc cgt    1008
   Ser Ala Thr Asp Ile Gly Gly Leu Pro Gly Lys Leu Ala Asp Cys Arg
                   325                 330                 335
   tcc acg gat ccg cgc aag tcc gaa ctg tat gtc gta gaa ggt gac tcg     1056
   Ser Thr Asp Pro Arg Lys Ser Glu Leu Tyr Val Val Glu Gly Asp Ser
               340                 345                 350
   gcc ggc ggt tct gca aaa agc ggt cgc gat tcg atg ttc cag gcg ata     1104
   Ala Gly Gly Ser Ala Lys Ser Gly Arg Asp Ser Met Phe Gln Ala Ile
           355                 360                 365
   ctt ccg ctg cgc ggc aag atc atc aat gtg gag aaa gcg cgc atc gac     1152
   Leu Pro Leu Arg Gly Lys Ile Ile Asn Val Glu Lys Ala Arg Ile Asp
       370                 375                 380
   cgg gtg cta aag aac acc gaa gtt cag gcg atc atc acg gcg ctg ggc     1200
   Arg Val Leu Lys Asn Thr Glu Val Gln Ala Ile Ile Thr Ala Leu Gly
   385                 390                 395                 400
   acc ggg atc cac gac gag ttc gat atc ggc aag ctg cgc tac cac aag     1248
   Thr Gly Ile His Asp Glu Phe Asp Ile Gly Lys Leu Arg Tyr His Lys
                   405                 410                 415
   atc gtg ctg                                                         1257
   Ile Val Leu


<210> 10
<211> 419
<212> PRT
<213> Mycobacterium bovis

<400> 10
Ser Asp Ala Tyr Ala Ile Ser Gly Gly Leu His Gly Val Gly Val Ser
1               5                   10                  15

Val Val Asn Ala Leu Ser Thr Arg Leu Glu Val Glu Ile Lys Arg Asp
            20                  25                  30

Gly Tyr Glu Trp Ser Gln Val Tyr Glu Lys Ser Glu Pro Leu Gly Leu
        35                  40                  45

Lys Gln Gly Ala Pro Thr Lys Lys Thr Gly Ser Thr Val Arg Phe Trp
    50                  55                  60

Ala Asp Pro Ala Val Phe Glu Thr Thr Glu Tyr Asp Phe Glu Thr Val
65                  70                  75                  80

Ala Arg Arg Leu Gln Glu Met Ala Phe Leu Asn Lys Gly Leu Thr Ile
                85                  90                  95

Asn Leu Thr Asp Glu Arg Val Thr Gln Asp Glu Val Val Asp Glu Val
            100                 105                 110

Val Ser Asp Val Ala Glu Ala Pro Lys Ser Ala Ser Glu Arg Ala Ala
        115                 120                 125

Glu Ser Thr Ala Pro His Lys Val Lys Ser Arg Thr Phe His Tyr Pro
    130                 135                 140

Gly Gly Leu Val Asp Phe Val Lys His Ile Asn Arg Thr Lys Asn Ala
145                 150                 155                 160

Ile His Ser Ser Ile Val Asp Phe Ser Gly Lys Gly Thr Gly His Glu
                165                 170                 175

Val Glu Ile Ala Met Gln Trp Asn Ala Gly Tyr Ser Glu Ser Val His
            180                 185                 190

Thr Phe Ala Asn Thr Ile Asn Thr His Glu Gly Gly Thr His Glu Glu
        195                 200                 205

Gly Phe Arg Ser Ala Leu Thr Ser Val Val Asn Lys Tyr Ala Lys Asp
    210                 215                 220

Arg Lys Leu Leu Lys Asp Lys Asp Pro Asn Leu Thr Gly Asp Asp Ile
225                 230                 235                 240

Arg Glu Gly Leu Ala Ala Val Ile Ser Val Lys Val Ser Glu Pro Gln
                245                 250                 255

Phe Glu Gly Gln Thr Lys Thr Lys Leu Gly Asn Thr Glu Val Lys Ser
            260                 265                 270

Phe Val Gln Lys Val Cys Asn Glu Gln Leu Thr His Trp Phe Glu Ala
        275                 280                 285

Asn Pro Thr Asp Ser Lys Val Val Val Asn Lys Ala Val Ser Ser Ala
    290                 295                 300

Gln Ala Arg Ile Ala Ala Arg Lys Ala Arg Glu Leu Val Arg Arg Lys
305                 310                 315                 320

Ser Ala Thr Asp Ile Gly Gly Leu Pro Gly Lys Leu Ala Asp Cys Arg
                325                 330                 335

Ser Thr Asp Pro Arg Lys Ser Glu Leu Tyr Val Val Glu Gly Asp Ser
            340                 345                 350

Ala Gly Gly Ser Ala Lys Ser Gly Arg Asp Ser Met Phe Gln Ala Ile
        355                 360                 365

Leu Pro Leu Arg Gly Lys Ile Ile Asn Val Glu Lys Ala Arg Ile Asp
    370                 375                 380

Arg Val Leu Lys Asn Thr Glu Val Gln Ala Ile Ile Thr Ala Leu Gly
385                 390                 395                 400

Thr Gly Ile His Asp Glu Phe Asp Ile Gly Lys Leu Arg Tyr His Lys
                405                 410                 415

Ile Val Leu

<210> 11
<211> 1263
<212> DNA

<213> Mycobacterium szulgai

<220>
<221> CDS
<222> (1)..(1263)

<400> 11
ggc gag aac agt ggc tac aac gtc agt ggt ggt ctg cac ggc gtc ggg     48
Gly Glu Asn Ser Gly Tyr Asn Val Ser Gly Gly Leu His Gly Val Gly
1               5                   10                  15

gtg tcg gtg gtg aac gcg ctg tcg acc cgg ctc gag gtc gac atc aag     96
Val Ser Val Val Asn Ala Leu Ser Thr Arg Leu Glu Val Asp Ile Lys
            20                  25                  30

cgt gac ggc cac aag tgg tcg cag ttc tac aac aag gcc gtg ccg ggc     144
Arg Asp Gly His Lys Trp Ser Gln Phe Tyr Asn Lys Ala Val Pro Gly
        35                  40                  45

acg ctc aaa cag ggt gaa gcc act aag aaa acc gga acg aca att agg     192
Thr Leu Lys Gln Gly Glu Ala Thr Lys Lys Thr Gly Thr Thr Ile Arg
    50                  55                  60

ttc tgg gcc gac ccg gac atc ttc gag acc acc gaa tac gac ttc gag     240
Phe Trp Ala Asp Pro Asp Ile Phe Glu Thr Thr Glu Tyr Asp Phe Glu
65                  70                  75                  80

acc gtg gca cgc cgg ctg cag gaa atg gca ttc ctg aac aag ggc ttg     288
Thr Val Ala Arg Arg Leu Gln Glu Met Ala Phe Leu Asn Lys Gly Leu
                85                  90                  95

acc atc aac ctc acc gac gag cga gtt gcc cag gac gag gtt gtc gac     336
Thr Ile Asn Leu Thr Asp Glu Arg Val Ala Gln Asp Glu Val Val Asp
            100                 105                 110

gag gtc gtc agc gac acc gcc gag gca ccc aag tcc gcc gaa gaa aag     384
Glu Val Val Ser Asp Thr Ala Glu Ala Pro Lys Ser Ala Glu Glu Lys
        115                 120                 125

gcg gcc gaa tcc aaa ggg ccg cat aag gtt aag cac cgc act ttc cat     432
Ala Ala Glu Ser Lys Gly Pro His Lys Val Lys His Arg Thr Phe His
    130                 135                 140

tac ccc ggc ggg ctg atc gac ttc gtc aag cac atc aac cgg acc aag     480
Tyr Pro Gly Gly Leu Ile Asp Phe Val Lys His Ile Asn Arg Thr Lys
145                 150                 155                 160

agc ccg atc cag cag agt gtc gtc gcc ttc gac ggc aag ggt gaa ggg     528
Ser Pro Ile Gln Gln Ser Val Val Ala Phe Asp Gly Lys Gly Glu Gly
                165                 170                 175

cac gag gtc gag atc gcg atg cag tgg aac ggc ggc tat tcg gag tcg     576
His Glu Val Glu Ile Ala Met Gln Trp Asn Gly Gly Tyr Ser Glu Ser
            180                 185                 190

gtg cac acc ttc gcc aac acc atc aac acc cac gag ggc ggc acc cac     624

Val His Thr Phe Ala Asn Thr Ile Asn Thr His Glu Gly Gly Thr His
        195                 200                 205

gaa gaa ggg ttc cgc agc gca ctg aca tcg gtg gtg aac aag tac gcc     672
Glu Glu Gly Phe Arg Ser Ala Leu Thr Ser Val Val Asn Lys Tyr Ala
    210                 215                 220

aaa gac aag aag ctg ctc aag gag aag gac gcc aac ctc acc ggc gac     720
Lys Asp Lys Lys Leu Leu Lys Glu Lys Asp Ala Asn Leu Thr Gly Asp
225                 230                 235                 240

gac att cgc gag ggc ctg gcc gcg gtc atc tcg gtg aaa gtt gcc gaa     768
Asp Ile Arg Glu Gly Leu Ala Ala Val Ile Ser Val Lys Val Ala Glu
                245                 250                 255

ccg cag ttc gag ggc cag acc aag acc aaa ctg ggt aac acc gag gtc     816
Pro Gln Phe Glu Gly Gln Thr Lys Thr Lys Leu Gly Asn Thr Glu Val
            260                 265                 270

aag tcg ttc gta cag aag gtc tgc aac gaa cag ctg acc cac tgg ttc     864
Lys Ser Phe Val Gln Lys Val Cys Asn Glu Gln Leu Thr His Trp Phe
        275                 280                 285

gag gcc aac ccg tcg gaa gcc aaa acc gtc gtg aac aag gcg gtc tcg     912
Glu Ala Asn Pro Ser Glu Ala Lys Thr Val Val Asn Lys Ala Val Ser
    290                 295                 300

tcg gca cag gcg cgt atc gcc gcc cgc aag gca cga gag ttg gtg cgt     960
Ser Ala Gln Ala Arg Ile Ala Ala Arg Lys Ala Arg Glu Leu Val Arg
305                 310                 315                 320

cgc aag agc gct acc gat ctc ggt ggg ctg ccc ggc aag ctg gcc gac     1008
Arg Lys Ser Ala Thr Asp Leu Gly Gly Leu Pro Gly Lys Leu Ala Asp
                325                 330                 335

tgc cgc tcc acc gat ccg cgc aag tcg gaa ttg tat gtg gtg gaa ggg     1056
Cys Arg Ser Thr Asp Pro Arg Lys Ser Glu Leu Tyr Val Val Glu Gly
            340                 345                 350

gac tcg gcc ggc ggc tcc gcc aag agc ggc cgc gac tcg atg ttt cag     1104
Asp Ser Ala Gly Gly Ser Ala Lys Ser Gly Arg Asp Ser Met Phe Gln
        355                 360                 365

gcg ata ctt ccg ttg cgc ggc aag atc atc aac gtc gag aag gcc cgc     1152
Ala Ile Leu Pro Leu Arg Gly Lys Ile Ile Asn Val Glu Lys Ala Arg
    370                 375                 380

atc gac cgg gtg ctg aag aac acc gaa gtc cag gcg atc atc acc gcg     1200
Ile Asp Arg Val Leu Lys Asn Thr Glu Val Gln Ala Ile Ile Thr Ala
385                 390                 395                 400

ctg ggt acc gga att cac gac gag ttc gac ctc gcc aaa ctg cgc tac     1248
Leu Gly Thr Gly Ile His Asp Glu Phe Asp Leu Ala Lys Leu Arg Tyr
                405                 410                 415

cac aag atc gtg ctg                                                 1263

His Lys Ile Val Leu
            420

<210> 12
<211> 421
<212> PRT
<213> Mycobacterium szulgai

<400> 12
Gly Glu Asn Ser Gly Tyr Asn Val Ser Gly Gly Leu His Gly Val Gly
 1               5                  10                  15

Val Ser Val Val Asn Ala Leu Ser Thr Arg Leu Glu Val Asp Ile Lys
            20                  25                  30

Arg Asp Gly His Lys Trp Ser Gln Phe Tyr Asn Lys Ala Val Pro Gly
        35                  40                  45

Thr Leu Lys Gln Gly Glu Ala Thr Lys Lys Thr Gly Thr Thr Ile Arg
    50                  55                  60

Phe Trp Ala Asp Pro Asp Ile Phe Glu Thr Thr Glu Tyr Asp Phe Glu
65                  70                  75                  80

Thr Val Ala Arg Arg Leu Gln Glu Met Ala Phe Leu Asn Lys Gly Leu
                85                  90                  95

Thr Ile Asn Leu Thr Asp Glu Arg Val Ala Gln Asp Glu Val Val Asp
            100                 105                 110

Glu Val Val Ser Asp Thr Ala Glu Ala Pro Lys Ser Ala Glu Glu Lys
        115                 120                 125

Ala Ala Glu Ser Lys Gly Pro His Lys Val Lys His Arg Thr Phe His
    130                 135                 140

Tyr Pro Gly Gly Leu Ile Asp Phe Val Lys His Ile Asn Arg Thr Lys
145                 150                 155                 160

Ser Pro Ile Gln Gln Ser Val Val Ala Phe Asp Gly Lys Gly Glu Gly
                165                 170                 175

His Glu Val Glu Ile Ala Met Gln Trp Asn Gly Gly Tyr Ser Glu Ser
            180                 185                 190

Val His Thr Phe Ala Asn Thr Ile Asn Thr His Glu Gly Gly Thr His
        195                 200                 205

Glu Glu Gly Phe Arg Ser Ala Leu Thr Ser Val Val Asn Lys Tyr Ala
    210                 215                 220

Lys Asp Lys Lys Leu Leu Lys Glu Lys Asp Ala Asn Leu Thr Gly Asp
225                 230                 235                 240

Asp Ile Arg Glu Gly Leu Ala Ala Val Ile Ser Val Lys Val Ala Glu

245                 250                 255

Pro Gln Phe Glu Gly Gln Thr Lys Thr Lys Leu Gly Asn Thr Glu Val
            260                 265                 270

Lys Ser Phe Val Gln Lys Val Cys Asn Glu Gln Leu Thr His Trp Phe
        275                 280                 285

Glu Ala Asn Pro Ser Glu Ala Lys Thr Val Val Asn Lys Ala Val Ser
    290                 295                 300

Ser Ala Gln Ala Arg Ile Ala Ala Arg Lys Ala Arg Glu Leu Val Arg
305                 310                 315                 320

Arg Lys Ser Ala Thr Asp Leu Gly Gly Leu Pro Gly Lys Leu Ala Asp
                325                 330                 335

Cys Arg Ser Thr Asp Pro Arg Lys Ser Glu Leu Tyr Val Val Glu Gly
            340                 345                 350

Asp Ser Ala Gly Gly Ser Ala Lys Ser Gly Arg Asp Ser Met Phe Gln
        355                 360                 365

Ala Ile Leu Pro Leu Arg Gly Lys Ile Ile Asn Val Glu Lys Ala Arg
    370                 375                 380

Ile Asp Arg Val Leu Lys Asn Thr Glu Val Gln Ala Ile Ile Thr Ala
385                 390                 395                 400

Leu Gly Thr Gly Ile His Asp Glu Phe Asp Leu Ala Lys Leu Arg Tyr
                405                 410                 415

His Lys Ile Val Leu
            420

<210> 13
<211> 1263
<212> DNA
<213> Mycobacterium malmoense

<220>
<221> CDS
<222> (1)..(1263)

<400> 13
ggc gag aac agc gga tac aac gtc agt ggc ggt ttg cac ggt gtc ggc     48
Gly Glu Asn Ser Gly Tyr Asn Val Ser Gly Gly Leu His Gly Val Gly
1               5                   10                  15

gtg tcg gtg gtc aac gcg ttg tcg acc cgg ctc gag gtg gat gtc gcc     96
Val Ser Val Val Asn Ala Leu Ser Thr Arg Leu Glu Val Asp Val Ala
            20                  25                  30

cgc gac ggc tac atg tgg tca cag ttc tac gat cac gcc gag ccg gga     144
Arg Asp Gly Tyr Met Trp Ser Gln Phe Tyr Asp His Ala Glu Pro Gly

35                  40                  45

acc ctc aaa cag ggc gag gcc acc aag acg acg gga acc acc atc agg     192
Thr Leu Lys Gln Gly Glu Ala Thr Lys Thr Thr Gly Thr Thr Ile Arg
    50                  55                  60

ttc tgg gcc gat ccc gac atc ttc gag acc acc gag tac gac ttc gag     240
Phe Trp Ala Asp Pro Asp Ile Phe Glu Thr Thr Glu Tyr Asp Phe Glu
65                  70                  75                  80

acg gtg gcg cgc cga ctg cag gaa atg gcg ttc ctg aac aag ggt ttg     288
Thr Val Ala Arg Arg Leu Gln Glu Met Ala Phe Leu Asn Lys Gly Leu
                85                  90                  95

acg atc aac ctc acc gac gag cgg gtc agt gaa gag gag gtc gtc gac     336
Thr Ile Asn Leu Thr Asp Glu Arg Val Ser Glu Glu Glu Val Val Asp
            100                 105                 110

gat gtc gtc agc gac acc gcc gag gca ccc aag tcc gcc gta gaa aaa     384
Asp Val Val Ser Asp Thr Ala Glu Ala Pro Lys Ser Ala Val Glu Lys
        115                 120                 125

gcg gcc gaa tcg act ggc cca cac aag gtt aag cac cgc acg ttc cac     432
Ala Ala Glu Ser Thr Gly Pro His Lys Val Lys His Arg Thr Phe His
    130                 135                 140

tac ccg ggc ggc ttg gtg gac ttc gtc aag cac atc aat cgg acc aag     480
Tyr Pro Gly Gly Leu Val Asp Phe Val Lys His Ile Asn Arg Thr Lys
145                 150                 155                 160

aac ccg att cac aac agc atc gtg gat ttc tcc ggc aag gga ccg ggc     528
Asn Pro Ile His Asn Ser Ile Val Asp Phe Ser Gly Lys Gly Pro Gly
                165                 170                 175

cac gag gtc gaa atc gcg atg cag tgg aat gcc ggc tac tcg gag tcg     576
His Glu Val Glu Ile Ala Met Gln Trp Asn Ala Gly Tyr Ser Glu Ser
            180                 185                 190

gtg cac acc ttc gcc aac acc atc aac acc cac gag ggc ggc acc cac     624
Val His Thr Phe Ala Asn Thr Ile Asn Thr His Glu Gly Gly Thr His
        195                 200                 205

gaa gag ggc ttc cgc agc gcg ttg acg tcg gtg gtc aac aaa tac gcc     672
Glu Glu Gly Phe Arg Ser Ala Leu Thr Ser Val Val Asn Lys Tyr Ala
    210                 215                 220

aag gac cgc aaa ctc ctg aag gac aaa gac ccc aac ctc acc ggc gac     720
Lys Asp Arg Lys Leu Leu Lys Asp Lys Asp Pro Asn Leu Thr Gly Asp
225                 230                 235                 240

gac atc cgg gaa ggc ctg gca gcg gtc att tcc gtc aag gtc agc gaa     768
Asp Ile Arg Glu Gly Leu Ala Ala Val Ile Ser Val Lys Val Ser Glu
                245                 250                 255

ccg caa ttc gag ggc cag acc aaa acc aag ctg ggc aac acc gag gtc     816
Pro Gln Phe Glu Gly Gln Thr Lys Thr Lys Leu Gly Asn Thr Glu Val

            260                 265                 270

aag tcg ttc gtg cag aag gtc tgc aac gaa cag ctc acg cac tgg ttc 864
Lys Ser Phe Val Gln Lys Val Cys Asn Glu Gln Leu Thr His Trp Phe
        275                 280                 285

gaa gcc aac ccg gcg gat gcc aaa act gtt gta aac aag gcg gtt tcg 912
Glu Ala Asn Pro Ala Asp Ala Lys Thr Val Val Asn Lys Ala Val Ser
    290                 295                 300

tcg gcc cag gcc cga atc gca gcg cgc aag gcg cga gaa ctg gtg cgc 960
Ser Ala Gln Ala Arg Ile Ala Ala Arg Lys Ala Arg Glu Leu Val Arg
305                 310                 315                 320

cgc aag agc gcc acc gac ctc ggt ggg ctg ccg ggt aag ctc gca gac 1008
Arg Lys Ser Ala Thr Asp Leu Gly Gly Leu Pro Gly Lys Leu Ala Asp
                325                 330                 335

tgc cgc tcc acc gac ccg cga aag tcg gaa ctg tat gtg gtg gag ggt 1056
Cys Arg Ser Thr Asp Pro Arg Lys Ser Glu Leu Tyr Val Val Glu Gly
            340                 345                 350

gac tcg gcc ggc ggc tcg gcc aag agc ggc cgc gac tcg atg ttc cag 1104
Asp Ser Ala Gly Gly Ser Ala Lys Ser Gly Arg Asp Ser Met Phe Gln
        355                 360                 365

gcg atc ctc ccg ctg cgt ggc aag atc atc aac gtc gag aag gcg cgc 1152
Ala Ile Leu Pro Leu Arg Gly Lys Ile Ile Asn Val Glu Lys Ala Arg
    370                 375                 380

atc gac cgg gtg ctg aag aac acc gaa gtt cag gcg atc atc acc gcg 1200
Ile Asp Arg Val Leu Lys Asn Thr Glu Val Gln Ala Ile Ile Thr Ala
385                 390                 395                 400

ctg ggc acg ggg att cac gac gag ttc gac atc acc aag ctc cgg tac 1248
Leu Gly Thr Gly Ile His Asp Glu Phe Asp Ile Thr Lys Leu Arg Tyr
                405                 410                 415

cac aag atc gtg ctg 1263
His Lys Ile Val Leu
            420

<210> 14
<211> 421
<212> PRT
<213> Mycobacterium malmoense

<400> 14
Gly Glu Asn Ser Gly Tyr Asn Val Ser Gly Gly Leu His Gly Val Gly
1               5                   10                  15

Val Ser Val Val Asn Ala Leu Ser Thr Arg Leu Glu Val Asp Val Ala
            20                  25                  30

Arg Asp Gly Tyr Met Trp Ser Gln Phe Tyr Asp His Ala Glu Pro Gly

35                          40                          45

Thr Leu Lys Gln Gly Glu Ala Thr Lys Thr Thr Gly Thr Thr Ile Arg
     50                          55                          60

Phe Trp Ala Asp Pro Asp Ile Phe Glu Thr Thr Glu Tyr Asp Phe Glu
 65                          70                          75                          80

Thr Val Ala Arg Arg Leu Gln Glu Met Ala Phe Leu Asn Lys Gly Leu
                         85                          90                          95

Thr Ile Asn Leu Thr Asp Glu Arg Val Ser Glu Glu Glu Val Val Asp
                    100                         105                         110

Asp Val Val Ser Asp Thr Ala Glu Ala Pro Lys Ser Ala Val Glu Lys
          115                         120                         125

Ala Ala Glu Ser Thr Gly Pro His Lys Val Lys His Arg Thr Phe His
     130                         135                         140

Tyr Pro Gly Gly Leu Val Asp Phe Val Lys His Ile Asn Arg Thr Lys
145                         150                         155                         160

Asn Pro Ile His Asn Ser Ile Val Asp Phe Ser Gly Lys Gly Pro Gly
                         165                         170                         175

His Glu Val Glu Ile Ala Met Gln Trp Asn Ala Gly Tyr Ser Glu Ser
                    180                         185                         190

Val His Thr Phe Ala Asn Thr Ile Asn Thr His Glu Gly Gly Thr His
          195                         200                         205

Glu Glu Gly Phe Arg Ser Ala Leu Thr Ser Val Val Asn Lys Tyr Ala
     210                         215                         220

Lys Asp Arg Lys Leu Leu Lys Asp Lys Asp Pro Asn Leu Thr Gly Asp
225                         230                         235                         240

Asp Ile Arg Glu Gly Leu Ala Ala Val Ile Ser Val Lys Val Ser Glu
                         245                         250                         255

Pro Gln Phe Glu Gly Gln Thr Lys Thr Lys Leu Gly Asn Thr Glu Val
                    260                         265                         270

Lys Ser Phe Val Gln Lys Val Cys Asn Glu Gln Leu Thr His Trp Phe
          275                         280                         285

Glu Ala Asn Pro Ala Asp Ala Lys Thr Val Val Asn Lys Ala Val Ser
     290                         295                         300

Ser Ala Gln Ala Arg Ile Ala Ala Arg Lys Ala Arg Glu Leu Val Arg
305                         310                         315                         320

Arg Lys Ser Ala Thr Asp Leu Gly Gly Leu Pro Gly Lys Leu Ala Asp
                         325                         330                         335

Cys Arg Ser Thr Asp Pro Arg Lys Ser Glu Leu Tyr Val Val Glu Gly
            340                 345                 350

Asp Ser Ala Gly Gly Ser Ala Lys Ser Gly Arg Asp Ser Met Phe Gln
        355                 360                 365

Ala Ile Leu Pro Leu Arg Gly Lys Ile Ile Asn Val Glu Lys Ala Arg
    370                 375                 380

Ile Asp Arg Val Leu Lys Asn Thr Glu Val Gln Ala Ile Ile Thr Ala
385                 390                 395                 400

Leu Gly Thr Gly Ile His Asp Glu Phe Asp Ile Thr Lys Leu Arg Tyr
                405                 410                 415

His Lys Ile Val Leu
            420

<210> 15
<211> 1263
<212> DNA
<213> Mycobacterium intracellulare

<220>
<221> CDS
<222> (1)..(1263)

<400> 15
ggt gag aac agc ggt tac aac gtc agc ggt ggc ctg cac ggc gtg ggc      48
Gly Glu Asn Ser Gly Tyr Asn Val Ser Gly Gly Leu His Gly Val Gly
  1               5                  10                  15

gtc tcg gtg gtc aac gcg ctg tcg acc cgg ctc gag gtg gac atc gcc      96
Val Ser Val Val Asn Ala Leu Ser Thr Arg Leu Glu Val Asp Ile Ala
             20                  25                  30

cgc gat ggc tac gaa tgg tcg cag ttc tac gac cac gcc gta ccc gga     144
Arg Asp Gly Tyr Glu Trp Ser Gln Phe Tyr Asp His Ala Val Pro Gly
         35                  40                  45

acg ctc aaa cag ggt gag gcc acc aag cgg acg ggc acc acg atc agg     192
Thr Leu Lys Gln Gly Glu Ala Thr Lys Arg Thr Gly Thr Thr Ile Arg
     50                  55                  60

ttc tgg gcc gac ccc gac atc ttc gag acc acc gag tac gac ttc gag     240
Phe Trp Ala Asp Pro Asp Ile Phe Glu Thr Thr Glu Tyr Asp Phe Glu
 65                  70                  75                  80

acg gtg gcg cgc cgg ctg cag gaa atg gcg ttc ctc aac aag ggg ttg     288
Thr Val Ala Arg Arg Leu Gln Glu Met Ala Phe Leu Asn Lys Gly Leu
                 85                  90                  95

acc atc aac ctc acc gac gag cgg gtg agc aac gag gag gtc gtc gac     336
Thr Ile Asn Leu Thr Asp Glu Arg Val Ser Asn Glu Glu Val Val Asp
            100                 105                 110

gag gtc gtc agc gat acc gcc gac gca ccc aag tcg gcc cag gaa aag 384
Glu Val Val Ser Asp Thr Ala Asp Ala Pro Lys Ser Ala Gln Glu Lys
        115                 120                 125

gcg gcg gaa tcg act gcg cca cat aag gtt aag cac cgc acc ttc cac 432
Ala Ala Glu Ser Thr Ala Pro His Lys Val Lys His Arg Thr Phe His
    130                 135                 140

tac ccc ggc ggt ctg gtc gac ttc gtc aag cac atc aac cgc acc aag 480
Tyr Pro Gly Gly Leu Val Asp Phe Val Lys His Ile Asn Arg Thr Lys
145                 150                 155                 160

agc ccg atc cag cag agc atc atc gac ttc gac ggc aaa ggt ccc ggc 528
Ser Pro Ile Gln Gln Ser Ile Ile Asp Phe Asp Gly Lys Gly Pro Gly
                165                 170                 175

cac gag gtc gag atc gcg atg cag tgg aac ggc ggc tac tcg gaa tcc 576
His Glu Val Glu Ile Ala Met Gln Trp Asn Gly Gly Tyr Ser Glu Ser
            180                 185                 190

gtg cac acc ttc gcc aac acc atc aac acc cac gag ggc ggc acc cac 624
Val His Thr Phe Ala Asn Thr Ile Asn Thr His Glu Gly Gly Thr His
        195                 200                 205

gaa gag ggc ttc cgc agc gcg ctg acg tcg gtg gtg aac aag tac gcc 672
Glu Glu Gly Phe Arg Ser Ala Leu Thr Ser Val Val Asn Lys Tyr Ala
    210                 215                 220

aaa gac aag aag ttg ctg aaa gac aag gac ccg aac ctc acc ggc gac 720
Lys Asp Lys Lys Leu Leu Lys Asp Lys Asp Pro Asn Leu Thr Gly Asp
225                 230                 235                 240

gac att cgc gaa ggc ctg gcc gcg gtg atc tcg gtc aag gtc agc gaa 768
Asp Ile Arg Glu Gly Leu Ala Ala Val Ile Ser Val Lys Val Ser Glu
                245                 250                 255

ccg cag ttc gag ggt cag acc aag acc aag ctg ggc aac acc gaa gtg 816
Pro Gln Phe Glu Gly Gln Thr Lys Thr Lys Leu Gly Asn Thr Glu Val
            260                 265                 270

aag tcg ttc gtg cag aag gtc tgc aac gaa cag ctc acc cac tgg ttc 864
Lys Ser Phe Val Gln Lys Val Cys Asn Glu Gln Leu Thr His Trp Phe
        275                 280                 285

gag gcc aac ccc gcg gac gcc aag gtg gtg gtc aac aag gcg gtg tcg 912
Glu Ala Asn Pro Ala Asp Ala Lys Val Val Val Asn Lys Ala Val Ser
    290                 295                 300

tcg gcg cag gcc cgg atc gcc gcg cgc aag gcg cga gag ttg gtg cgt 960
Ser Ala Gln Ala Arg Ile Ala Ala Arg Lys Ala Arg Glu Leu Val Arg
305                 310                 315                 320

cgc aag agc gcc acc gat ctg ggc ggg ctg ccc ggc aag ctc gcc gac 1008
Arg Lys Ser Ala Thr Asp Leu Gly Gly Leu Pro Gly Lys Leu Ala Asp
                325                 330                 335

tgc cgc tcg acg gat ccg cgc aag tcg gaa ctg tat gtg gtg gag ggt 1056
Cys Arg Ser Thr Asp Pro Arg Lys Ser Glu Leu Tyr Val Val Glu Gly
            340                 345                 350

gat tcg gcc ggc ggc tcg gcg aag agc ggc cgc gac tcg atg ttc cag 1104
Asp Ser Ala Gly Gly Ser Ala Lys Ser Gly Arg Asp Ser Met Phe Gln
        355                 360                 365

gcc atc ctg ccg ctg cgc ggc aag atc atc aac gtc gag aag gcc cgc 1152
Ala Ile Leu Pro Leu Arg Gly Lys Ile Ile Asn Val Glu Lys Ala Arg
    370                 375                 380

atc gac cgg gtg ttg aag aac acc gag gtg cag gcc atc atc acc gcc 1200
Ile Asp Arg Val Leu Lys Asn Thr Glu Val Gln Ala Ile Ile Thr Ala
385                 390                 395                 400

ctg ggc acc ggc atc cac gac gag ttc gac atc acc aag ctg cgc tat 1248
Leu Gly Thr Gly Ile His Asp Glu Phe Asp Ile Thr Lys Leu Arg Tyr
                405                 410                 415

cac aag atc gtg ctg 1263
His Lys Ile Val Leu
            420

<210> 16
<211> 421
<212> PRT
<213> Mycobacterium intracellulare

<400> 16
Gly Glu Asn Ser Gly Tyr Asn Val Ser Gly Gly Leu His Gly Val Gly
1               5                   10                  15

Val Ser Val Val Asn Ala Leu Ser Thr Arg Leu Glu Val Asp Ile Ala
            20                  25                  30

Arg Asp Gly Tyr Glu Trp Ser Gln Phe Tyr Asp His Ala Val Pro Gly
        35                  40                  45

Thr Leu Lys Gln Gly Glu Ala Thr Lys Arg Thr Gly Thr Thr Ile Arg
    50                  55                  60

Phe Trp Ala Asp Pro Asp Ile Phe Glu Thr Thr Glu Tyr Asp Phe Glu
65                  70                  75                  80

Thr Val Ala Arg Arg Leu Gln Glu Met Ala Phe Leu Asn Lys Gly Leu
                85                  90                  95

Thr Ile Asn Leu Thr Asp Glu Arg Val Ser Asn Glu Glu Val Val Asp
            100                 105                 110

Glu Val Val Ser Asp Thr Ala Asp Ala Pro Lys Ser Ala Gln Glu Lys
        115                 120                 125

Ala Ala Glu Ser Thr Ala Pro His Lys Val Lys His Arg Thr Phe His
    130                 135                 140
Tyr Pro Gly Gly Leu Val Asp Phe Val Lys His Ile Asn Arg Thr Lys
145                 150                 155                 160
Ser Pro Ile Gln Gln Ser Ile Ile Asp Phe Asp Gly Lys Gly Pro Gly
                165                 170                 175
His Glu Val Glu Ile Ala Met Gln Trp Asn Gly Gly Tyr Ser Glu Ser
            180                 185                 190
Val His Thr Phe Ala Asn Thr Ile Asn Thr His Glu Gly Gly Thr His
        195                 200                 205
Glu Glu Gly Phe Arg Ser Ala Leu Thr Ser Val Val Asn Lys Tyr Ala
    210                 215                 220
Lys Asp Lys Lys Leu Leu Lys Asp Lys Asp Pro Asn Leu Thr Gly Asp
225                 230                 235                 240
Asp Ile Arg Glu Gly Leu Ala Ala Val Ile Ser Val Lys Val Ser Glu
                245                 250                 255
Pro Gln Phe Glu Gly Gln Thr Lys Thr Lys Leu Gly Asn Thr Glu Val
            260                 265                 270
Lys Ser Phe Val Gln Lys Val Cys Asn Glu Gln Leu Thr His Trp Phe
        275                 280                 285
Glu Ala Asn Pro Ala Asp Ala Lys Val Val Val Asn Lys Ala Val Ser
    290                 295                 300
Ser Ala Gln Ala Arg Ile Ala Ala Arg Lys Ala Arg Glu Leu Val Arg
305                 310                 315                 320
Arg Lys Ser Ala Thr Asp Leu Gly Gly Leu Pro Gly Lys Leu Ala Asp
                325                 330                 335
Cys Arg Ser Thr Asp Pro Arg Lys Ser Glu Leu Tyr Val Val Glu Gly
            340                 345                 350
Asp Ser Ala Gly Gly Ser Ala Lys Ser Gly Arg Asp Ser Met Phe Gln
        355                 360                 365
Ala Ile Leu Pro Leu Arg Gly Lys Ile Ile Asn Val Glu Lys Ala Arg
    370                 375                 380
Ile Asp Arg Val Leu Lys Asn Thr Glu Val Gln Ala Ile Ile Thr Ala
385                 390                 395                 400
Leu Gly Thr Gly Ile His Asp Glu Phe Asp Ile Thr Lys Leu Arg Tyr
                405                 410                 415
His Lys Ile Val Leu
            420

<210> 17
<211> 1263
<212> DNA
<213> Mycobacterium avium

<220>
<221> CDS
<222> (1)..(1263)

<400> 17
ggc gag aac agc ggc tac aac gtc agc ggc ggt ctg cac ggc gtc ggc 48
Gly Glu Asn Ser Gly Tyr Asn Val Ser Gly Gly Leu His Gly Val Gly
1               5                   10                  15

gtc tcg gtg gtc aac gcg ctg tcc act cgg ctc gag gtc aac atc gcc 96
Val Ser Val Val Asn Ala Leu Ser Thr Arg Leu Glu Val Asn Ile Ala
            20                  25                  30

cgc gac ggc tac gag tgg tcg cag tac tac gac cac gcc gtg ccc ggc 144
Arg Asp Gly Tyr Glu Trp Ser Gln Tyr Tyr Asp His Ala Val Pro Gly
        35                  40                  45

acc ctc aag cag ggc gag gcc acc aag cgc acc ggc acc acc atc cgg 192
Thr Leu Lys Gln Gly Glu Ala Thr Lys Arg Thr Gly Thr Thr Ile Arg
    50                  55                  60

ttc tgg gcc gac ccc gac atc ttc gag acc acc gag tac gac ttc gaa 240
Phe Trp Ala Asp Pro Asp Ile Phe Glu Thr Thr Glu Tyr Asp Phe Glu
65                  70                  75                  80

acg gtg gcc cgg cgg ctg cag gaa atg gcg ttc ctc aac aag ggc ctg 288
Thr Val Ala Arg Arg Leu Gln Glu Met Ala Phe Leu Asn Lys Gly Leu
                85                  90                  95

acc atc aac ctc acc gac gag cgg gtg acc aac gaa gag gtc gtc gac 336
Thr Ile Asn Leu Thr Asp Glu Arg Val Thr Asn Glu Glu Val Val Asp
            100                 105                 110

gag gtg gtc agc gac acc gcc gac gca ccc aag tcg gcg cag gag aag 384
Glu Val Val Ser Asp Thr Ala Asp Ala Pro Lys Ser Ala Gln Glu Lys
        115                 120                 125

gcg gcg gaa tcg gct gcg ccg cat aag gtc aag cac cgc acc ttc cac 432
Ala Ala Glu Ser Ala Ala Pro His Lys Val Lys His Arg Thr Phe His
    130                 135                 140

tac ccc ggc ggc ctg gtc gac ttc gtc aaa cac atc aat cgc acc aaa 480
Tyr Pro Gly Gly Leu Val Asp Phe Val Lys His Ile Asn Arg Thr Lys
145                 150                 155                 160

aac ccc atc cac cag agc atc atc gat ttc ggt ggg aag ggc ccc ggc 528
Asn Pro Ile His Gln Ser Ile Ile Asp Phe Gly Gly Lys Gly Pro Gly
                165                 170                 175

cac gag gtc gag atc gcg atg cag tgg aac ggc ggc tac tcc gaa tcg 576
His Glu Val Glu Ile Ala Met Gln Trp Asn Gly Gly Tyr Ser Glu Ser
            180                 185                 190

gtg cac acc ttc gcc aac acc atc aac acg cac gag ggc ggc acc cac 624
Val His Thr Phe Ala Asn Thr Ile Asn Thr His Glu Gly Gly Thr His
        195                 200                 205

gag gag ggc ttc cgc agc gcg ctg acc tcc gtg gtc aac aag tac gcc 672
Glu Glu Gly Phe Arg Ser Ala Leu Thr Ser Val Val Asn Lys Tyr Ala
    210                 215                 220

aag gac aag aag ctg ctc aag gac aag gac ccc aac ctg acc ggc gac 720
Lys Asp Lys Lys Leu Leu Lys Asp Lys Asp Pro Asn Leu Thr Gly Asp
225                 230                 235                 240

gac atc cgc gag ggt ttg gcc gcg gtg atc tcg gtc aag gtg agc gaa 768
Asp Ile Arg Glu Gly Leu Ala Ala Val Ile Ser Val Lys Val Ser Glu
                245                 250                 255

ccg cag ttc gag ggc cag acc aag acc aaa ctg ggc aac acc gag gtg 816
Pro Gln Phe Glu Gly Gln Thr Lys Thr Lys Leu Gly Asn Thr Glu Val
            260                 265                 270

aag tcg ttc gtg cag aag gtg tgc aac gaa cag ctc acc cac tgg ttc 864
Lys Ser Phe Val Gln Lys Val Cys Asn Glu Gln Leu Thr His Trp Phe
        275                 280                 285

gaa gcc aac ccc gca gac gcc aaa gtc att gtc aac aag gcg gtt tca 912
Glu Ala Asn Pro Ala Asp Ala Lys Val Ile Val Asn Lys Ala Val Ser
    290                 295                 300

tca gcg cag gcg cgc atc gcc gcg cgc aag gcg cga gag ttg gtg cgc 960
Ser Ala Gln Ala Arg Ile Ala Ala Arg Lys Ala Arg Glu Leu Val Arg
305                 310                 315                 320

cgc aag agc gca acc gac ctg ggc ggg ctg ccc ggc aag ctc gcc gac 1008
Arg Lys Ser Ala Thr Asp Leu Gly Gly Leu Pro Gly Lys Leu Ala Asp
                325                 330                 335

tgc cgg tcg acc gac ccg cgc aag tcg gaa ttg tat gtg gtc gag ggt 1056
Cys Arg Ser Thr Asp Pro Arg Lys Ser Glu Leu Tyr Val Val Glu Gly
            340                 345                 350

gac tcg gcc ggc ggc tcg gcg aaa agc ggc cgg gac tcg atg ttc cag 1104
Asp Ser Ala Gly Gly Ser Ala Lys Ser Gly Arg Asp Ser Met Phe Gln
        355                 360                 365

gcc atc ctt ccg ctg cgc ggc aag atc atc aac gtc gaa aag gcc cgc 1152
Ala Ile Leu Pro Leu Arg Gly Lys Ile Ile Asn Val Glu Lys Ala Arg
    370                 375                 380

atc gac cgg gtg ctg aag aac acc gag gtg cag gcg atc atc acc gcg 1200
Ile Asp Arg Val Leu Lys Asn Thr Glu Val Gln Ala Ile Ile Thr Ala
385                 390                 395                 400

ctg ggc acc ggg att cac gac gag ttc gac atc acc aag ctg cgc tac 1248
Leu Gly Thr Gly Ile His Asp Glu Phe Asp Ile Thr Lys Leu Arg Tyr
                405                 410                 415

cac aag atc gtg ttg 1263
His Lys Ile Val Leu
            420

<210> 18
<211> 421
<212> PRT
<213> Mycobacterium avium

<400> 18
Gly Glu Asn Ser Gly Tyr Asn Val Ser Gly Gly Leu His Gly Val Gly
1               5                   10                  15

Val Ser Val Val Asn Ala Leu Ser Thr Arg Leu Glu Val Asn Ile Ala
            20                  25                  30

Arg Asp Gly Tyr Glu Trp Ser Gln Tyr Tyr Asp His Ala Val Pro Gly
        35                  40                  45

Thr Leu Lys Gln Gly Glu Ala Thr Lys Arg Thr Gly Thr Thr Ile Arg
    50                  55                  60

Phe Trp Ala Asp Pro Asp Ile Phe Glu Thr Thr Glu Tyr Asp Phe Glu
65                  70                  75                  80

Thr Val Ala Arg Arg Leu Gln Glu Met Ala Phe Leu Asn Lys Gly Leu
                85                  90                  95

Thr Ile Asn Leu Thr Asp Glu Arg Val Thr Asn Glu Glu Val Val Asp
            100                 105                 110

Glu Val Val Ser Asp Thr Ala Asp Ala Pro Lys Ser Ala Gln Glu Lys
        115                 120                 125

Ala Ala Glu Ser Ala Ala Pro His Lys Val Lys His Arg Thr Phe His
    130                 135                 140

Tyr Pro Gly Gly Leu Val Asp Phe Val Lys His Ile Asn Arg Thr Lys
145                 150                 155                 160

Asn Pro Ile His Gln Ser Ile Ile Asp Phe Gly Gly Lys Gly Pro Gly
                165                 170                 175

His Glu Val Glu Ile Ala Met Gln Trp Asn Gly Gly Tyr Ser Glu Ser
            180                 185                 190

Val His Thr Phe Ala Asn Thr Ile Asn Thr His Glu Gly Gly Thr His
        195                 200                 205

Glu Glu Gly Phe Arg Ser Ala Leu Thr Ser Val Val Asn Lys Tyr Ala
    210                 215                 220

Lys Asp Lys Lys Leu Leu Lys Asp Lys Asp Pro Asn Leu Thr Gly Asp
225                 230                 235                 240

Asp Ile Arg Glu Gly Leu Ala Ala Val Ile Ser Val Lys Val Ser Glu
                245                 250                 255

Pro Gln Phe Glu Gly Gln Thr Lys Thr Lys Leu Gly Asn Thr Glu Val
            260                 265                 270

Lys Ser Phe Val Gln Lys Val Cys Asn Glu Gln Leu Thr His Trp Phe
        275                 280                 285

Glu Ala Asn Pro Ala Asp Ala Lys Val Ile Val Asn Lys Ala Val Ser
    290                 295                 300

Ser Ala Gln Ala Arg Ile Ala Ala Arg Lys Ala Arg Glu Leu Val Arg
305                 310                 315                 320

Arg Lys Ser Ala Thr Asp Leu Gly Gly Leu Pro Gly Lys Leu Ala Asp
                325                 330                 335

Cys Arg Ser Thr Asp Pro Arg Lys Ser Glu Leu Tyr Val Val Glu Gly
            340                 345                 350

Asp Ser Ala Gly Gly Ser Ala Lys Ser Gly Arg Asp Ser Met Phe Gln
        355                 360                 365

Ala Ile Leu Pro Leu Arg Gly Lys Ile Ile Asn Val Glu Lys Ala Arg
    370                 375                 380

Ile Asp Arg Val Leu Lys Asn Thr Glu Val Gln Ala Ile Ile Thr Ala
385                 390                 395                 400

Leu Gly Thr Gly Ile His Asp Glu Phe Asp Ile Thr Lys Leu Arg Tyr
                405                 410                 415

His Lys Ile Val Leu
            420

<210> 19
<211> 1263
<212> DNA
<213> Mycobacterium gordonae

<220>
<221> CDS
<222> (1)..(1263)

<400> 19
ggc gag aac agc ggc tac acg gtc agc ggt ggg ttg cac ggc gtg ggc      48
Gly Glu Asn Ser Gly Tyr Thr Val Ser Gly Gly Leu His Gly Val Gly
  1               5                  10                  15

gtg tcg gtg gtt aac gcg ttg tcg acg cgg ttg gaa gtc gac atc aaa      96

Val Ser Val Val Asn Ala Leu Ser Thr Arg Leu Glu Val Asp Ile Lys
            20                  25                  30

cgc gac ggg cac gag tgg tcg cag tat tac aag cgc gcg gtg ccg ggc 144
Arg Asp Gly His Glu Trp Ser Gln Tyr Tyr Lys Arg Ala Val Pro Gly
        35                  40                  45

acc ctc aag cag ggt gag acg acc cgc aag acc ggc acc aca atc cgg 192
Thr Leu Lys Gln Gly Glu Thr Thr Arg Lys Thr Gly Thr Thr Ile Arg
    50                  55                  60

ttc tgg gcg gat ccg gag atc ttc gag acc acc caa tac gac ttc gag 240
Phe Trp Ala Asp Pro Glu Ile Phe Glu Thr Thr Gln Tyr Asp Phe Glu
65                  70                  75                  80

acg gtg gcg cgc cgg ctg cag gag atg gcg ttc ctg aac aag ggt ctg 288
Thr Val Ala Arg Arg Leu Gln Glu Met Ala Phe Leu Asn Lys Gly Leu
                85                  90                  95

acg atc aat ctg acc gac gaa cgc gtc gag cag gac gag gtt gtc gac 336
Thr Ile Asn Leu Thr Asp Glu Arg Val Glu Gln Asp Glu Val Val Asp
            100                 105                 110

gag gtc gtc agc gac acc gcc gaa gcg ccc aaa tcc gcc gaa gag aag 384
Glu Val Val Ser Asp Thr Ala Glu Ala Pro Lys Ser Ala Glu Glu Lys
        115                 120                 125

gct gcc gaa tcc aag gcc ccg cac aag gtc aag cag cgc acc ttc cac 432
Ala Ala Glu Ser Lys Ala Pro His Lys Val Lys Gln Arg Thr Phe His
    130                 135                 140

tat ccc ggt ggt ctg gtc gac ttc gtc aaa cac atc aac cgc acc aaa 480
Tyr Pro Gly Gly Leu Val Asp Phe Val Lys His Ile Asn Arg Thr Lys
145                 150                 155                 160

agc ccg atc cag cag agc gtc atc gac ttc gaa ggc aaa ggc acc ggc 528
Ser Pro Ile Gln Gln Ser Val Ile Asp Phe Glu Gly Lys Gly Thr Gly
                165                 170                 175

cac gag gtc gaa atc gcg atg cag tgg aac ggc ggc tac tcc gaa tcg 576
His Glu Val Glu Ile Ala Met Gln Trp Asn Gly Gly Tyr Ser Glu Ser
            180                 185                 190

gtg cac acc ttc gcc aac acc atc aac acc cac gag ggc ggc acc cac 624
Val His Thr Phe Ala Asn Thr Ile Asn Thr His Glu Gly Gly Thr His
        195                 200                 205

gaa gag ggc ttc cgc agt gcg ctg acc tcg gtg gtc aac aag tac gcc 672
Glu Glu Gly Phe Arg Ser Ala Leu Thr Ser Val Val Asn Lys Tyr Ala
    210                 215                 220

aaa gac aag aag ctg ctc aag gag aag gac ccg aat ctc acc ggt gac 720
Lys Asp Lys Lys Leu Leu Lys Glu Lys Asp Pro Asn Leu Thr Gly Asp
225                 230                 235                 240

gac atc cgg gag ggg ttg gcc gcg gtg atc tcg gtg aag gtc gcc gaa 768

Asp Ile Arg Glu Gly Leu Ala Ala Val Ile Ser Val Lys Val Ala Glu
                245                 250                 255

ccg cag ttc gag ggt cag acc aag acc aag ctg ggc aac acc gag gtc      816
Pro Gln Phe Glu Gly Gln Thr Lys Thr Lys Leu Gly Asn Thr Glu Val
            260                 265                 270

aag tcg ttc gtg cag aag gtg tgc aac gaa cag ctc acc cac tgg ttc      864
Lys Ser Phe Val Gln Lys Val Cys Asn Glu Gln Leu Thr His Trp Phe
        275                 280                 285

gag gcc aat ccg tcg gaa gct aaa acc gtt gtg aac aaa gcg gtg tcg      912
Glu Ala Asn Pro Ser Glu Ala Lys Thr Val Val Asn Lys Ala Val Ser
    290                 295                 300

tcc gcc cag gcg cgg atc gcc gcg cgc aaa gcg cga gag ctg gtg cgc      960
Ser Ala Gln Ala Arg Ile Ala Ala Arg Lys Ala Arg Glu Leu Val Arg
305                 310                 315                 320

cgc aag agc gca acc gac ctc ggc ggc ctg ccg ggc aag ctc gcc gac      1008
Arg Lys Ser Ala Thr Asp Leu Gly Gly Leu Pro Gly Lys Leu Ala Asp
                325                 330                 335

tgc cgt tcg acg gat ccc cgc aaa tcc gaa ctg tat gtg gtg gag ggg      1056
Cys Arg Ser Thr Asp Pro Arg Lys Ser Glu Leu Tyr Val Val Glu Gly
            340                 345                 350

gac tcc gcc ggc ggc tcg gcc aag agc ggt cgg gat tcg atg ttc cag      1104
Asp Ser Ala Gly Gly Ser Ala Lys Ser Gly Arg Asp Ser Met Phe Gln
        355                 360                 365

gcg att ctt ccg ttg cgc ggc aag atc atc aac gtc gag aag gcc cgc      1152
Ala Ile Leu Pro Leu Arg Gly Lys Ile Ile Asn Val Glu Lys Ala Arg
    370                 375                 380

atc gac cgg gtg ctg aag aac acc gaa gtc cag gcc atc atc acc gcg      1200
Ile Asp Arg Val Leu Lys Asn Thr Glu Val Gln Ala Ile Ile Thr Ala
385                 390                 395                 400

ctg ggc acc ggg atc cac gac gag ttc gac atc acc aaa ctg cgc tac      1248
Leu Gly Thr Gly Ile His Asp Glu Phe Asp Ile Thr Lys Leu Arg Tyr
                405                 410                 415

cac aag atc gta ttg                                                  1263
His Lys Ile Val Leu
            420


<210> 20
<211> 421
<212> PRT
<213> Mycobacterium gordonae

<400> 20
Gly Glu Asn Ser Gly Tyr Thr Val Ser Gly Gly Leu His Gly Val Gly
  1               5                  10                  15

Val Ser Val Val Asn Ala Leu Ser Thr Arg Leu Glu Val Asp Ile Lys
            20                  25                  30

Arg Asp Gly His Glu Trp Ser Gln Tyr Tyr Lys Arg Ala Val Pro Gly
        35                  40                  45

Thr Leu Lys Gln Gly Glu Thr Thr Arg Lys Thr Gly Thr Thr Ile Arg
    50                  55                  60

Phe Trp Ala Asp Pro Glu Ile Phe Glu Thr Thr Gln Tyr Asp Phe Glu
65                  70                  75                  80

Thr Val Ala Arg Arg Leu Gln Glu Met Ala Phe Leu Asn Lys Gly Leu
                85                  90                  95

Thr Ile Asn Leu Thr Asp Glu Arg Val Glu Gln Asp Glu Val Val Asp
            100                 105                 110

Glu Val Val Ser Asp Thr Ala Glu Ala Pro Lys Ser Ala Glu Glu Lys
        115                 120                 125

Ala Ala Glu Ser Lys Ala Pro His Lys Val Lys Gln Arg Thr Phe His
    130                 135                 140

Tyr Pro Gly Gly Leu Val Asp Phe Val Lys His Ile Asn Arg Thr Lys
145                 150                 155                 160

Ser Pro Ile Gln Gln Ser Val Ile Asp Phe Glu Gly Lys Gly Thr Gly
                165                 170                 175

His Glu Val Glu Ile Ala Met Gln Trp Asn Gly Gly Tyr Ser Glu Ser
            180                 185                 190

Val His Thr Phe Ala Asn Thr Ile Asn Thr His Glu Gly Gly Thr His
        195                 200                 205

Glu Glu Gly Phe Arg Ser Ala Leu Thr Ser Val Val Asn Lys Tyr Ala
    210                 215                 220

Lys Asp Lys Lys Leu Leu Lys Glu Lys Asp Pro Asn Leu Thr Gly Asp
225                 230                 235                 240

Asp Ile Arg Glu Gly Leu Ala Ala Val Ile Ser Val Lys Val Ala Glu
                245                 250                 255

Pro Gln Phe Glu Gly Gln Thr Lys Thr Lys Leu Gly Asn Thr Glu Val
            260                 265                 270

Lys Ser Phe Val Gln Lys Val Cys Asn Glu Gln Leu Thr His Trp Phe
        275                 280                 285

Glu Ala Asn Pro Ser Glu Ala Lys Thr Val Val Asn Lys Ala Val Ser
    290                 295                 300

Ser Ala Gln Ala Arg Ile Ala Ala Arg Lys Ala Arg Glu Leu Val Arg

305                 310                 315                 320

Arg Lys Ser Ala Thr Asp Leu Gly Gly Leu Pro Gly Lys Leu Ala Asp
                325                 330                 335

Cys Arg Ser Thr Asp Pro Arg Lys Ser Glu Leu Tyr Val Val Glu Gly
            340                 345                 350

Asp Ser Ala Gly Gly Ser Ala Lys Ser Gly Arg Asp Ser Met Phe Gln
        355                 360                 365

Ala Ile Leu Pro Leu Arg Gly Lys Ile Ile Asn Val Glu Lys Ala Arg
    370                 375                 380

Ile Asp Arg Val Leu Lys Asn Thr Glu Val Gln Ala Ile Ile Thr Ala
385                 390                 395                 400

Leu Gly Thr Gly Ile His Asp Glu Phe Asp Ile Thr Lys Leu Arg Tyr
                405                 410                 415

His Lys Ile Val Leu
            420


<210> 21
<211> 1257
<212> DNA
<213> Mycobacterium africanum

<220>
<221> CDS
<222> (1)..(1257)

<400> 21
tcg gac gcg tat gcg ata tct ggt ggt ctg cac ggc gtc ggc gtg tcg      48
Ser Asp Ala Tyr Ala Ile Ser Gly Gly Leu His Gly Val Gly Val Ser
1               5                   10                  15

gtg gtt aac gcg cta tcc acc cgg ctc gaa gtc gag atc aag cgc gac      96
Val Val Asn Ala Leu Ser Thr Arg Leu Glu Val Glu Ile Lys Arg Asp
            20                  25                  30

ggg tac gag tgg tct cag gtt tat gag aag tcg gaa ccc ctg ggc ctc      144
Gly Tyr Glu Trp Ser Gln Val Tyr Glu Lys Ser Glu Pro Leu Gly Leu
        35                  40                  45

aag caa ggg gcg ccg acc aag aag acg ggg tca acg gtg cgg ttc tgg      192
Lys Gln Gly Ala Pro Thr Lys Lys Thr Gly Ser Thr Val Arg Phe Trp
    50                  55                  60

gcc gac ccc gct gtt ttc gaa acc acg gaa tac gac ttc gaa acc gtc      240
Ala Asp Pro Ala Val Phe Glu Thr Thr Glu Tyr Asp Phe Glu Thr Val
65                  70                  75                  80

gcc cgc cgg ctg caa gag atg gcg ttc ctc aac aag ggg ctg acc atc      288
Ala Arg Arg Leu Gln Glu Met Ala Phe Leu Asn Lys Gly Leu Thr Ile

                85                  90                  95

aac ctg acc gac gag agg gtg acc caa gac gag gtc gtc gac gaa gtg 336
Asn Leu Thr Asp Glu Arg Val Thr Gln Asp Glu Val Val Asp Glu Val
            100                 105                 110

gtc agc gac gtc gcc gag gcg ccg aag tcg gca agt gaa cgc gca gcc 384
Val Ser Asp Val Ala Glu Ala Pro Lys Ser Ala Ser Glu Arg Ala Ala
        115                 120                 125

gaa tcc act gca ccg cac aaa gtt aag agc cgc acc ttt cac tat ccg 432
Glu Ser Thr Ala Pro His Lys Val Lys Ser Arg Thr Phe His Tyr Pro
    130                 135                 140

ggt ggc ctg gtg gac ttc gtg aaa cac atc aac cgc acc aag aac gcg 480
Gly Gly Leu Val Asp Phe Val Lys His Ile Asn Arg Thr Lys Asn Ala
145                 150                 155                 160

att cat agc agc atc gtg gac ttt tcc ggc aag ggc acc ggg cac gag 528
Ile His Ser Ser Ile Val Asp Phe Ser Gly Lys Gly Thr Gly His Glu
                165                 170                 175

gtg gag atc gcg atg caa tgg aac gcc ggg tat tcg gag tcg gtg cac 576
Val Glu Ile Ala Met Gln Trp Asn Ala Gly Tyr Ser Glu Ser Val His
            180                 185                 190

acc ttc gcc aac acc atc aac acc cac gag ggc ggc acc cac gaa gag 624
Thr Phe Ala Asn Thr Ile Asn Thr His Glu Gly Gly Thr His Glu Glu
        195                 200                 205

ggc ttc cgc agc gcg ctg acg tcg gtg gtg aac aag tac gcc aag gac 672
Gly Phe Arg Ser Ala Leu Thr Ser Val Val Asn Lys Tyr Ala Lys Asp
    210                 215                 220

cgc aag cta ctg aag gac aag gac ccc aac ctc acc ggt gac gat atc 720
Arg Lys Leu Leu Lys Asp Lys Asp Pro Asn Leu Thr Gly Asp Asp Ile
225                 230                 235                 240

cgg gaa ggc ctg gcc gct gtg atc tcg gtg aag gtc agc gaa ccg cag 768
Arg Glu Gly Leu Ala Ala Val Ile Ser Val Lys Val Ser Glu Pro Gln
                245                 250                 255

ttc gag ggc cag acc aag acc aag ttg ggc aac acc gag gtc aaa tcg 816
Phe Glu Gly Gln Thr Lys Thr Lys Leu Gly Asn Thr Glu Val Lys Ser
            260                 265                 270

ttt gtg cag aag gtc tgt aac gaa cag ctg acc cac tgg ttt gaa gcc 864
Phe Val Gln Lys Val Cys Asn Glu Gln Leu Thr His Trp Phe Glu Ala
        275                 280                 285

aac ccc acc gac tcg aaa gtc gtt gtg aac aag gct gtg tcc tcg gcg 912
Asn Pro Thr Asp Ser Lys Val Val Val Asn Lys Ala Val Ser Ser Ala
    290                 295                 300

caa gcc cgt atc gcg gca cgt aag gca cga gag ttg gtg cgg cgt aag 960
Gln Ala Arg Ile Ala Ala Arg Lys Ala Arg Glu Leu Val Arg Arg Lys

305                 310                 315                 320

agc gcc acc gac atc ggt gga ttg ccc ggc aag ctg gcc gat tgc cgt      1008
Ser Ala Thr Asp Ile Gly Gly Leu Pro Gly Lys Leu Ala Asp Cys Arg
                325                 330                 335

tcc acg gat ccg cgc aag tcc gaa ctg tat gtc gta gaa ggt gac tcg      1056
Ser Thr Asp Pro Arg Lys Ser Glu Leu Tyr Val Val Glu Gly Asp Ser
            340                 345                 350

gcc ggc ggt tct gca aaa agc ggt cgc gat tcg atg ttc cag gcg ata      1104
Ala Gly Gly Ser Ala Lys Ser Gly Arg Asp Ser Met Phe Gln Ala Ile
        355                 360                 365

ctt ccg ctg cgc ggc aag atc atc aat gtg gag aaa gcg cgc atc gac      1152
Leu Pro Leu Arg Gly Lys Ile Ile Asn Val Glu Lys Ala Arg Ile Asp
    370                 375                 380

cgg gtg cta aag aac acc gaa gtt cag gcg atc atc acg gcg ctg ggc      1200
Arg Val Leu Lys Asn Thr Glu Val Gln Ala Ile Ile Thr Ala Leu Gly
385                 390                 395                 400

acc ggg atc cac gac gag ttc gat atc ggc aag ctg cgc tac cac aag      1248
Thr Gly Ile His Asp Glu Phe Asp Ile Gly Lys Leu Arg Tyr His Lys
                405                 410                 415

atc gtg ctg                                                          1257
Ile Val Leu


<210> 22
<211> 419
<212> PRT
<213> Mycobacterium africanum

<400> 22
Ser Asp Ala Tyr Ala Ile Ser Gly Gly Leu His Gly Val Gly Val Ser
1               5                   10                  15

Val Val Asn Ala Leu Ser Thr Arg Leu Glu Val Glu Ile Lys Arg Asp
            20                  25                  30

Gly Tyr Glu Trp Ser Gln Val Tyr Glu Lys Ser Glu Pro Leu Gly Leu
        35                  40                  45

Lys Gln Gly Ala Pro Thr Lys Lys Thr Gly Ser Thr Val Arg Phe Trp
    50                  55                  60

Ala Asp Pro Ala Val Phe Glu Thr Thr Glu Tyr Asp Phe Glu Thr Val
65                  70                  75                  80

Ala Arg Arg Leu Gln Glu Met Ala Phe Leu Asn Lys Gly Leu Thr Ile
                85                  90                  95

Asn Leu Thr Asp Glu Arg Val Thr Gln Asp Glu Val Val Asp Glu Val
            100                 105                 110

Val Ser Asp Val Ala Glu Ala Pro Lys Ser Ala Ser Glu Arg Ala Ala
        115                 120                 125

Glu Ser Thr Ala Pro His Lys Val Lys Ser Arg Thr Phe His Tyr Pro
    130                 135                 140

Gly Gly Leu Val Asp Phe Val Lys His Ile Asn Arg Thr Lys Asn Ala
145                 150                 155                 160

Ile His Ser Ser Ile Val Asp Phe Ser Gly Lys Gly Thr Gly His Glu
                165                 170                 175

Val Glu Ile Ala Met Gln Trp Asn Ala Gly Tyr Ser Glu Ser Val His
            180                 185                 190

Thr Phe Ala Asn Thr Ile Asn Thr His Glu Gly Gly Thr His Glu Glu
        195                 200                 205

Gly Phe Arg Ser Ala Leu Thr Ser Val Val Asn Lys Tyr Ala Lys Asp
    210                 215                 220

Arg Lys Leu Leu Lys Asp Lys Asp Pro Asn Leu Thr Gly Asp Asp Ile
225                 230                 235                 240

Arg Glu Gly Leu Ala Ala Val Ile Ser Val Lys Val Ser Glu Pro Gln
                245                 250                 255

Phe Glu Gly Gln Thr Lys Thr Lys Leu Gly Asn Thr Glu Val Lys Ser
            260                 265                 270

Phe Val Gln Lys Val Cys Asn Glu Gln Leu Thr His Trp Phe Glu Ala
        275                 280                 285

Asn Pro Thr Asp Ser Lys Val Val Val Asn Lys Ala Val Ser Ser Ala
    290                 295                 300

Gln Ala Arg Ile Ala Ala Arg Lys Ala Arg Glu Leu Val Arg Arg Lys
305                 310                 315                 320

Ser Ala Thr Asp Ile Gly Gly Leu Pro Gly Lys Leu Ala Asp Cys Arg
                325                 330                 335

Ser Thr Asp Pro Arg Lys Ser Glu Leu Tyr Val Val Glu Gly Asp Ser
            340                 345                 350

Ala Gly Gly Ser Ala Lys Ser Gly Arg Asp Ser Met Phe Gln Ala Ile
        355                 360                 365

Leu Pro Leu Arg Gly Lys Ile Ile Asn Val Glu Lys Ala Arg Ile Asp
    370                 375                 380

Arg Val Leu Lys Asn Thr Glu Val Gln Ala Ile Ile Thr Ala Leu Gly
385                 390                 395                 400

Thr Gly Ile His Asp Glu Phe Asp Ile Gly Lys Leu Arg Tyr His Lys

                405                 410                 415

Ile Val Leu

<210> 23
<211> 1257
<212> DNA
<213> Mycobacterium tuberculosis

<220>
<221> CDS
<222> (1)..(1257)

<400> 23
tcg gac gcg tat gcg ata tct ggt ggt ctg cac ggc gtc ggc gtg tcg 48
Ser Asp Ala Tyr Ala Ile Ser Gly Gly Leu His Gly Val Gly Val Ser
1               5                   10                  15

gtg gtt aac gcg cta tcc acc cgg ctc gaa gtc gag atc aag cgc gac 96
Val Val Asn Ala Leu Ser Thr Arg Leu Glu Val Glu Ile Lys Arg Asp
            20                  25                  30

ggg tac gag tgg tct cag gtt tat gag aag tcg gaa ccc ctg ggc ctc 144
Gly Tyr Glu Trp Ser Gln Val Tyr Glu Lys Ser Glu Pro Leu Gly Leu
        35                  40                  45

aag caa ggg gcg ccg acc aag aag acg ggg tca acg gtg cgg ttc tgg 192
Lys Gln Gly Ala Pro Thr Lys Lys Thr Gly Ser Thr Val Arg Phe Trp
    50                  55                  60

gcc gac ccc gct gtt ttc gaa acc acg gaa tac gac ttc gaa acc gtc 240
Ala Asp Pro Ala Val Phe Glu Thr Thr Glu Tyr Asp Phe Glu Thr Val
65                  70                  75                  80

gcc cgc cgg ctg caa gag atg gcg ttc ctc aac aag ggg ctg acc atc 288
Ala Arg Arg Leu Gln Glu Met Ala Phe Leu Asn Lys Gly Leu Thr Ile
                85                  90                  95

aac ctg acc gac gag agg gtg acc caa gac gag gtc gtc gac gaa gtg 336
Asn Leu Thr Asp Glu Arg Val Thr Gln Asp Glu Val Val Asp Glu Val
            100                 105                 110

gtc agc gac gtc gcc gag gcg ccg aag tcg gca agt gaa cgc gca gcc 384
Val Ser Asp Val Ala Glu Ala Pro Lys Ser Ala Ser Glu Arg Ala Ala
        115                 120                 125

gaa tcc act gca ccg cac aaa gtt aag agc cgc acc ttt cac tat ccg 432
Glu Ser Thr Ala Pro His Lys Val Lys Ser Arg Thr Phe His Tyr Pro
    130                 135                 140

ggt ggc ctg gtg gac ttc gtg aaa cac atc aac cgc acc aag aac gcg 480
Gly Gly Leu Val Asp Phe Val Lys His Ile Asn Arg Thr Lys Asn Ala
145                 150                 155                 160

att cat agc agc atc gtg gac ttt tcc ggc aag ggc acc ggg cac gag 528
Ile His Ser Ser Ile Val Asp Phe Ser Gly Lys Gly Thr Gly His Glu
                165                 170                 175

gtg gag atc gcg atg caa tgg aac gcc ggg tat tcg gag tcg gtg cac 576
Val Glu Ile Ala Met Gln Trp Asn Ala Gly Tyr Ser Glu Ser Val His
            180                 185                 190

acc ttc gcc aac acc atc aac acc cac gag ggc ggc acc cac gaa gag 624
Thr Phe Ala Asn Thr Ile Asn Thr His Glu Gly Gly Thr His Glu Glu
        195                 200                 205

ggc ttc cgc agc gcg ctg acg tcg gtg gtg aac aag tac gcc aag gac 672
Gly Phe Arg Ser Ala Leu Thr Ser Val Val Asn Lys Tyr Ala Lys Asp
    210                 215                 220

cgc aag cta ctg aag gac aag gac ccc aac ctc acc ggt gac gat atc 720
Arg Lys Leu Leu Lys Asp Lys Asp Pro Asn Leu Thr Gly Asp Asp Ile
225                 230                 235                 240

cgg gaa ggc ctg gcc gct gtg atc tcg gtg aag gtc agc gaa ccg cag 768
Arg Glu Gly Leu Ala Ala Val Ile Ser Val Lys Val Ser Glu Pro Gln
                245                 250                 255

ttc gag ggc cag acc aag acc aag ttg ggc aac acc gag gtc aaa tcg 816
Phe Glu Gly Gln Thr Lys Thr Lys Leu Gly Asn Thr Glu Val Lys Ser
            260                 265                 270

ttt gtg cag aag gtc tgt aac gaa cag ctg acc cac tgg ttt gaa gcc 864
Phe Val Gln Lys Val Cys Asn Glu Gln Leu Thr His Trp Phe Glu Ala
        275                 280                 285

aac ccc acc gac gcg aaa gtc gtt gtg aac aag gct gtg tcc tcg gcg 912
Asn Pro Thr Asp Ala Lys Val Val Val Asn Lys Ala Val Ser Ser Ala
    290                 295                 300

caa gcc cgt atc gcg gca cgt aag gca cga gag ttg gtg cgg cgt aag 960
Gln Ala Arg Ile Ala Ala Arg Lys Ala Arg Glu Leu Val Arg Arg Lys
305                 310                 315                 320

agc gcc acc gac atc ggt gga ttg ccc ggc aag ctg gcc gat tgc cgt 1008
Ser Ala Thr Asp Ile Gly Gly Leu Pro Gly Lys Leu Ala Asp Cys Arg
                325                 330                 335

tcc acg gat ccg cgc aag tcc gaa ctg tat gtc gta gaa ggt gac tcg 1056
Ser Thr Asp Pro Arg Lys Ser Glu Leu Tyr Val Val Glu Gly Asp Ser
            340                 345                 350

gcc ggc ggt tct gca aaa agc ggt cgc gat tcg atg ttc cag gcg ata 1104
Ala Gly Gly Ser Ala Lys Ser Gly Arg Asp Ser Met Phe Gln Ala Ile
        355                 360                 365

ctt ccg ctg cgc ggc aag atc atc aat gtg gag aaa gcg cgc atc gac 1152
Leu Pro Leu Arg Gly Lys Ile Ile Asn Val Glu Lys Ala Arg Ile Asp
    370                 375                 380

cgg gtg cta aag aac acc gaa gtt cag gcg atc atc acg gcg ctg ggc     1200
Arg Val Leu Lys Asn Thr Glu Val Gln Ala Ile Ile Thr Ala Leu Gly
385                 390                 395                 400

acc ggg atc cac gac gag ttc gat atc ggc aag ctg cgc tac cac aag     1248
Thr Gly Ile His Asp Glu Phe Asp Ile Gly Lys Leu Arg Tyr His Lys
                405                 410                 415

atc gtg ctg                                                         1257
Ile Val Leu

<210> 24
<211> 419
<212> PRT
<213> Mycobacterium tuberculosis

<400> 24
Ser Asp Ala Tyr Ala Ile Ser Gly Gly Leu His Gly Val Gly Val Ser
  1               5                  10                  15

Val Val Asn Ala Leu Ser Thr Arg Leu Glu Val Glu Ile Lys Arg Asp
             20                  25                  30

Gly Tyr Glu Trp Ser Gln Val Tyr Glu Lys Ser Glu Pro Leu Gly Leu
         35                  40                  45

Lys Gln Gly Ala Pro Thr Lys Lys Thr Gly Ser Thr Val Arg Phe Trp
     50                  55                  60

Ala Asp Pro Ala Val Phe Glu Thr Thr Glu Tyr Asp Phe Glu Thr Val
 65                  70                  75                  80

Ala Arg Arg Leu Gln Glu Met Ala Phe Leu Asn Lys Gly Leu Thr Ile
                 85                  90                  95

Asn Leu Thr Asp Glu Arg Val Thr Gln Asp Glu Val Val Asp Glu Val
            100                 105                 110

Val Ser Asp Val Ala Glu Ala Pro Lys Ser Ala Ser Glu Arg Ala Ala
        115                 120                 125

Glu Ser Thr Ala Pro His Lys Val Lys Ser Arg Thr Phe His Tyr Pro
    130                 135                 140

Gly Gly Leu Val Asp Phe Val Lys His Ile Asn Arg Thr Lys Asn Ala
145                 150                 155                 160

Ile His Ser Ser Ile Val Asp Phe Ser Gly Lys Gly Thr Gly His Glu
                165                 170                 175

Val Glu Ile Ala Met Gln Trp Asn Ala Gly Tyr Ser Glu Ser Val His
            180                 185                 190

Thr Phe Ala Asn Thr Ile Asn Thr His Glu Gly Gly Thr His Glu Glu
        195                 200                 205

Gly Phe Arg Ser Ala Leu Thr Ser Val Val Asn Lys Tyr Ala Lys Asp
    210                 215                 220

Arg Lys Leu Leu Lys Asp Lys Asp Pro Asn Leu Thr Gly Asp Asp Ile
225                 230                 235                 240

Arg Glu Gly Leu Ala Ala Val Ile Ser Val Lys Val Ser Glu Pro Gln
                245                 250                 255

Phe Glu Gly Gln Thr Lys Thr Lys Leu Gly Asn Thr Glu Val Lys Ser
            260                 265                 270

Phe Val Gln Lys Val Cys Asn Glu Gln Leu Thr His Trp Phe Glu Ala
        275                 280                 285

Asn Pro Thr Asp Ala Lys Val Val Val Asn Lys Ala Val Ser Ser Ala
    290                 295                 300

Gln Ala Arg Ile Ala Ala Arg Lys Ala Arg Glu Leu Val Arg Arg Lys
305                 310                 315                 320

Ser Ala Thr Asp Ile Gly Gly Leu Pro Gly Lys Leu Ala Asp Cys Arg
                325                 330                 335

Ser Thr Asp Pro Arg Lys Ser Glu Leu Tyr Val Val Glu Gly Asp Ser
            340                 345                 350

Ala Gly Gly Ser Ala Lys Ser Gly Arg Asp Ser Met Phe Gln Ala Ile
        355                 360                 365

Leu Pro Leu Arg Gly Lys Ile Ile Asn Val Glu Lys Ala Arg Ile Asp
    370                 375                 380

Arg Val Leu Lys Asn Thr Glu Val Gln Ala Ile Ile Thr Ala Leu Gly
385                 390                 395                 400

Thr Gly Ile His Asp Glu Phe Asp Ile Gly Lys Leu Arg Tyr His Lys
                405                 410                 415

Ile Val Leu

<210> 25
<211> 1257
<212> DNA
<213> Mycobacterium gastri

<220>
<221> CDS
<222> (1)..(1257)

<400> 25
tcc gac gcc tat gcg ata tcg ggt gga ctg cac ggt gtg ggt gtc tcg      48
Ser Asp Ala Tyr Ala Ile Ser Gly Gly Leu His Gly Val Gly Val Ser

1                 5                   10                  15

gtg gtc aac gcg ctg tcc atc cgg ctg gag gtg gag atc aag cgc gac    96
Val Val Asn Ala Leu Ser Ile Arg Leu Glu Val Glu Ile Lys Arg Asp
            20                  25                  30

ggc cat gag tgg tcg caa gtt tat gag aag tcc gag ccg atg gga ctc    144
Gly His Glu Trp Ser Gln Val Tyr Glu Lys Ser Glu Pro Met Gly Leu
        35                  40                  45

aag caa ggc gcg ccg acg aag aag acc ggc acg acg gtg cgg ttc tgg    192
Lys Gln Gly Ala Pro Thr Lys Lys Thr Gly Thr Thr Val Arg Phe Trp
    50                  55                  60

gcc gac ccc aac gtt ttt gaa acc acc gag tac gac ttc gaa acc gtc    240
Ala Asp Pro Asn Val Phe Glu Thr Thr Glu Tyr Asp Phe Glu Thr Val
65                  70                  75                  80

gcg cga cgg ttg cag gag atg gcg ttt ctc aac aag ggg ctc acc atc    288
Ala Arg Arg Leu Gln Glu Met Ala Phe Leu Asn Lys Gly Leu Thr Ile
                85                  90                  95

aac ctg acc gat cag cgg gta acc cag gac gaa gtg gtc gac gag gtg    336
Asn Leu Thr Asp Gln Arg Val Thr Gln Asp Glu Val Val Asp Glu Val
            100                 105                 110

gtc agc gac gtc gcc gag gcc ccg aag tcg gcc agt gag aag gcg gcc    384
Val Ser Asp Val Ala Glu Ala Pro Lys Ser Ala Ser Glu Lys Ala Ala
        115                 120                 125

gaa ttc acc gcc ccc cac aag gtg aag aag cgt acc ttt cac tat ccc    432
Glu Phe Thr Ala Pro His Lys Val Lys Lys Arg Thr Phe His Tyr Pro
    130                 135                 140

ggt ggc ttg gtt gac ttc gtc aag cac atc aac cgc acc aag aac gcc    480
Gly Gly Leu Val Asp Phe Val Lys His Ile Asn Arg Thr Lys Asn Ala
145                 150                 155                 160

atc cac agc agc atc gtc gac ttc tcc gga aag ggg acc ggc cac gaa    528
Ile His Ser Ser Ile Val Asp Phe Ser Gly Lys Gly Thr Gly His Glu
                165                 170                 175

gtg gag atc gcg atg cag tgg aat gcc ggc tat tcg gag tcg gtg cac    576
Val Glu Ile Ala Met Gln Trp Asn Ala Gly Tyr Ser Glu Ser Val His
            180                 185                 190

acc ttc gcc aac acc atc aac acc cat gag ggc ggg acc cat gaa gaa    624
Thr Phe Ala Asn Thr Ile Asn Thr His Glu Gly Gly Thr His Glu Glu
        195                 200                 205

ggg ttc cgc agc gcg ctc acg tcc gtg gtg aac aag tac gcc aag gac    672
Gly Phe Arg Ser Ala Leu Thr Ser Val Val Asn Lys Tyr Ala Lys Asp
    210                 215                 220

cgc aaa ctg ctc aaa gac aag gac ccc aac ctc acc ggc gac gac atc    720
Arg Lys Leu Leu Lys Asp Lys Asp Pro Asn Leu Thr Gly Asp Asp Ile

225                 230                 235                 240

cgg gaa ggg ttg gcc gcg gtg att tcg gtc aaa gtc agc gaa ccg cag     768
Arg Glu Gly Leu Ala Ala Val Ile Ser Val Lys Val Ser Glu Pro Gln
                245                 250                 255

ttc gag ggc cag acc aag acg aaa cta ggc aac acc gag gtg aag tcg     816
Phe Glu Gly Gln Thr Lys Thr Lys Leu Gly Asn Thr Glu Val Lys Ser
            260                 265                 270

ttc gtg cag aag gtg tgc aat gaa cag ctc acc cat tgg ttc gag gcc     864
Phe Val Gln Lys Val Cys Asn Glu Gln Leu Thr His Trp Phe Glu Ala
        275                 280                 285

aac ccc gct gat gct aaa acc gtt gtc aac aag gca gtt tca tcg gcg     912
Asn Pro Ala Asp Ala Lys Thr Val Val Asn Lys Ala Val Ser Ser Ala
    290                 295                 300

cag gcc agg att gcg gcc cgc aag gcg cgc gag ttg gtg cgc cgc aag     960
Gln Ala Arg Ile Ala Ala Arg Lys Ala Arg Glu Leu Val Arg Arg Lys
305                 310                 315                 320

agc gca acc gat ctg ggc gga cta ccg ggc aag ttg gcc gac tgc cgc     1008
Ser Ala Thr Asp Leu Gly Gly Leu Pro Gly Lys Leu Ala Asp Cys Arg
                325                 330                 335

tcg acc gac ccc cgt aag tcc gaa tta tat gtg gtg gag ggt gat tca     1056
Ser Thr Asp Pro Arg Lys Ser Glu Leu Tyr Val Val Glu Gly Asp Ser
            340                 345                 350

gcc ggc ggc tcg gcg aag agc ggc cgc gac tcg atg ttt caa gcg atc     1104
Ala Gly Gly Ser Ala Lys Ser Gly Arg Asp Ser Met Phe Gln Ala Ile
        355                 360                 365

ttg ccg ttg cgc ggc aag atc atc aac gtc gag aag gcc cgc atc gac     1152
Leu Pro Leu Arg Gly Lys Ile Ile Asn Val Glu Lys Ala Arg Ile Asp
    370                 375                 380

cgg gtg ctg aag aac acc gaa gtc cag gcg atc atc acc gcg ttg ggc     1200
Arg Val Leu Lys Asn Thr Glu Val Gln Ala Ile Ile Thr Ala Leu Gly
385                 390                 395                 400

acc ggt att cac gac gaa ttc gac atc gcg aga ctg cgt tac cac aag     1248
Thr Gly Ile His Asp Glu Phe Asp Ile Ala Arg Leu Arg Tyr His Lys
                405                 410                 415

atc gtg ctg                                                         1257
Ile Val Leu


<210> 26
<211> 419
<212> PRT
<213> Mycobacterium gastri

<400> 26

Ser Asp Ala Tyr Ala Ile Ser Gly Gly Leu His Gly Val Gly Val Ser
1               5                   10                  15

Val Val Asn Ala Leu Ser Ile Arg Leu Glu Val Glu Ile Lys Arg Asp
            20                  25                  30

Gly His Glu Trp Ser Gln Val Tyr Glu Lys Ser Glu Pro Met Gly Leu
        35                  40                  45

Lys Gln Gly Ala Pro Thr Lys Lys Thr Gly Thr Thr Val Arg Phe Trp
    50                  55                  60

Ala Asp Pro Asn Val Phe Glu Thr Thr Glu Tyr Asp Phe Glu Thr Val
65                  70                  75                  80

Ala Arg Arg Leu Gln Glu Met Ala Phe Leu Asn Lys Gly Leu Thr Ile
                85                  90                  95

Asn Leu Thr Asp Gln Arg Val Thr Gln Asp Glu Val Val Asp Glu Val
            100                 105                 110

Val Ser Asp Val Ala Glu Ala Pro Lys Ser Ala Ser Glu Lys Ala Ala
        115                 120                 125

Glu Phe Thr Ala Pro His Lys Val Lys Lys Arg Thr Phe His Tyr Pro
    130                 135                 140

Gly Gly Leu Val Asp Phe Val Lys His Ile Asn Arg Thr Lys Asn Ala
145                 150                 155                 160

Ile His Ser Ser Ile Val Asp Phe Ser Gly Lys Gly Thr Gly His Glu
                165                 170                 175

Val Glu Ile Ala Met Gln Trp Asn Ala Gly Tyr Ser Glu Ser Val His
            180                 185                 190

Thr Phe Ala Asn Thr Ile Asn Thr His Glu Gly Gly Thr His Glu Glu
        195                 200                 205

Gly Phe Arg Ser Ala Leu Thr Ser Val Val Asn Lys Tyr Ala Lys Asp
    210                 215                 220

Arg Lys Leu Leu Lys Asp Lys Asp Pro Asn Leu Thr Gly Asp Asp Ile
225                 230                 235                 240

Arg Glu Gly Leu Ala Ala Val Ile Ser Val Lys Val Ser Glu Pro Gln
                245                 250                 255

Phe Glu Gly Gln Thr Lys Thr Lys Leu Gly Asn Thr Glu Val Lys Ser
            260                 265                 270

Phe Val Gln Lys Val Cys Asn Glu Gln Leu Thr His Trp Phe Glu Ala
        275                 280                 285

Asn Pro Ala Asp Ala Lys Thr Val Val Asn Lys Ala Val Ser Ser Ala
    290                 295                 300

Gln Ala Arg Ile Ala Ala Arg Lys Ala Arg Glu Leu Val Arg Arg Lys
305                 310                 315                 320

Ser Ala Thr Asp Leu Gly Gly Leu Pro Gly Lys Leu Ala Asp Cys Arg
                325                 330                 335

Ser Thr Asp Pro Arg Lys Ser Glu Leu Tyr Val Val Glu Gly Asp Ser
            340                 345                 350

Ala Gly Gly Ser Ala Lys Ser Gly Arg Asp Ser Met Phe Gln Ala Ile
        355                 360                 365

Leu Pro Leu Arg Gly Lys Ile Ile Asn Val Glu Lys Ala Arg Ile Asp
    370                 375                 380

Arg Val Leu Lys Asn Thr Glu Val Gln Ala Ile Ile Thr Ala Leu Gly
385                 390                 395                 400

Thr Gly Ile His Asp Glu Phe Asp Ile Ala Arg Leu Arg Tyr His Lys
                405                 410                 415

Ile Val Leu

<210> 27
<211> 1263
<212> DNA
<213> Mycobacterium marinum

<220>
<221> CDS
<222> (1)..(1263)

<400> 27
ggc gag aac agt ggt tac aac gtc agt ggt ggt ctg cac ggc gtg ggt      48
Gly Glu Asn Ser Gly Tyr Asn Val Ser Gly Gly Leu His Gly Val Gly
1               5                   10                  15

gtg tcg gtg gtc aac gcg ctg tcc acc cga ctg gaa gtc gac atc aag      96
Val Ser Val Val Asn Ala Leu Ser Thr Arg Leu Glu Val Asp Ile Lys
            20                  25                  30

cgc gac gga tac gag tgg tcg cag ttc tac gac cgc gcc cag ccg ggc      144
Arg Asp Gly Tyr Glu Trp Ser Gln Phe Tyr Asp Arg Ala Gln Pro Gly
        35                  40                  45

acc ctc aaa cag ggc gag gca acc aag aag acc gga acc acc atc cgg      192
Thr Leu Lys Gln Gly Glu Ala Thr Lys Lys Thr Gly Thr Thr Ile Arg
    50                  55                  60

ttc tgg gcc gac tcg gac atc ttt gag acc acc gaa tac gac ttc gag      240
Phe Trp Ala Asp Ser Asp Ile Phe Glu Thr Thr Glu Tyr Asp Phe Glu
65                  70                  75                  80

acg gtg gcg cgg cgc ctg cag gag atg gcg ttc ctc aac aag ggc ctg 288
Thr Val Ala Arg Arg Leu Gln Glu Met Ala Phe Leu Asn Lys Gly Leu
                85                  90                  95

acc atc aac ctc acc gac gag cgg gtc acc ccg gac gag gtc gtc gac 336
Thr Ile Asn Leu Thr Asp Glu Arg Val Thr Pro Asp Glu Val Val Asp
            100                 105                 110

gac gtc gtc agt gat acc gcc gaa gca cca aag tcc gcc cag gag aag 384
Asp Val Val Ser Asp Thr Ala Glu Ala Pro Lys Ser Ala Gln Glu Lys
        115                 120                 125

gcc gcc gaa tcg acc gcg ccg cac aag gtc aag agc cgc acc ttc cac 432
Ala Ala Glu Ser Thr Ala Pro His Lys Val Lys Ser Arg Thr Phe His
    130                 135                 140

tat ccc ggc ggt ttg gtc gat ttc gtc aag cac atc aac cgc acc aag 480
Tyr Pro Gly Gly Leu Val Asp Phe Val Lys His Ile Asn Arg Thr Lys
145                 150                 155                 160

agt ccg att cag cag agc atc gtc gac ttc gag ggc aag ggc tcc ggc 528
Ser Pro Ile Gln Gln Ser Ile Val Asp Phe Glu Gly Lys Gly Ser Gly
                165                 170                 175

cac gaa gtc gaa atc gcg atg cag tgg aac ggc ggc tac tcg gag tcg 576
His Glu Val Glu Ile Ala Met Gln Trp Asn Gly Gly Tyr Ser Glu Ser
            180                 185                 190

gtg cac acc ttc gcc aac acc atc aac acc cat gag ggt gga acg cac 624
Val His Thr Phe Ala Asn Thr Ile Asn Thr His Glu Gly Gly Thr His
        195                 200                 205

gaa gag ggc ttc cgc agt gcg ttg acc tcg gtg gtg aac aag tac gcc 672
Glu Glu Gly Phe Arg Ser Ala Leu Thr Ser Val Val Asn Lys Tyr Ala
    210                 215                 220

aaa gac aag aag ctg ctc aag gac aag gac ccc aac ctc acc ggt gac 720
Lys Asp Lys Lys Leu Leu Lys Asp Lys Asp Pro Asn Leu Thr Gly Asp
225                 230                 235                 240

gac atc cgc gag ggg ttg gcc gcg gtc atc tcg gtg cgg gtg gca gag 768
Asp Ile Arg Glu Gly Leu Ala Ala Val Ile Ser Val Arg Val Ala Glu
                245                 250                 255

ccg cag ttc gag ggt cag acg aag acc aag ctg ggc aac acc gag gtc 816
Pro Gln Phe Glu Gly Gln Thr Lys Thr Lys Leu Gly Asn Thr Glu Val
            260                 265                 270

aag tcg ttt gtc cag aag gtt tgt aac gag cag ctc acc cac tgg ttc 864
Lys Ser Phe Val Gln Lys Val Cys Asn Glu Gln Leu Thr His Trp Phe
        275                 280                 285

gag gcc aat cct tcg gaa gcc aaa acc att gtg aac aag gcg gta tcc 912
Glu Ala Asn Pro Ser Glu Ala Lys Thr Ile Val Asn Lys Ala Val Ser
    290                 295                 300

tcg gcg cag gca cgt ctc gcc gcg cgc aag gcg cga gag ttg gtg cgt     960
Ser Ala Gln Ala Arg Leu Ala Ala Arg Lys Ala Arg Glu Leu Val Arg
305                 310                 315                 320

cgc aag agc gca acc gat ctc ggt ggg ctg ccc ggc aag ttg gcc gac     1008
Arg Lys Ser Ala Thr Asp Leu Gly Gly Leu Pro Gly Lys Leu Ala Asp
                325                 330                 335

tgc cgc tcg aca gat ccg cgt aag tcg gaa ctg tat gtg gtg gag ggt     1056
Cys Arg Ser Thr Asp Pro Arg Lys Ser Glu Leu Tyr Val Val Glu Gly
            340                 345                 350

gac tcg gcc ggc ggc tcg gca aag agt ggc cgc gat tcg atg ttc cag     1104
Asp Ser Ala Gly Gly Ser Ala Lys Ser Gly Arg Asp Ser Met Phe Gln
        355                 360                 365

gcg atc ctg ccg ctg cgc ggc aag atc atc aat gtc gaa aag gca cgc     1152
Ala Ile Leu Pro Leu Arg Gly Lys Ile Ile Asn Val Glu Lys Ala Arg
    370                 375                 380

atc gac cga gtc ctg aaa aac act gaa gtc cag gcg atc atc acc gcg     1200
Ile Asp Arg Val Leu Lys Asn Thr Glu Val Gln Ala Ile Ile Thr Ala
385                 390                 395                 400

ttg ggt acc ggt att cac gac gaa ttc gac ctc tcg aag ctg cgc tat     1248
Leu Gly Thr Gly Ile His Asp Glu Phe Asp Leu Ser Lys Leu Arg Tyr
                405                 410                 415

cac aag atc gtc ttg                                                 1263
His Lys Ile Val Leu
            420


<210> 28
<211> 421
<212> PRT
<213> Mycobacterium marinum

<400> 28
Gly Glu Asn Ser Gly Tyr Asn Val Ser Gly Gly Leu His Gly Val Gly
1               5                   10                  15

Val Ser Val Val Asn Ala Leu Ser Thr Arg Leu Glu Val Asp Ile Lys
            20                  25                  30

Arg Asp Gly Tyr Glu Trp Ser Gln Phe Tyr Asp Arg Ala Gln Pro Gly
        35                  40                  45

Thr Leu Lys Gln Gly Glu Ala Thr Lys Lys Thr Gly Thr Thr Ile Arg
    50                  55                  60

Phe Trp Ala Asp Ser Asp Ile Phe Glu Thr Thr Glu Tyr Asp Phe Glu
65                  70                  75                  80

Thr Val Ala Arg Arg Leu Gln Glu Met Ala Phe Leu Asn Lys Gly Leu
                85                  90                  95

Thr Ile Asn Leu Thr Asp Glu Arg Val Thr Pro Asp Glu Val Val Asp
            100                 105                 110

Asp Val Val Ser Asp Thr Ala Glu Ala Pro Lys Ser Ala Gln Glu Lys
        115                 120                 125

Ala Ala Glu Ser Thr Ala Pro His Lys Val Lys Ser Arg Thr Phe His
    130                 135                 140

Tyr Pro Gly Gly Leu Val Asp Phe Val Lys His Ile Asn Arg Thr Lys
145                 150                 155                 160

Ser Pro Ile Gln Gln Ser Ile Val Asp Phe Glu Gly Lys Gly Ser Gly
                165                 170                 175

His Glu Val Glu Ile Ala Met Gln Trp Asn Gly Gly Tyr Ser Glu Ser
            180                 185                 190

Val His Thr Phe Ala Asn Thr Ile Asn Thr His Glu Gly Gly Thr His
        195                 200                 205

Glu Glu Gly Phe Arg Ser Ala Leu Thr Ser Val Val Asn Lys Tyr Ala
    210                 215                 220

Lys Asp Lys Lys Leu Leu Lys Asp Lys Asp Pro Asn Leu Thr Gly Asp
225                 230                 235                 240

Asp Ile Arg Glu Gly Leu Ala Ala Val Ile Ser Val Arg Val Ala Glu
                245                 250                 255

Pro Gln Phe Glu Gly Gln Thr Lys Thr Lys Leu Gly Asn Thr Glu Val
            260                 265                 270

Lys Ser Phe Val Gln Lys Val Cys Asn Glu Gln Leu Thr His Trp Phe
        275                 280                 285

Glu Ala Asn Pro Ser Glu Ala Lys Thr Ile Val Asn Lys Ala Val Ser
    290                 295                 300

Ser Ala Gln Ala Arg Leu Ala Ala Arg Lys Ala Arg Glu Leu Val Arg
305                 310                 315                 320

Arg Lys Ser Ala Thr Asp Leu Gly Gly Leu Pro Gly Lys Leu Ala Asp
                325                 330                 335

Cys Arg Ser Thr Asp Pro Arg Lys Ser Glu Leu Tyr Val Val Glu Gly
            340                 345                 350

Asp Ser Ala Gly Gly Ser Ala Lys Ser Gly Arg Asp Ser Met Phe Gln
        355                 360                 365

Ala Ile Leu Pro Leu Arg Gly Lys Ile Ile Asn Val Glu Lys Ala Arg
    370                 375                 380

Ile Asp Arg Val Leu Lys Asn Thr Glu Val Gln Ala Ile Ile Thr Ala

385                 390                 395                 400

Leu Gly Thr Gly Ile His Asp Glu Phe Asp Leu Ser Lys Leu Arg Tyr
                405                 410                 415

His Lys Ile Val Leu
            420

<210> 29
<211> 1257
<212> DNA
<213> Mycobacterium microti

<220>
<221> CDS
<222> (1)..(1257)

<400> 29
tcg gac gcg tat gcg ata tct ggt ggt ctg cac ggc gtc ggc gtg tcg      48
Ser Asp Ala Tyr Ala Ile Ser Gly Gly Leu His Gly Val Gly Val Ser
1               5                   10                  15

gtg gtt aac gcg cta tcc acc cgg ctc gaa gtc gag atc aag cgc gac      96
Val Val Asn Ala Leu Ser Thr Arg Leu Glu Val Glu Ile Lys Arg Asp
            20                  25                  30

ggg tat gag tgg tct cag gtt tat gag aag tcg gaa ccc ctg ggc ctc      144
Gly Tyr Glu Trp Ser Gln Val Tyr Glu Lys Ser Glu Pro Leu Gly Leu
        35                  40                  45

aag caa ggg gcg ccg acc aag aag acg ggg tca acg gtg cgg ttc tgg      192
Lys Gln Gly Ala Pro Thr Lys Lys Thr Gly Ser Thr Val Arg Phe Trp
    50                  55                  60

gcc gac ccc gct gtt ttc gaa acc acg gaa tac gac ttc gaa acc gtc      240
Ala Asp Pro Ala Val Phe Glu Thr Thr Glu Tyr Asp Phe Glu Thr Val
65                  70                  75                  80

gcc cgc cgg ctg caa gag atg gcg ttc ctc aac aag ggg ctg acc atc      288
Ala Arg Arg Leu Gln Glu Met Ala Phe Leu Asn Lys Gly Leu Thr Ile
                85                  90                  95

aac ctg acc gac gag agg gtg acc caa gac gag gtc gtc gac gaa gtg      336
Asn Leu Thr Asp Glu Arg Val Thr Gln Asp Glu Val Val Asp Glu Val
            100                 105                 110

gtc agc gac gtc gcc gag gcg ccg aag tcg gca agt gaa cgc gca gcc      384
Val Ser Asp Val Ala Glu Ala Pro Lys Ser Ala Ser Glu Arg Ala Ala
        115                 120                 125

gaa tcc act gca ccg cac aaa gtt aag agc cgc acc ttt cac tat ccg      432
Glu Ser Thr Ala Pro His Lys Val Lys Ser Arg Thr Phe His Tyr Pro
    130                 135                 140

ggt ggc ctg gtg gac ttc gtg aaa cac atc aac cgc acc aag aac gcg      480

Gly Gly Leu Val Asp Phe Val Lys His Ile Asn Arg Thr Lys Asn Ala
145                 150                 155                 160

att cat agc agc atc gtg gac ttt tcc ggc aag ggc acc ggg cac gag     528
Ile His Ser Ser Ile Val Asp Phe Ser Gly Lys Gly Thr Gly His Glu
                165                 170                 175

gtg gag atc gcg atg caa tgg aac gcc ggg tat tcg gag tcg gtg cac     576
Val Glu Ile Ala Met Gln Trp Asn Ala Gly Tyr Ser Glu Ser Val His
            180                 185                 190

acc ttc gcc aac acc atc aac acc cac gag ggc ggc acc cac gaa gag     624
Thr Phe Ala Asn Thr Ile Asn Thr His Glu Gly Gly Thr His Glu Glu
        195                 200                 205

ggc ttc cgc agc gcg ctg acg tcg gtg gtg aac aag tac gcc aag gac     672
Gly Phe Arg Ser Ala Leu Thr Ser Val Val Asn Lys Tyr Ala Lys Asp
    210                 215                 220

cgc aag cta ctg aag gac aag gac ccc aac ctc acc ggt gac gat atc     720
Arg Lys Leu Leu Lys Asp Lys Asp Pro Asn Leu Thr Gly Asp Asp Ile
225                 230                 235                 240

cgg gaa ggc ctg gcc gct gtg atc tcg gtg aag gtc agc gaa ccg cag     768
Arg Glu Gly Leu Ala Ala Val Ile Ser Val Lys Val Ser Glu Pro Gln
                245                 250                 255

ttc gag ggc cag acc aag acc aag ttg ggc aac acc gag gtc aaa tcg     816
Phe Glu Gly Gln Thr Lys Thr Lys Leu Gly Asn Thr Glu Val Lys Ser
            260                 265                 270

ttt gtg cag aag gtc tgt aac gaa cag ctg acc cac tgg ttt gaa gcc     864
Phe Val Gln Lys Val Cys Asn Glu Gln Leu Thr His Trp Phe Glu Ala
        275                 280                 285

aac ccc acc gac tcg aaa gtc gtt gtg aac aag gct gtg tcc tcg gcg     912
Asn Pro Thr Asp Ser Lys Val Val Val Asn Lys Ala Val Ser Ser Ala
    290                 295                 300

caa gcc cgt atc gcg gca cgt aag gca cga gag ttg gtg cgg cgt aag     960
Gln Ala Arg Ile Ala Ala Arg Lys Ala Arg Glu Leu Val Arg Arg Lys
305                 310                 315                 320

agc gcc acc gac atc ggt gga ttg ccc ggc aag ctg gcc gat tgc cgt     1008
Ser Ala Thr Asp Ile Gly Gly Leu Pro Gly Lys Leu Ala Asp Cys Arg
                325                 330                 335

tcc acg gat ccg cgc aag tcc gaa ctg tat gtc gta gaa ggt gac tcg     1056
Ser Thr Asp Pro Arg Lys Ser Glu Leu Tyr Val Val Glu Gly Asp Ser
            340                 345                 350

gcc ggc ggt tct gca aaa agc ggt cgc gat tcg atg ttc cag gcg ata     1104
Ala Gly Gly Ser Ala Lys Ser Gly Arg Asp Ser Met Phe Gln Ala Ile
        355                 360                 365

ctt ccg ctg cgc ggc aag atc atc aat gtg gag aaa gcg cgc atc gac     1152

Leu Pro Leu Arg Gly Lys Ile Ile Asn Val Glu Lys Ala Arg Ile Asp
    370                 375                 380

cgg gtg cta aag aac acc gaa gtt cag gcg atc atc acg gcg ctg ggc     1200
Arg Val Leu Lys Asn Thr Glu Val Gln Ala Ile Ile Thr Ala Leu Gly
385                 390                 395                 400

acc ggg atc cac gac gag ttc gat atc ggc aag ctg cgc tac cac aag     1248
Thr Gly Ile His Asp Glu Phe Asp Ile Gly Lys Leu Arg Tyr His Lys
                405                 410                 415

atc gtg ctg                                                         1257
Ile Val Leu

<210> 30
<211> 419
<212> PRT
<213> Mycobacterium microti

<400> 30
Ser Asp Ala Tyr Ala Ile Ser Gly Gly Leu His Gly Val Gly Val Ser
  1               5                  10                  15

Val Val Asn Ala Leu Ser Thr Arg Leu Glu Val Glu Ile Lys Arg Asp
             20                  25                  30

Gly Tyr Glu Trp Ser Gln Val Tyr Glu Lys Ser Glu Pro Leu Gly Leu
         35                  40                  45

Lys Gln Gly Ala Pro Thr Lys Lys Thr Gly Ser Thr Val Arg Phe Trp
     50                  55                  60

Ala Asp Pro Ala Val Phe Glu Thr Thr Glu Tyr Asp Phe Glu Thr Val
 65                  70                  75                  80

Ala Arg Arg Leu Gln Glu Met Ala Phe Leu Asn Lys Gly Leu Thr Ile
                 85                  90                  95

Asn Leu Thr Asp Glu Arg Val Thr Gln Asp Glu Val Val Asp Glu Val
            100                 105                 110

Val Ser Asp Val Ala Glu Ala Pro Lys Ser Ala Ser Glu Arg Ala Ala
        115                 120                 125

Glu Ser Thr Ala Pro His Lys Val Lys Ser Arg Thr Phe His Tyr Pro
    130                 135                 140

Gly Gly Leu Val Asp Phe Val Lys His Ile Asn Arg Thr Lys Asn Ala
145                 150                 155                 160

Ile His Ser Ser Ile Val Asp Phe Ser Gly Lys Gly Thr Gly His Glu
                165                 170                 175

Val Glu Ile Ala Met Gln Trp Asn Ala Gly Tyr Ser Glu Ser Val His
            180                 185                 190

Thr Phe Ala Asn Thr Ile Asn Thr His Glu Gly Gly Thr His Glu Glu
        195                 200                 205

Gly Phe Arg Ser Ala Leu Thr Ser Val Val Asn Lys Tyr Ala Lys Asp
    210                 215                 220

Arg Lys Leu Leu Lys Asp Lys Asp Pro Asn Leu Thr Gly Asp Asp Ile
225                 230                 235                 240

Arg Glu Gly Leu Ala Ala Val Ile Ser Val Lys Val Ser Glu Pro Gln
                245                 250                 255

Phe Glu Gly Gln Thr Lys Thr Lys Leu Gly Asn Thr Glu Val Lys Ser
            260                 265                 270

Phe Val Gln Lys Val Cys Asn Glu Gln Leu Thr His Trp Phe Glu Ala
        275                 280                 285

Asn Pro Thr Asp Ser Lys Val Val Val Asn Lys Ala Val Ser Ser Ala
    290                 295                 300

Gln Ala Arg Ile Ala Ala Arg Lys Ala Arg Glu Leu Val Arg Arg Lys
305                 310                 315                 320

Ser Ala Thr Asp Ile Gly Gly Leu Pro Gly Lys Leu Ala Asp Cys Arg
                325                 330                 335

Ser Thr Asp Pro Arg Lys Ser Glu Leu Tyr Val Val Glu Gly Asp Ser
            340                 345                 350

Ala Gly Gly Ser Ala Lys Ser Gly Arg Asp Ser Met Phe Gln Ala Ile
        355                 360                 365

Leu Pro Leu Arg Gly Lys Ile Ile Asn Val Glu Lys Ala Arg Ile Asp
    370                 375                 380

Arg Val Leu Lys Asn Thr Glu Val Gln Ala Ile Ile Thr Ala Leu Gly
385                 390                 395                 400

Thr Gly Ile His Asp Glu Phe Asp Ile Gly Lys Leu Arg Tyr His Lys
                405                 410                 415

Ile Val Leu



<210> 31
<211> 1263
<212> DNA
<213> Mycobacterium asiaticum

<220>
<221> CDS
<222> (1)..(1263)

<400> 31
ggt gag aac agc ggc tac acc gtc agc ggt ggg ctg cac ggt gtc ggt     48
Gly Glu Asn Ser Gly Tyr Thr Val Ser Gly Gly Leu His Gly Val Gly
1               5                   10                  15

gtg tca gtg gtc aac gcg ttg tcg acc cga ctc gag gtc gac atc aag     96
Val Ser Val Val Asn Ala Leu Ser Thr Arg Leu Glu Val Asp Ile Lys
            20                  25                  30

cgc gac ggg cac gag tgg tcc cag tat tac gag cgc gcc gtt cct ggc     144
Arg Asp Gly His Glu Trp Ser Gln Tyr Tyr Glu Arg Ala Val Pro Gly
        35                  40                  45

acg ctc aag cag ggc gag gcg acc aag aag acc ggc acc acc atc cgg     192
Thr Leu Lys Gln Gly Glu Ala Thr Lys Lys Thr Gly Thr Thr Ile Arg
    50                  55                  60

ttc tgg gcg gac ccg gac atc ttc gag acc acc cag tac gac ttc gag     240
Phe Trp Ala Asp Pro Asp Ile Phe Glu Thr Thr Gln Tyr Asp Phe Glu
65                  70                  75                  80

acg gtg gcg cgc cgg ctc caa gag atg gcg ttc ctg aac aag ggc ttg     288
Thr Val Ala Arg Arg Leu Gln Glu Met Ala Phe Leu Asn Lys Gly Leu
                85                  90                  95

acc atc aac ttg acc gac gag cgg gtg gac cag gac gag gtc gtc gat     336
Thr Ile Asn Leu Thr Asp Glu Arg Val Asp Gln Asp Glu Val Val Asp
            100                 105                 110

gaa gtc gtc agc gac acc gcc gat gcg ccc aag tcc gcc gaa gag aag     384
Glu Val Val Ser Asp Thr Ala Asp Ala Pro Lys Ser Ala Glu Glu Lys
        115                 120                 125

gcg gcc gaa tcc aaa gcg ccg cac aag gtt aag cac cgc acc ttc cac     432
Ala Ala Glu Ser Lys Ala Pro His Lys Val Lys His Arg Thr Phe His
    130                 135                 140

tac ccc ggc ggc ttg gtc gac ttc gtc aag cac atc aac cgg acc aag     480
Tyr Pro Gly Gly Leu Val Asp Phe Val Lys His Ile Asn Arg Thr Lys
145                 150                 155                 160

agc ccg atc caa cag agc gtc atc gac ttc gag ggc aaa ggc acc ggc     528
Ser Pro Ile Gln Gln Ser Val Ile Asp Phe Glu Gly Lys Gly Thr Gly
                165                 170                 175

cac gag gtc gag atc gcg atg cag tgg aac ggt ggc tac tcg gag tcg     576
His Glu Val Glu Ile Ala Met Gln Trp Asn Gly Gly Tyr Ser Glu Ser
            180                 185                 190

gtg cac acc ttc gcc aac acg atc aac acc cac gag ggc ggt acg cac     624
Val His Thr Phe Ala Asn Thr Ile Asn Thr His Glu Gly Gly Thr His
        195                 200                 205

gaa gaa ggg ttc cgc agt gcg ctg acg tcg gtg gtg aac aaa tac gcc     672
Glu Glu Gly Phe Arg Ser Ala Leu Thr Ser Val Val Asn Lys Tyr Ala
    210                 215                 220

aaa gac aag aag ctg ctg aaa gac aag gac ccg aac ctc acc ggt gac 720
Lys Asp Lys Lys Leu Leu Lys Asp Lys Asp Pro Asn Leu Thr Gly Asp
225                 230                 235                 240

gac atc cgc gag gga ctg gcc gcg gtg atc tcg gtc aag gtc gcc gaa 768
Asp Ile Arg Glu Gly Leu Ala Ala Val Ile Ser Val Lys Val Ala Glu
                245                 250                 255

ccc cag ttc gag ggc cag aca aag acc aag ctg ggc aac acc gag gtc 816
Pro Gln Phe Glu Gly Gln Thr Lys Thr Lys Leu Gly Asn Thr Glu Val
            260                 265                 270

aag tcg ttc gtg cag aag gtg tgc aac gaa cag ctc acc cac tgg ttc 864
Lys Ser Phe Val Gln Lys Val Cys Asn Glu Gln Leu Thr His Trp Phe
        275                 280                 285

gag gcc aat ccg tcg gaa gcc aaa acc gtt gtc aac aag gcg gtt tcg 912
Glu Ala Asn Pro Ser Glu Ala Lys Thr Val Val Asn Lys Ala Val Ser
    290                 295                 300

tcc gca cag gcc cgg atc gcg gcg cgg aag gcc cga gag ttg gtg cgg 960
Ser Ala Gln Ala Arg Ile Ala Ala Arg Lys Ala Arg Glu Leu Val Arg
305                 310                 315                 320

cgc aag agc gcg acc gat ttg ggc ggg ctg ccc ggc aag ctg gcc gac 1008
Arg Lys Ser Ala Thr Asp Leu Gly Gly Leu Pro Gly Lys Leu Ala Asp
                325                 330                 335

tgc cgt tcc acc gac ccg cgc aag tcc gaa ctg tat gtg gtg gag ggt 1056
Cys Arg Ser Thr Asp Pro Arg Lys Ser Glu Leu Tyr Val Val Glu Gly
            340                 345                 350

gac tcg gca ggt ggc tcg gcc aag agc ggc cgt gac tcg atg ttc cag 1104
Asp Ser Ala Gly Gly Ser Ala Lys Ser Gly Arg Asp Ser Met Phe Gln
        355                 360                 365

gcc atc ctg ccg ctg cgc ggc aag atc atc aac gtc gag aag gcc cgc 1152
Ala Ile Leu Pro Leu Arg Gly Lys Ile Ile Asn Val Glu Lys Ala Arg
    370                 375                 380

atc gac cgg gtc ctg aag aac acc gaa gtc cag gcg atc atc acc gcg 1200
Ile Asp Arg Val Leu Lys Asn Thr Glu Val Gln Ala Ile Ile Thr Ala
385                 390                 395                 400

ctg ggt acc ggt att cac gac gag ttc gac att tct aaa ctg cgt tac 1248
Leu Gly Thr Gly Ile His Asp Glu Phe Asp Ile Ser Lys Leu Arg Tyr
                405                 410                 415

cac aag atc gtg ttg 1263
His Lys Ile Val Leu
            420

<210> 32
<211> 421

<212> PRT
<213> Mycobacterium asiaticum

<400> 32
Gly Glu Asn Ser Gly Tyr Thr Val Ser Gly Gly Leu His Gly Val Gly
1               5                   10                  15

Val Ser Val Val Asn Ala Leu Ser Thr Arg Leu Glu Val Asp Ile Lys
            20                  25                  30

Arg Asp Gly His Glu Trp Ser Gln Tyr Tyr Glu Arg Ala Val Pro Gly
        35                  40                  45

Thr Leu Lys Gln Gly Glu Ala Thr Lys Lys Thr Gly Thr Thr Ile Arg
    50                  55                  60

Phe Trp Ala Asp Pro Asp Ile Phe Glu Thr Thr Gln Tyr Asp Phe Glu
65                  70                  75                  80

Thr Val Ala Arg Arg Leu Gln Glu Met Ala Phe Leu Asn Lys Gly Leu
                85                  90                  95

Thr Ile Asn Leu Thr Asp Glu Arg Val Asp Gln Asp Glu Val Val Asp
            100                 105                 110

Glu Val Val Ser Asp Thr Ala Asp Ala Pro Lys Ser Ala Glu Glu Lys
        115                 120                 125

Ala Ala Glu Ser Lys Ala Pro His Lys Val Lys His Arg Thr Phe His
    130                 135                 140

Tyr Pro Gly Gly Leu Val Asp Phe Val Lys His Ile Asn Arg Thr Lys
145                 150                 155                 160

Ser Pro Ile Gln Gln Ser Val Ile Asp Phe Glu Gly Lys Gly Thr Gly
                165                 170                 175

His Glu Val Glu Ile Ala Met Gln Trp Asn Gly Gly Tyr Ser Glu Ser
            180                 185                 190

Val His Thr Phe Ala Asn Thr Ile Asn Thr His Glu Gly Gly Thr His
        195                 200                 205

Glu Glu Gly Phe Arg Ser Ala Leu Thr Ser Val Val Asn Lys Tyr Ala
    210                 215                 220

Lys Asp Lys Lys Leu Leu Lys Asp Lys Asp Pro Asn Leu Thr Gly Asp
225                 230                 235                 240

Asp Ile Arg Glu Gly Leu Ala Ala Val Ile Ser Val Lys Val Ala Glu
                245                 250                 255

Pro Gln Phe Glu Gly Gln Thr Lys Thr Lys Leu Gly Asn Thr Glu Val
            260                 265                 270

Lys Ser Phe Val Gln Lys Val Cys Asn Glu Gln Leu Thr His Trp Phe

275                 280                 285

Glu Ala Asn Pro Ser Glu Ala Lys Thr Val Val Asn Lys Ala Val Ser
    290                 295                 300

Ser Ala Gln Ala Arg Ile Ala Ala Arg Lys Ala Arg Glu Leu Val Arg
305                 310                 315                 320

Arg Lys Ser Ala Thr Asp Leu Gly Gly Leu Pro Gly Lys Leu Ala Asp
                325                 330                 335

Cys Arg Ser Thr Asp Pro Arg Lys Ser Glu Leu Tyr Val Val Glu Gly
            340                 345                 350

Asp Ser Ala Gly Gly Ser Ala Lys Ser Gly Arg Asp Ser Met Phe Gln
        355                 360                 365

Ala Ile Leu Pro Leu Arg Gly Lys Ile Ile Asn Val Glu Lys Ala Arg
    370                 375                 380

Ile Asp Arg Val Leu Lys Asn Thr Glu Val Gln Ala Ile Ile Thr Ala
385                 390                 395                 400

Leu Gly Thr Gly Ile His Asp Glu Phe Asp Ile Ser Lys Leu Arg Tyr
                405                 410                 415

His Lys Ile Val Leu
            420


<210> 33
<211> 1263
<212> DNA
<213> Mycobacterium scrofulceum

<220>
<221> CDS
<222> (1)..(1263)

<400> 33
ggc gag aac agc ggc tac acc gtc agc ggt ggg ttg cac gga gtg ggc      48
Gly Glu Asn Ser Gly Tyr Thr Val Ser Gly Gly Leu His Gly Val Gly
1               5                   10                  15

gtg tcg gtg gtc aac gcg ctg tcc acc cgc ctg gag gtc acc atc aag      96
Val Ser Val Val Asn Ala Leu Ser Thr Arg Leu Glu Val Thr Ile Lys
            20                  25                  30

cgc gac ggg cac gag tgg ttt cag tac tac gac cgc gcc gtg ccc gga      144
Arg Asp Gly His Glu Trp Phe Gln Tyr Tyr Asp Arg Ala Val Pro Gly
        35                  40                  45

acc ctc aag cag ggc gag gcc acc aag aag acc gga acc acg atc agg      192
Thr Leu Lys Gln Gly Glu Ala Thr Lys Lys Thr Gly Thr Thr Ile Arg
    50                  55                  60

ttc tgg gcg gac ccc gaa atc ttc gaa acc aca cag tac gac ttc gag 240
Phe Trp Ala Asp Pro Glu Ile Phe Glu Thr Thr Gln Tyr Asp Phe Glu
65                  70                  75                  80

acc gtg gcg cgg cgg ctg cag gag atg gcc ttc ctc aac aag ggc ctc 288
Thr Val Ala Arg Arg Leu Gln Glu Met Ala Phe Leu Asn Lys Gly Leu
                85                  90                  95

acc atc aac ctc acc gac gaa cga gtg gag cag gac gag gtc gtc gac 336
Thr Ile Asn Leu Thr Asp Glu Arg Val Glu Gln Asp Glu Val Val Asp
            100                 105                 110

gag gtc gtc agc gac acc gcc gag gca ccg aag tcc gcc gaa gag aag 384
Glu Val Val Ser Asp Thr Ala Glu Ala Pro Lys Ser Ala Glu Glu Lys
        115                 120                 125

gcc gcg gaa tcg act gcg cca cac aag gtc aag cac cgc acc ttc cac 432
Ala Ala Glu Ser Thr Ala Pro His Lys Val Lys His Arg Thr Phe His
    130                 135                 140

tac ccc ggc ggt ctg gtc gac ttc gtc aag cac atc aac cgc acc aag 480
Tyr Pro Gly Gly Leu Val Asp Phe Val Lys His Ile Asn Arg Thr Lys
145                 150                 155                 160

agc ccg atc cag cag agc gtc atc gat ttc gac ggc aag ggc acc ggc 528
Ser Pro Ile Gln Gln Ser Val Ile Asp Phe Asp Gly Lys Gly Thr Gly
                165                 170                 175

cac gag gtc gag atc gcc atg cag tgg aac ggc ggc tac tcg gag tcc 576
His Glu Val Glu Ile Ala Met Gln Trp Asn Gly Gly Tyr Ser Glu Ser
            180                 185                 190

gtc cac acc ttc gcc aac acc atc aac acg cac gag ggc ggc acc cac 624
Val His Thr Phe Ala Asn Thr Ile Asn Thr His Glu Gly Gly Thr His
        195                 200                 205

gag gag ggc ttc cgc agc gcg ctg acg tcg gtg gtg aac aag tac gcc 672
Glu Glu Gly Phe Arg Ser Ala Leu Thr Ser Val Val Asn Lys Tyr Ala
    210                 215                 220

aaa gac aag aaa ctg ctg aag gac aaa gat ccc aac ctc acc ggt gac 720
Lys Asp Lys Lys Leu Leu Lys Asp Lys Asp Pro Asn Leu Thr Gly Asp
225                 230                 235                 240

gac atc cgt gag ggc ttg gcc gcg gtc atc tcg gtg aag gtc gcc gag 768
Asp Ile Arg Glu Gly Leu Ala Ala Val Ile Ser Val Lys Val Ala Glu
                245                 250                 255

cca cag ttc gaa ggc cag acc aag aca aag ctg ggc aac acc gag gtg 816
Pro Gln Phe Glu Gly Gln Thr Lys Thr Lys Leu Gly Asn Thr Glu Val
            260                 265                 270

aag tcg ttc gtg cag aag gtg tgc aac gag cag ctc acc cac tgg ttc 864
Lys Ser Phe Val Gln Lys Val Cys Asn Glu Gln Leu Thr His Trp Phe
        275                 280                 285

gag gcc aac cca tcc gag gcg aaa acg gtg gtg aac aaa gcg gtg tcg 912
Glu Ala Asn Pro Ser Glu Ala Lys Thr Val Val Asn Lys Ala Val Ser
    290                 295                 300

tcg gct cag gcg cgc att gcc gcc cgc aag gcg cgt gaa ctg gtg cgc 960
Ser Ala Gln Ala Arg Ile Ala Ala Arg Lys Ala Arg Glu Leu Val Arg
305                 310                 315                 320

cgc aag agc gcc acc gac ctc ggc ggt ctg ccc ggg aag ctg gcc gac 1008
Arg Lys Ser Ala Thr Asp Leu Gly Gly Leu Pro Gly Lys Leu Ala Asp
                325                 330                 335

tgc cgc tcc acc gac ccg cgg aaa tcg gaa ctg tat gtg gtg gag ggc 1056
Cys Arg Ser Thr Asp Pro Arg Lys Ser Glu Leu Tyr Val Val Glu Gly
            340                 345                 350

gat tcg gcc ggc ggc tcg gcc aag agc ggg cgc gac tcg atg ttc cag 1104
Asp Ser Ala Gly Gly Ser Ala Lys Ser Gly Arg Asp Ser Met Phe Gln
        355                 360                 365

gcg atc ctg ccg ctg cgc ggc aag atc atc aat gtc gag aag gcc cgc 1152
Ala Ile Leu Pro Leu Arg Gly Lys Ile Ile Asn Val Glu Lys Ala Arg
    370                 375                 380

atc gac cgg gtg ctg aag aac acc gaa gtt cag gcg atc atc acc gcg 1200
Ile Asp Arg Val Leu Lys Asn Thr Glu Val Gln Ala Ile Ile Thr Ala
385                 390                 395                 400

ctg ggt acc ggg att cac gac gag ttc gac atc acc aag ctg cgc tat 1248
Leu Gly Thr Gly Ile His Asp Glu Phe Asp Ile Thr Lys Leu Arg Tyr
                405                 410                 415

cac aag atc gtg ctg 1263
His Lys Ile Val Leu
            420

<210> 34
<211> 421
<212> PRT
<213> Mycobacterium scrofulceum

<400> 34
Gly Glu Asn Ser Gly Tyr Thr Val Ser Gly Gly Leu His Gly Val Gly
1               5                   10                  15

Val Ser Val Val Asn Ala Leu Ser Thr Arg Leu Glu Val Thr Ile Lys
            20                  25                  30

Arg Asp Gly His Glu Trp Phe Gln Tyr Tyr Asp Arg Ala Val Pro Gly
        35                  40                  45

Thr Leu Lys Gln Gly Glu Ala Thr Lys Lys Thr Gly Thr Thr Ile Arg
    50                  55                  60

Phe Trp Ala Asp Pro Glu Ile Phe Glu Thr Thr Gln Tyr Asp Phe Glu

65                  70                  75                  80

Thr Val Ala Arg Arg Leu Gln Glu Met Ala Phe Leu Asn Lys Gly Leu
                85                  90                  95

Thr Ile Asn Leu Thr Asp Glu Arg Val Glu Gln Asp Glu Val Val Asp
            100                 105                 110

Glu Val Val Ser Asp Thr Ala Glu Ala Pro Lys Ser Ala Glu Glu Lys
        115                 120                 125

Ala Ala Glu Ser Thr Ala Pro His Lys Val Lys His Arg Thr Phe His
    130                 135                 140

Tyr Pro Gly Gly Leu Val Asp Phe Val Lys His Ile Asn Arg Thr Lys
145                 150                 155                 160

Ser Pro Ile Gln Gln Ser Val Ile Asp Phe Asp Gly Lys Gly Thr Gly
                165                 170                 175

His Glu Val Glu Ile Ala Met Gln Trp Asn Gly Gly Tyr Ser Glu Ser
            180                 185                 190

Val His Thr Phe Ala Asn Thr Ile Asn Thr His Glu Gly Gly Thr His
        195                 200                 205

Glu Glu Gly Phe Arg Ser Ala Leu Thr Ser Val Val Asn Lys Tyr Ala
    210                 215                 220

Lys Asp Lys Lys Leu Leu Lys Asp Lys Asp Pro Asn Leu Thr Gly Asp
225                 230                 235                 240

Asp Ile Arg Glu Gly Leu Ala Ala Val Ile Ser Val Lys Val Ala Glu
                245                 250                 255

Pro Gln Phe Glu Gly Gln Thr Lys Thr Lys Leu Gly Asn Thr Glu Val
            260                 265                 270

Lys Ser Phe Val Gln Lys Val Cys Asn Glu Gln Leu Thr His Trp Phe
        275                 280                 285

Glu Ala Asn Pro Ser Glu Ala Lys Thr Val Val Asn Lys Ala Val Ser
    290                 295                 300

Ser Ala Gln Ala Arg Ile Ala Ala Arg Lys Ala Arg Glu Leu Val Arg
305                 310                 315                 320

Arg Lys Ser Ala Thr Asp Leu Gly Gly Leu Pro Gly Lys Leu Ala Asp
                325                 330                 335

Cys Arg Ser Thr Asp Pro Arg Lys Ser Glu Leu Tyr Val Val Glu Gly
            340                 345                 350

Asp Ser Ala Gly Gly Ser Ala Lys Ser Gly Arg Asp Ser Met Phe Gln
        355                 360                 365

Ala Ile Leu Pro Leu Arg Gly Lys Ile Ile Asn Val Glu Lys Ala Arg
    370                 375                 380

Ile Asp Arg Val Leu Lys Asn Thr Glu Val Gln Ala Ile Ile Thr Ala
385                 390                 395                 400

Leu Gly Thr Gly Ile His Asp Glu Phe Asp Ile Thr Lys Leu Arg Tyr
                405                 410                 415

His Lys Ile Val Leu
            420


<210> 35
<211> 1260
<212> DNA
<213> Mycobacterium branderi

<220>
<221> CDS
<222> (1)..(1260)

<400> 35
ggc gat gac agc gcc tac gcg gtc tcg ggt ggt ctg cac ggc gtg ggc      48
Gly Asp Asp Ser Ala Tyr Ala Val Ser Gly Gly Leu His Gly Val Gly
  1               5                  10                  15

gtg tcg gtg gtc aac gca ttg tcg act cga ctc gag gtg gag atc gcg      96
Val Ser Val Val Asn Ala Leu Ser Thr Arg Leu Glu Val Glu Ile Ala
             20                  25                  30

acc gac ggg tac gag tgg ttt cag cat tac gac cgc tct gtc ccc ggc     144
Thr Asp Gly Tyr Glu Trp Phe Gln His Tyr Asp Arg Ser Val Pro Gly
         35                  40                  45

acg ctc aag caa ggc gag aaa acc aaa aag acc ggc acc acg gtc cgc     192
Thr Leu Lys Gln Gly Glu Lys Thr Lys Lys Thr Gly Thr Thr Val Arg
     50                  55                  60

ttc tgg gcc gac ccg gac atc ttc gag acg acg gat tac gac ttc gag     240
Phe Trp Ala Asp Pro Asp Ile Phe Glu Thr Thr Asp Tyr Asp Phe Glu
 65                  70                  75                  80

acg gtc gca cgc cgg ctg cag gaa atg gcg ttc ctc aac aaa ggg ctg     288
Thr Val Ala Arg Arg Leu Gln Glu Met Ala Phe Leu Asn Lys Gly Leu
                 85                  90                  95

acc atc aac ctg acc gac gag cgg gtg cga aac gaa gaa gtc gtc gac     336
Thr Ile Asn Leu Thr Asp Glu Arg Val Arg Asn Glu Glu Val Val Asp
            100                 105                 110

gag gtc gtc agc gac acc gcc gac gcg ccg aag tcg gcg cgc gaa gag     384
Glu Val Val Ser Asp Thr Ala Asp Ala Pro Lys Ser Ala Arg Glu Glu
        115                 120                 125

gcc gaa gaa cgg acc acg cag aaa gtc aag cac cgc acg ttc cat tac     432

Ala Glu Glu Arg Thr Thr Gln Lys Val Lys His Arg Thr Phe His Tyr
    130                 135                 140

ccc ggc ggc ttg gtc gat ttc gtc aaa cac atc aac cgc aca aag aac     480
Pro Gly Gly Leu Val Asp Phe Val Lys His Ile Asn Arg Thr Lys Asn
145                 150                 155                 160

ccc atc cat tcg agc atc gtc gac ttc tcc ggc aag ggt ccc ggc cac     528
Pro Ile His Ser Ser Ile Val Asp Phe Ser Gly Lys Gly Pro Gly His
                165                 170                 175

gag gtc gag atc gca atg cag tgg aac gcc ggc tat tcg gag tcg gtg     576
Glu Val Glu Ile Ala Met Gln Trp Asn Ala Gly Tyr Ser Glu Ser Val
            180                 185                 190

cac acc ttc gcc aac acc atc aac acc cac gag ggc ggc acc cac gaa     624
His Thr Phe Ala Asn Thr Ile Asn Thr His Glu Gly Gly Thr His Glu
        195                 200                 205

gaa ggg ttc cgc gcg gca ctg acg tcc gtg gtg aac aag tac gcc aag     672
Glu Gly Phe Arg Ala Ala Leu Thr Ser Val Val Asn Lys Tyr Ala Lys
    210                 215                 220

gac cga aaa ctg ctg aag gac aag gac ccc aac ctc acc ggc gac gac     720
Asp Arg Lys Leu Leu Lys Asp Lys Asp Pro Asn Leu Thr Gly Asp Asp
225                 230                 235                 240

att cgt gag ggc ctg gcg gcg gtc atc tcg gtc aag gtc agc gag ccg     768
Ile Arg Glu Gly Leu Ala Ala Val Ile Ser Val Lys Val Ser Glu Pro
                245                 250                 255

cag ttc gag ggc cag acc aaa acc aaa ctc ggc aac acc gaa gtc aag     816
Gln Phe Glu Gly Gln Thr Lys Thr Lys Leu Gly Asn Thr Glu Val Lys
            260                 265                 270

tcg ttt gtg cag aag gtc tgc aac gaa cag ctc acc cac tgg ttc gag     864
Ser Phe Val Gln Lys Val Cys Asn Glu Gln Leu Thr His Trp Phe Glu
        275                 280                 285

gcc aat ccc agc gac gcc aag acc gtc gtc aac aaa gcg gtg tcg tcg     912
Ala Asn Pro Ser Asp Ala Lys Thr Val Val Asn Lys Ala Val Ser Ser
    290                 295                 300

gcg cag gcc cgc att gcc gcc cgc aaa gcg cga gaa ttg gtg cgc cgc     960
Ala Gln Ala Arg Ile Ala Ala Arg Lys Ala Arg Glu Leu Val Arg Arg
305                 310                 315                 320

aag agc gca acc gat ctt ggc ggg ctg ccg ggc aag ctg gct gac tgc     1008
Lys Ser Ala Thr Asp Leu Gly Gly Leu Pro Gly Lys Leu Ala Asp Cys
                325                 330                 335

cgc tcg acc gat cca cgc aag tcc gaa ttg tat gtg gtg gag ggt gat     1056
Arg Ser Thr Asp Pro Arg Lys Ser Glu Leu Tyr Val Val Glu Gly Asp
            340                 345                 350

tcg gcc ggc ggc tcg gcc aag agc ggc cgc gac tcg atg ttt cag gcg     1104

Ser Ala Gly Gly Ser Ala Lys Ser Gly Arg Asp Ser Met Phe Gln Ala
        355                 360                 365

atc ctg ccg ttg cgg ggc aag atc atc aac gtg gag aag gcc cgc atc      1152
Ile Leu Pro Leu Arg Gly Lys Ile Ile Asn Val Glu Lys Ala Arg Ile
    370                 375                 380

gac cgg gtg ctg aag aac act gag gtg cag gcg atc atc acc gcg ctg      1200
Asp Arg Val Leu Lys Asn Thr Glu Val Gln Ala Ile Ile Thr Ala Leu
385                 390                 395                 400

ggc acc ggg att cac gac gag ttc gac atc tcc aag ctg cgc tac cac      1248
Gly Thr Gly Ile His Asp Glu Phe Asp Ile Ser Lys Leu Arg Tyr His
                405                 410                 415

aag atc gtg ctg                                                      1260
Lys Ile Val Leu
            420

<210> 36
<211> 420
<212> PRT
<213> Mycobacterium branderi

<400> 36
Gly Asp Asp Ser Ala Tyr Ala Val Ser Gly Gly Leu His Gly Val Gly
  1               5                  10                  15

Val Ser Val Val Asn Ala Leu Ser Thr Arg Leu Glu Val Glu Ile Ala
            20                  25                  30

Thr Asp Gly Tyr Glu Trp Phe Gln His Tyr Asp Arg Ser Val Pro Gly
        35                  40                  45

Thr Leu Lys Gln Gly Glu Lys Thr Lys Lys Thr Gly Thr Thr Val Arg
    50                  55                  60

Phe Trp Ala Asp Pro Asp Ile Phe Glu Thr Thr Asp Tyr Asp Phe Glu
 65                  70                  75                  80

Thr Val Ala Arg Arg Leu Gln Glu Met Ala Phe Leu Asn Lys Gly Leu
                85                  90                  95

Thr Ile Asn Leu Thr Asp Glu Arg Val Arg Asn Glu Glu Val Val Asp
            100                 105                 110

Glu Val Val Ser Asp Thr Ala Asp Ala Pro Lys Ser Ala Arg Glu Glu
        115                 120                 125

Ala Glu Glu Arg Thr Thr Gln Lys Val Lys His Arg Thr Phe His Tyr
    130                 135                 140

Pro Gly Gly Leu Val Asp Phe Val Lys His Ile Asn Arg Thr Lys Asn
145                 150                 155                 160

Pro Ile His Ser Ser Ile Val Asp Phe Ser Gly Lys Gly Pro Gly His
                165                 170                 175

Glu Val Glu Ile Ala Met Gln Trp Asn Ala Gly Tyr Ser Glu Ser Val
            180                 185                 190

His Thr Phe Ala Asn Thr Ile Asn Thr His Glu Gly Gly Thr His Glu
        195                 200                 205

Glu Gly Phe Arg Ala Ala Leu Thr Ser Val Val Asn Lys Tyr Ala Lys
    210                 215                 220

Asp Arg Lys Leu Leu Lys Asp Lys Asp Pro Asn Leu Thr Gly Asp Asp
225                 230                 235                 240

Ile Arg Glu Gly Leu Ala Ala Val Ile Ser Val Lys Val Ser Glu Pro
                245                 250                 255

Gln Phe Glu Gly Gln Thr Lys Thr Lys Leu Gly Asn Thr Glu Val Lys
            260                 265                 270

Ser Phe Val Gln Lys Val Cys Asn Glu Gln Leu Thr His Trp Phe Glu
        275                 280                 285

Ala Asn Pro Ser Asp Ala Lys Thr Val Val Asn Lys Ala Val Ser Ser
    290                 295                 300

Ala Gln Ala Arg Ile Ala Ala Arg Lys Ala Arg Glu Leu Val Arg Arg
305                 310                 315                 320

Lys Ser Ala Thr Asp Leu Gly Gly Leu Pro Gly Lys Leu Ala Asp Cys
                325                 330                 335

Arg Ser Thr Asp Pro Arg Lys Ser Glu Leu Tyr Val Val Glu Gly Asp
            340                 345                 350

Ser Ala Gly Gly Ser Ala Lys Ser Gly Arg Asp Ser Met Phe Gln Ala
        355                 360                 365

Ile Leu Pro Leu Arg Gly Lys Ile Ile Asn Val Glu Lys Ala Arg Ile
    370                 375                 380

Asp Arg Val Leu Lys Asn Thr Glu Val Gln Ala Ile Ile Thr Ala Leu
385                 390                 395                 400

Gly Thr Gly Ile His Asp Glu Phe Asp Ile Ser Lys Leu Arg Tyr His
                405                 410                 415

Lys Ile Val Leu
            420

<210> 37
<211> 1263
<212> DNA
<213> Mycobacterium paratuberculosis

<220>
<221> CDS
<222> (1)..(1263)

<400> 37
ggc gag aac agc ggc tac aac gtc agc ggc ggt ctg cac ggc gtc ggc     48
Gly Glu Asn Ser Gly Tyr Asn Val Ser Gly Gly Leu His Gly Val Gly
1               5                   10                  15

gtc tcg gtg gtc aac gcg ctg tcc act cgg ctc gag gtc aac atc gcc     96
Val Ser Val Val Asn Ala Leu Ser Thr Arg Leu Glu Val Asn Ile Ala
            20                  25                  30

cgc gac ggc tac gag tgg tcg cag tac tac gac cac gcc gtg ccc ggc     144
Arg Asp Gly Tyr Glu Trp Ser Gln Tyr Tyr Asp His Ala Val Pro Gly
        35                  40                  45

acc ctc aag cag ggc gag gcc acc aag cgc acc ggc acc acc atc cgg     192
Thr Leu Lys Gln Gly Glu Ala Thr Lys Arg Thr Gly Thr Thr Ile Arg
    50                  55                  60

ttc tgg gcc gac ccc gac atc ttc gag acc acc gag tac gac ttc gaa     240
Phe Trp Ala Asp Pro Asp Ile Phe Glu Thr Thr Glu Tyr Asp Phe Glu
65                  70                  75                  80

acg gtg gcc cgg cgg ctg cag gaa atg gcg ttc ctc aac aag ggc ctg     288
Thr Val Ala Arg Arg Leu Gln Glu Met Ala Phe Leu Asn Lys Gly Leu
                85                  90                  95

acc atc aac ctc acc gac gag cgg gtg acc aac gaa gag gtc gtc gac     336
Thr Ile Asn Leu Thr Asp Glu Arg Val Thr Asn Glu Glu Val Val Asp
            100                 105                 110

gag gtg gtc agc gac acc gcc gac gca ccc aag tcg gcg cag gag aag     384
Glu Val Val Ser Asp Thr Ala Asp Ala Pro Lys Ser Ala Gln Glu Lys
        115                 120                 125

gcg gcg gaa tcg gct gcg ccg cat aag gtc aag cac cgc acc ttc cac     432
Ala Ala Glu Ser Ala Ala Pro His Lys Val Lys His Arg Thr Phe His
    130                 135                 140

tac ccc ggc ggc ctg gtc gac ttc gtc aaa cac atc aat cgc acc aaa     480
Tyr Pro Gly Gly Leu Val Asp Phe Val Lys His Ile Asn Arg Thr Lys
145                 150                 155                 160

aac ccc atc cac cag agc atc atc gat ttc ggt ggg aag ggc ccc ggc     528
Asn Pro Ile His Gln Ser Ile Ile Asp Phe Gly Gly Lys Gly Pro Gly
                165                 170                 175

cac gag gtc gag atc gcg atg cag tgg aac ggc ggc tac tcc gaa tcg     576
His Glu Val Glu Ile Ala Met Gln Trp Asn Gly Gly Tyr Ser Glu Ser
            180                 185                 190

gtg cac acc ttc gcc aac acc atc aac acg cac gag ggc ggc acc cac     624
Val His Thr Phe Ala Asn Thr Ile Asn Thr His Glu Gly Gly Thr His

        195                 200                 205

gag gag ggc ttc cgc agc gcg ctg acc tcc gtg gtc aac aag tac gcc 672
Glu Glu Gly Phe Arg Ser Ala Leu Thr Ser Val Val Asn Lys Tyr Ala
    210                 215                 220

aag gac aag aag ctg ctc aag gac aag gac ccc aac ctg acc ggt gac 720
Lys Asp Lys Lys Leu Leu Lys Asp Lys Asp Pro Asn Leu Thr Gly Asp
225                 230                 235                 240

gac atc cgc gag ggt ttg gcc gcg gtg atc tcg gtc aag gtg agc gaa 768
Asp Ile Arg Glu Gly Leu Ala Ala Val Ile Ser Val Lys Val Ser Glu
                245                 250                 255

ccg cag ttc gag ggc cag acc aag acc aaa ctg ggc aac acc gag gtg 816
Pro Gln Phe Glu Gly Gln Thr Lys Thr Lys Leu Gly Asn Thr Glu Val
            260                 265                 270

aag tcg ttc gtg cag aag gtg tgc aac gaa cag ctc acc cac tgg ttc 864
Lys Ser Phe Val Gln Lys Val Cys Asn Glu Gln Leu Thr His Trp Phe
        275                 280                 285

gaa gcc aac ccc gca gac gcc aaa gtc att gtc aac aag gcg gtt tca 912
Glu Ala Asn Pro Ala Asp Ala Lys Val Ile Val Asn Lys Ala Val Ser
    290                 295                 300

tca gcg cag gcg cgc atc gcc gcg cgc aag gcg cga gag ttg gtg cgc 960
Ser Ala Gln Ala Arg Ile Ala Ala Arg Lys Ala Arg Glu Leu Val Arg
305                 310                 315                 320

cgc aag agc gca acc gac ctg ggc ggg ctg ccc ggc aag ctc gcc gac 1008
Arg Lys Ser Ala Thr Asp Leu Gly Gly Leu Pro Gly Lys Leu Ala Asp
                325                 330                 335

tgc cgg tcg acc gac ccg cgc aag tcg gaa ttg tat gtg gtc gag ggt 1056
Cys Arg Ser Thr Asp Pro Arg Lys Ser Glu Leu Tyr Val Val Glu Gly
            340                 345                 350

gac tcg gcc ggc ggc tcg gcg aaa agc ggc cgg gac tcg atg ttc cag 1104
Asp Ser Ala Gly Gly Ser Ala Lys Ser Gly Arg Asp Ser Met Phe Gln
        355                 360                 365

gcc atc ctt ccg ctg cgc ggc aag atc atc aac gtc gaa aag gcc cgc 1152
Ala Ile Leu Pro Leu Arg Gly Lys Ile Ile Asn Val Glu Lys Ala Arg
    370                 375                 380

atc gac cgg gtg ctg aag aac acc gag gtg cag gcg atc atc acc gcg 1200
Ile Asp Arg Val Leu Lys Asn Thr Glu Val Gln Ala Ile Ile Thr Ala
385                 390                 395                 400

ctg ggc acc ggg att cac gac gag ttc gac atc acc aag ctg cgc tac 1248
Leu Gly Thr Gly Ile His Asp Glu Phe Asp Ile Thr Lys Leu Arg Tyr
                405                 410                 415

cac aag atc gtg ttg 1263
His Lys Ile Val Leu

            420

<210> 38
<211> 421
<212> PRT
<213> Mycobacterium paratuberculosis

<400> 38
Gly Glu Asn Ser Gly Tyr Asn Val Ser Gly Gly Leu His Gly Val Gly
1               5                   10                  15

Val Ser Val Val Asn Ala Leu Ser Thr Arg Leu Glu Val Asn Ile Ala
            20                  25                  30

Arg Asp Gly Tyr Glu Trp Ser Gln Tyr Tyr Asp His Ala Val Pro Gly
        35                  40                  45

Thr Leu Lys Gln Gly Glu Ala Thr Lys Arg Thr Gly Thr Thr Ile Arg
    50                  55                  60

Phe Trp Ala Asp Pro Asp Ile Phe Glu Thr Thr Glu Tyr Asp Phe Glu
65                  70                  75                  80

Thr Val Ala Arg Arg Leu Gln Glu Met Ala Phe Leu Asn Lys Gly Leu
                85                  90                  95

Thr Ile Asn Leu Thr Asp Glu Arg Val Thr Asn Glu Glu Val Val Asp
            100                 105                 110

Glu Val Val Ser Asp Thr Ala Asp Ala Pro Lys Ser Ala Gln Glu Lys
        115                 120                 125

Ala Ala Glu Ser Ala Ala Pro His Lys Val Lys His Arg Thr Phe His
    130                 135                 140

Tyr Pro Gly Gly Leu Val Asp Phe Val Lys His Ile Asn Arg Thr Lys
145                 150                 155                 160

Asn Pro Ile His Gln Ser Ile Ile Asp Phe Gly Gly Lys Gly Pro Gly
                165                 170                 175

His Glu Val Glu Ile Ala Met Gln Trp Asn Gly Gly Tyr Ser Glu Ser
            180                 185                 190

Val His Thr Phe Ala Asn Thr Ile Asn Thr His Glu Gly Gly Thr His
        195                 200                 205

Glu Glu Gly Phe Arg Ser Ala Leu Thr Ser Val Val Asn Lys Tyr Ala
    210                 215                 220

Lys Asp Lys Lys Leu Leu Lys Asp Lys Asp Pro Asn Leu Thr Gly Asp
225                 230                 235                 240

Asp Ile Arg Glu Gly Leu Ala Ala Val Ile Ser Val Lys Val Ser Glu
                245                 250                 255

Pro Gln Phe Glu Gly Gln Thr Lys Thr Lys Leu Gly Asn Thr Glu Val
            260                 265                 270

Lys Ser Phe Val Gln Lys Val Cys Asn Glu Gln Leu Thr His Trp Phe
        275                 280                 285

Glu Ala Asn Pro Ala Asp Ala Lys Val Ile Val Asn Lys Ala Val Ser
    290                 295                 300

Ser Ala Gln Ala Arg Ile Ala Ala Arg Lys Ala Arg Glu Leu Val Arg
305                 310                 315                 320

Arg Lys Ser Ala Thr Asp Leu Gly Gly Leu Pro Gly Lys Leu Ala Asp
                325                 330                 335

Cys Arg Ser Thr Asp Pro Arg Lys Ser Glu Leu Tyr Val Val Glu Gly
            340                 345                 350

Asp Ser Ala Gly Gly Ser Ala Lys Ser Gly Arg Asp Ser Met Phe Gln
        355                 360                 365

Ala Ile Leu Pro Leu Arg Gly Lys Ile Ile Asn Val Glu Lys Ala Arg
    370                 375                 380

Ile Asp Arg Val Leu Lys Asn Thr Glu Val Gln Ala Ile Ile Thr Ala
385                 390                 395                 400

Leu Gly Thr Gly Ile His Asp Glu Phe Asp Ile Thr Lys Leu Arg Tyr
                405                 410                 415

His Lys Ile Val Leu
            420


<210> 39
<211> 1257
<212> DNA
<213> Mycobacterium kansasii

<220>
<221> CDS
<222> (1)..(1257)

<400> 39
tcc gac gcc tac gcg ata tcg ggc ggg ctg cac ggt gtg ggt gtc tcg      48
Ser Asp Ala Tyr Ala Ile Ser Gly Gly Leu His Gly Val Gly Val Ser
1               5                   10                  15

gtg gtc aac gca ctg tcc acc cgg ctg gag gtg gag atc aag cgc gac      96
Val Val Asn Ala Leu Ser Thr Arg Leu Glu Val Glu Ile Lys Arg Asp
            20                  25                  30

ggc cat gag tgg tcg cag gtt tac gag aaa tcc gag ccg atg gga ctc      144
Gly His Glu Trp Ser Gln Val Tyr Glu Lys Ser Glu Pro Met Gly Leu
        35                  40                  45

aag caa ggc gcg ccg act aag aag acc ggc acg acg gtg cgg ttc tgg 192
Lys Gln Gly Ala Pro Thr Lys Lys Thr Gly Thr Thr Val Arg Phe Trp
    50                  55                  60

gcc gat ccc aat gtt ttt gag acc acc gag tac gac ttc gaa acc gtc 240
Ala Asp Pro Asn Val Phe Glu Thr Thr Glu Tyr Asp Phe Glu Thr Val
65                  70                  75                  80

gca cga cgg ttg cag gag atg gcg ttt ctc aac aag ggg ctc acc atc 288
Ala Arg Arg Leu Gln Glu Met Ala Phe Leu Asn Lys Gly Leu Thr Ile
                85                  90                  95

aat ctg acc gat cag cgg gtg acc cag gac gag gtc gtc gac gag gtg 336
Asn Leu Thr Asp Gln Arg Val Thr Gln Asp Glu Val Val Asp Glu Val
            100                 105                 110

gtc agc gac gtc gcc gag gcc cca aag tcg gcc agc gag aag gcg gcc 384
Val Ser Asp Val Ala Glu Ala Pro Lys Ser Ala Ser Glu Lys Ala Ala
        115                 120                 125

gaa tcc gcc gcc ccg cac aag gtc aag aag cgt acc ttc cac tat ccc 432
Glu Ser Ala Ala Pro His Lys Val Lys Lys Arg Thr Phe His Tyr Pro
    130                 135                 140

ggg ggt ctg gtt gac ttc gtc aag cac atc aac cgg acc aag aac gcc 480
Gly Gly Leu Val Asp Phe Val Lys His Ile Asn Arg Thr Lys Asn Ala
145                 150                 155                 160

atc cac agc agc atc gtc gac ttc tcc ggt aag gga ccc ggc cac gaa 528
Ile His Ser Ser Ile Val Asp Phe Ser Gly Lys Gly Pro Gly His Glu
                165                 170                 175

gtg gag atc gcg atg cag tgg aat gcc ggc tat tcg gag tcg gtg cat 576
Val Glu Ile Ala Met Gln Trp Asn Ala Gly Tyr Ser Glu Ser Val His
            180                 185                 190

acc ttc gcc aac acc atc aac acc cac gag ggt ggg acc cac gaa gag 624
Thr Phe Ala Asn Thr Ile Asn Thr His Glu Gly Gly Thr His Glu Glu
        195                 200                 205

ggg ttc cgc agc gcg ctg acc tcg gtg gtg aac aag tac gcc aag gac 672
Gly Phe Arg Ser Ala Leu Thr Ser Val Val Asn Lys Tyr Ala Lys Asp
    210                 215                 220

cgc aaa ctg ctc aag gaa aag gac ccc aac ctc acc ggc gac gac atc 720
Arg Lys Leu Leu Lys Glu Lys Asp Pro Asn Leu Thr Gly Asp Asp Ile
225                 230                 235                 240

cgg gaa ggg ttg gcc gcg gtg att tcg gtc aag gtc agc gag ccg cag 768
Arg Glu Gly Leu Ala Ala Val Ile Ser Val Lys Val Ser Glu Pro Gln
                245                 250                 255

ttc gag ggc cag acc aag acg aaa ctg ggc aac acc gag gtg aag tcg 816
Phe Glu Gly Gln Thr Lys Thr Lys Leu Gly Asn Thr Glu Val Lys Ser
            260                 265                 270

ttc gtg cag aag gtg tgc aac gaa cag ctc acc cat tgg ttc gag gcc     864
Phe Val Gln Lys Val Cys Asn Glu Gln Leu Thr His Trp Phe Glu Ala
        275                 280                 285

aac ccc gct gac gct aaa acc gtt gtc aac aag gcg gtt tca tcg gcg     912
Asn Pro Ala Asp Ala Lys Thr Val Val Asn Lys Ala Val Ser Ser Ala
    290                 295                 300

caa gca cgc att gcg gcc cgc aag gcg cgc gag ttg gtg cgc cgc aag     960
Gln Ala Arg Ile Ala Ala Arg Lys Ala Arg Glu Leu Val Arg Arg Lys
305                 310                 315                 320

agc gca acc gat ctg ggc gga cta ccc ggc aag ctc gcc gac tgc cgc     1008
Ser Ala Thr Asp Leu Gly Gly Leu Pro Gly Lys Leu Ala Asp Cys Arg
                325                 330                 335

tcg acc gac ccg cgc aag tcc gaa ctg tat gtg gtg gag ggt gat tca     1056
Ser Thr Asp Pro Arg Lys Ser Glu Leu Tyr Val Val Glu Gly Asp Ser
            340                 345                 350

gcc ggc ggc tcg gcg aag agc ggt cgc gac tcg atg ttc cag gcc atc     1104
Ala Gly Gly Ser Ala Lys Ser Gly Arg Asp Ser Met Phe Gln Ala Ile
        355                 360                 365

ttg ccg ttg cgc ggc aag atc atc aac gtc gag aag gcc cgc atc gac     1152
Leu Pro Leu Arg Gly Lys Ile Ile Asn Val Glu Lys Ala Arg Ile Asp
    370                 375                 380

cgg gtg ctg aag aac acc gaa gtc cag gcg atc atc acc gcg ttg ggt     1200
Arg Val Leu Lys Asn Thr Glu Val Gln Ala Ile Ile Thr Ala Leu Gly
385                 390                 395                 400

acc ggc atc cac gac gaa ttc gac atc gcg aga ctg cgt tac cac aag     1248
Thr Gly Ile His Asp Glu Phe Asp Ile Ala Arg Leu Arg Tyr His Lys
                405                 410                 415

atc gtg ctc                                                         1257
Ile Val Leu


<210> 40
<211> 419
<212> PRT
<213> Mycobacterium kansasii

<400> 40
Ser Asp Ala Tyr Ala Ile Ser Gly Gly Leu His Gly Val Gly Val Ser
1               5                   10                  15

Val Val Asn Ala Leu Ser Thr Arg Leu Glu Val Glu Ile Lys Arg Asp
            20                  25                  30

Gly His Glu Trp Ser Gln Val Tyr Glu Lys Ser Glu Pro Met Gly Leu
        35                  40                  45

Lys Gln Gly Ala Pro Thr Lys Lys Thr Gly Thr Thr Val Arg Phe Trp
    50                  55                  60

Ala Asp Pro Asn Val Phe Glu Thr Thr Glu Tyr Asp Phe Glu Thr Val
65                  70                  75                  80

Ala Arg Arg Leu Gln Glu Met Ala Phe Leu Asn Lys Gly Leu Thr Ile
                85                  90                  95

Asn Leu Thr Asp Gln Arg Val Thr Gln Asp Glu Val Val Asp Glu Val
            100                 105                 110

Val Ser Asp Val Ala Glu Ala Pro Lys Ser Ala Ser Glu Lys Ala Ala
        115                 120                 125

Glu Ser Ala Ala Pro His Lys Val Lys Lys Arg Thr Phe His Tyr Pro
    130                 135                 140

Gly Gly Leu Val Asp Phe Val Lys His Ile Asn Arg Thr Lys Asn Ala
145                 150                 155                 160

Ile His Ser Ser Ile Val Asp Phe Ser Gly Lys Gly Pro Gly His Glu
                165                 170                 175

Val Glu Ile Ala Met Gln Trp Asn Ala Gly Tyr Ser Glu Ser Val His
            180                 185                 190

Thr Phe Ala Asn Thr Ile Asn Thr His Glu Gly Gly Thr His Glu Glu
        195                 200                 205

Gly Phe Arg Ser Ala Leu Thr Ser Val Val Asn Lys Tyr Ala Lys Asp
    210                 215                 220

Arg Lys Leu Leu Lys Glu Lys Asp Pro Asn Leu Thr Gly Asp Asp Ile
225                 230                 235                 240

Arg Glu Gly Leu Ala Ala Val Ile Ser Val Lys Val Ser Glu Pro Gln
                245                 250                 255

Phe Glu Gly Gln Thr Lys Thr Lys Leu Gly Asn Thr Glu Val Lys Ser
            260                 265                 270

Phe Val Gln Lys Val Cys Asn Glu Gln Leu Thr His Trp Phe Glu Ala
        275                 280                 285

Asn Pro Ala Asp Ala Lys Thr Val Val Asn Lys Ala Val Ser Ser Ala
    290                 295                 300

Gln Ala Arg Ile Ala Ala Arg Lys Ala Arg Glu Leu Val Arg Arg Lys
305                 310                 315                 320

Ser Ala Thr Asp Leu Gly Gly Leu Pro Gly Lys Leu Ala Asp Cys Arg
                325                 330                 335

Ser Thr Asp Pro Arg Lys Ser Glu Leu Tyr Val Val Glu Gly Asp Ser
            340                 345                 350

Ala Gly Gly Ser Ala Lys Ser Gly Arg Asp Ser Met Phe Gln Ala Ile
        355                 360                 365

Leu Pro Leu Arg Gly Lys Ile Ile Asn Val Glu Lys Ala Arg Ile Asp
    370                 375                 380

Arg Val Leu Lys Asn Thr Glu Val Gln Ala Ile Ile Thr Ala Leu Gly
385                 390                 395                 400

Thr Gly Ile His Asp Glu Phe Asp Ile Ala Arg Leu Arg Tyr His Lys
                405                 410                 415

Ile Val Leu

<210> 41
<211> 20
<212> DNA
<213> Artificial Sequence

<220>
<223> Description of Artificial Sequence: Synthetic DNA

<400> 41
tcggacgcgt atgcgatatc                                                20

<210> 42
<211> 7
<212> PRT
<213> Artificial Sequence

<220>
<223> Description of Artificial Sequence: Synthetic DNA

<400> 42
Ser Asp Ala Tyr Ala Ile Ser
  1               5

<210> 43
<211> 20
<212> DNA
<213> Artificial Sequence

<220>
<223> Description of Artificial Sequence: Synthetic DNA

<400> 43
acatacagtt cggacttgcg                                                20

<210> 44
<211> 7

<212> PRT
<213> Artificial Sequence

<220>
<223> Description of Artificial Sequence: Amino acid
      sequence corresponding to primer or probe

<400> 44
Arg Lys Ser Glu Leu Tyr Val
  1               5


<210> 45
<211> 19
<212> DNA
<213> Artificial Sequence

<220>
<223> Description of Artificial Sequence: Synthetic DNA

<400> 45
gaagacgggg tcaacggtg                                                19


<210> 46
<211> 6
<212> PRT
<213> Artificial Sequence

<220>
<223> Description of Artificial Sequence: Amino acid
      sequence corresponding to primer or probe

<400> 46
Lys Thr Gly Ser Thr Val
  1               5


<210> 47
<211> 22
<212> DNA
<213> Artificial Sequence

<220>
<223> Description of Artificial Sequence: Synthetic DNA

<400> 47
ccttgttcac aacgactttc gc                                            22


<210> 48
<211> 7
<212> PRT
<213> Artificial Sequence

<220>

<223> Description of Artificial Sequence: Amino acid
      sequence corresponding to primer or probe

<400> 48
Ala Lys Val Val Val Asn Lys
  1               5


<210> 49
<211> 19
<212> DNA
<213> Artificial Sequence

<220>
<223> Description of Artificial Sequence: Synthetic DNA

<400> 49
gaagacgggg tcaacggta                                                    19


<210> 50
<211> 6
<212> PRT
<213> Artificial Sequence

<220>
<223> Description of Artificial Sequence: Amino acid
      sequence corresponding to primer or probe

<400> 50
Lys Thr Gly Ser Thr Val
  1               5


<210> 51
<211> 19
<212> DNA
<213> Artificial Sequence

<220>
<223> Description of Artificial Sequence: Synthetic DNA

<400> 51
ccagtgggtc agctgttca                                                    19


<210> 52
<211> 6
<212> PRT
<213> Artificial Sequence

<220>
<223> Description of Artificial Sequence: Amino acid
      sequence corresponding to primer or probe

<400> 52

Glu Gln Leu Thr His Trp
1               5

<210> 53
<211> 22
<212> DNA
<213> Artificial Sequence

<220>
<223> Description of Artificial Sequence: Synthetic DNA

<400> 53
ccttgttcac aacgactttc ga                                                22

<210> 54
<211> 7
<212> PRT
<213> Artificial Sequence

<220>
<223> Description of Artificial Sequence: Amino acid
      sequence corresponding to primer or probe

<400> 54
Ser Lys Val Val Val Asn Lys
1               5

<210> 55
<211> 20
<212> DNA
<213> Artificial Sequence

<220>
<223> Description of Artificial Sequence: Synthetic DNA

<400> 55
agatcaagcg cgacgggtat                                                   20

<210> 56
<211> 6
<212> PRT
<213> Artificial Sequence

<220>
<223> Description of Artificial Sequence: Amino acid
      sequence corresponding to primer or probe

<400> 56
Ile Lys Arg Asp Gly Tyr
1               5

<210> 57
<211> 19
<212> DNA
<213> Artificial Sequence

<220>
<223> Description of Artificial Sequence: Synthetic DNA

<400> 57
ccagtgggtc agctgttcg                                                    19


<210> 58
<211> 6
<212> PRT
<213> Artificial Sequence

<220>
<223> Description of Artificial Sequence: Amino acid
      sequence corresponding to primer or probe

<400> 58
Glu Gln Leu Thr His Trp
  1               5


<210> 59
<211> 38
<212> DNA
<213> Artificial Sequence

<220>
<223> Description of Artificial Sequence: Synthetic DNA

<400> 59
tgtaaaacga cggccagtca ygcnggnggn aarttyga                               38


<210> 60
<211> 36
<212> DNA
<213> Artificial Sequence

<220>
<223> Description of Artificial Sequence: Synthetic DNA

<400> 60
ctgcgttcgt atatgagcnc crtcnacrtc ngcrtc                                 36


<210> 61
<211> 17
<212> DNA
<213> Artificial Sequence

<220>

<223> Description of Artificial Sequence: Synthetic DNA

<400> 61
gacgcstayg cgatatc                                                    17

<210> 62
<211> 17
<212> DNA
<213> Artificial Sequence

<220>
<223> Description of Artificial Sequence: Synthetic DNA

<400> 62
agcggytaca acgtcag                                                    17

## Description

### FIELD OF THE INVENTION

The present invention relates to a method for identification or detection of slow growing mycobacteria having a large number of clinical cases as causative microorganisms of tuberculosis and atypical mycobacterial disease (especially, tubercle bacilli group bacteria), which utilizing a nucleotide sequence of a DNA coding for DNA gyrase β subunit (to be referred to as "*gyrB* gene" or "*gyrB*" hereinafter). The identification and detection methods of the present invention are useful in various industrial fields, such as medical science, immunology and veterinary science.

### BACKGROUND ART

A plurality of species belonging to slow growing mycobacteria are known as acid-fast bacterial species which cause tuberculosis and diseases analogous to tuberculosis in human. Among all, *Mycobacterium tuberculosis* complex, *Mycobacterium avium* complex and *Mycobacterium kansasii* occupy the most part of clinical cases. Recently, these bacteria are causing a serious problem for the prognosis of patients of acquired immunodeficiency syndrome (AIDS), because they induce systemic disseminated infection in AIDS patients.

Conventionally, identification and detection of these bacterial species have been carried out by physiological and biochemical methods based on the culturing. For example, identification and detection have been carried out using such differences in color development because (1) since there are three groups in the slow growing mycobacteria, namely a group which develops yellow color only when it is cultured in the dark after irradiation with light (photochromogen), (2) a group which develops color even when cultured without irradiation of light (scotochromogen) and (3) a group which does not develop color even when light is irradiated (achromogen). Known methods include identification and detection based on the ability of cultured bacteria to produce catalase or show urease activity, Tween hydrolyzing activity or nitrate reducing activity.

Tuberculosis is a most important infectious disease, because ninety million people in the world newly contract the disease every year and thirty million people of them die every year, but its countermeasure is not sufficient. Tubercle bacilli as its causative agents are classified into four strains, namely a tubercle strain (*Mycobacterium tuberculosis*), a bovine type strain (*Mycobacterium bovis*) and an Africa strain (*Mycobacterium africanum*) which are pathogenic for human and a rat type strain (*Mycobacterium microti*) which is not pathogenic for human. Conventionally, tests of acid-fast bacteria including these mycobacteria were mainly carried out by smear staining by the Ziehl-Neelsen method, isolation culturing method using Ogawa medium and a drug-sensitivity test. With the development of techniques thereafter, BACTEC 460 TB System, Septi-Check AFB, MGIT (Mycobacteria Growth Indicator Tube) and the like novel culture techniques have been developed.

However, these tests require pure culture. In addition, because phenotype to be compared is liable to change, the judgment often becomes subjective. As a result, not only a prolonged period of time is required but also accurate judgment of the species is extremely difficult. In order to solve such problems, certain identification and detection methods have recently been considered and put into use, e.g., a method for judging the presence of a specific nucleotide sequence of a gene utilizing the polymerase chain reaction (to be referred to as "PCR" hereinafter) or the like, sub-classification of mycobacteria using an insertion sequence IS6110, and the like. The PCR method is suited for identifying and detecting slow growing mycobacteria from the viewpoint that quick and objective judgment is possible without requiring culturing.

In that case, the gene to be used is a rRNA gene in most cases. T. Rogall *et al*. (1990, *J. Gen. Microbiol*., 136, 1915 - 1920) have proposed a method for the identification of mycobacteria species based on PCR using 16S rRNA sequences. However, these primers could not distinguish between *Mycobacterium gastri* and *Mycobacterium kansasii*, which show different phenotype characteristics. On the other hand, B. Boddinghaus *et al*. (1990, *J. Clin*. *Microbiol*., 28: 1751 - 1759) have reported an oligonucleotide derived from 16S rRNA sequence, which is specific for human type mycobacterium group, avian type mycobacterium-paramycobacterium and *Mycobacterium intracellulare* group. Even the use of this oligonucleotide could not give necessary resolution for carrying out identification at species level. An identification method using these rRNA gene sequences is now on the market and available from Nippon Roche as a gene diagnosis kit under a trade name of "Amplicore Mycobacterium". In addition to this, detection or identification methods using rRNA sequences have been disclosed by Toyobo (JP-A-10-323189; the term "JP-A" as used herein means an "unexamined published Japanese patent application") and Becton, Dickinson and CO. (JP-A-10-057098). In order to solve the aforementioned problem of not being able to distinguish two species, an identification or detection method using the sequence of a region between 16S rRNA and 23S rRNA has been proposed by A. Roth *et al*. (1998, *J. Clin. Microbiol*., 36: 139 - 147). However, since the region between 16S rRNA and 23S rRNA has only about 200 base pairs, it is difficult to carry out high accuracy molecular phylogenic analysis by such a short sequence, and, when a new strain having an intermediate sequence which does not coincide with any one of the sequences of two strains is generated, it is not able to judge its closeness to which of them.

On the other hand, it was shown that more minute and accurate classification and identification of many bacteria including those of the genus *Pseudomonas* and the genus *Acinetobacter* is possible by using a gene which encodes a protein having high evolution rate, particularly a 1,200 bp sequence of *gyrB* gene (Yamamoto, S. and S. Harayama, 1995, *Appl. Environ. Microbiol*., 61: 1104 - 1109, Yamamoto, S. and S. Harayama, 1996, *Int. J. Syst. Bacteriol*., 46: 506 - 511, Harayama, S. and S. Yamamoto, 1996, pp. 250 - 258 in Molecular Biology of Pseudomonas, T. Nakazawa, K. Fukuda, D. Haas, S. Silver (eds), ASM Press, Washington, D.C., S. Yamamoto and S. Harayama, *Kagaku-to-Seibutsu* (Chemistry and Biology, Japan), 1996, vol. 34, no. 3, pp. 149 - 151, S. Yamamoto and S. Harayama*, Nippon Nogei Kagaku Kaishi* (Journal of Agricultural Chemistry, Japan), 1997, vol. 71, no. 9, pp. 894 - 897).

Attempts have been made to carry out identification of slow growing mycobacteria using genes coding for proteins other than the *gyrB* gene. For example, C.T. Shivannvar *et al*. have discussed on the phylogenic relationship among slow growing mycobacteria and their relationship to antigenicity using superoxide dismutase gene (1994, *J. Clin. Microbiol*., 32: 2801 - 2812), and D.S. Swanson *et al*. have attempted to carry out minute classification of avian type mycobacterium-paramycobacterium and *Mycobacterium intracellulare* group using a 65 kD heat shock protein gene (1997, *Int. J. Syst. Bacteriol*., 47: 414 - 419). In addition to the rRNA gene, Abbott Laboratories, USA, has disclosed a detection method which uses a gene coding for a protein antigen B of *Mycobacterium tuberculosis*, gene sequences of 65 kD heat shock protein, 10-kD heat shock proteins and the like of *Mycobacterium tuberculosis* and sequences related to insertion sequences IS987 and IS6110, in JP-W-10-500567 (the term "JP-W" as used herein means an "Japanese national publication of a PCT application") (International Publication No. WO 95/31571). In addition, Becton, Dickinson and Co. has disclosed in JP-A-06-319560 a detection or identification probe derived from a gene which encodes a 70 kD heat shock protein of *Mycobacterium paratuberculosis*. However, among these genes, only the *gyrB* gene shows no contradiction when molecular phylogenical data are compared with the identification of species by the conventional taxonomic means (Yamamoto and Harayama, 1998, *Int. J. Syst. Bacteriol*., 48: 813 - 819, Yamamoto *et al*., 199, *Int. J. Syst. Bacteriol*., 49: 87 - 95, Suzuki *et al*., *Int. J. Syst. Bacteriol*., in press, Kasai *et al*., *Int. J. Syst. Bacteriol*., in press).

A patent application relating to a method for the identification or detection of bacteria using the *gyrB* gene has already been filed by the present applicant (JP-A-11-16917). However, this document does not disclose identification and detection of slow growing mycobacteria and also does not teach or suggest which region of the *gyrB* gene can be used in carrying out identification and detection of slow growing mycobacteria.

Because the slow growing mycobacteria include the bacteria that cause tuberculosis and the like serious diseases, great concern has been directed toward the development of a method for accurately identifying and detecting this bacterial group. On the other hand, because the growth rate of slow growing mycobacteria is lower than that of general bacteria, it is difficult to identify or detect them by physiological and biochemical methods which essentially require culturing of bacteria.

The present invention has been accomplished under such technical background to provide a method for the identification or detection of slow growing mycobacteria, especially tubercle bacilli group bacteria, utilizing the *gyrB* gene.

With the aim of solving the aforementioned problems, the present inventors have conducted extensive studies and, as a result, found that at least a part of the nucleotide sequence of *gyrB* DNA is different among the slow growing mycobacteria.

The present inventors further determined *gyrB* gene sequences of standard strains of the slow growing mycobacteria. Taxonomic positioning of strains isolated from clinical cases was carried out based on these sequences. Then, the resulting taxonomic positioning was checked by the DNA-DNA hybridization method, which is a standard method for identifying species of bacteria. As a result, it was unexpectedly found that the taxonomic positioning determined by using *gyrB* gene sequences shows good agreement with the result of the conventional classification method.

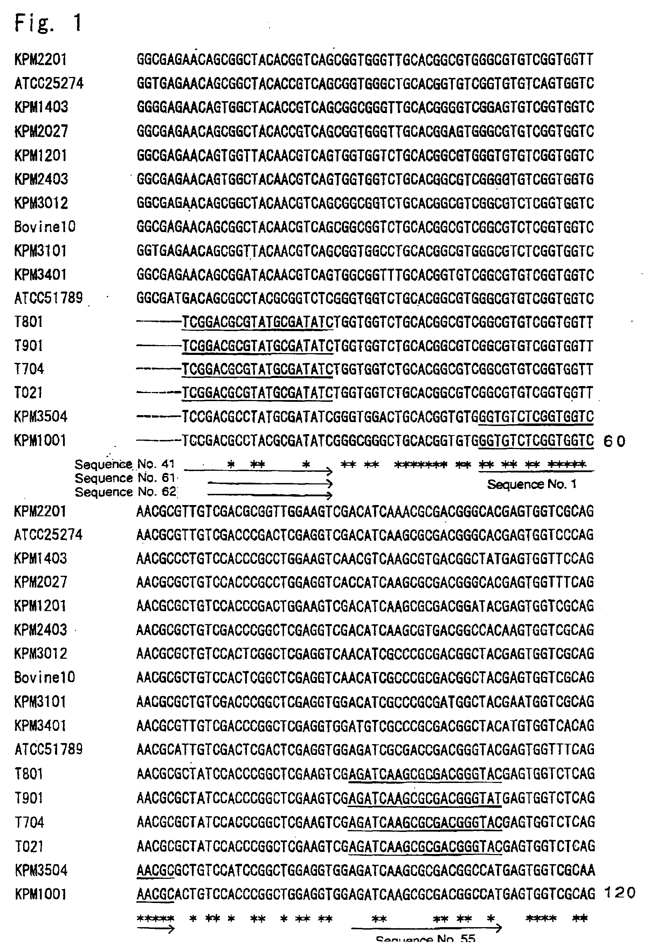

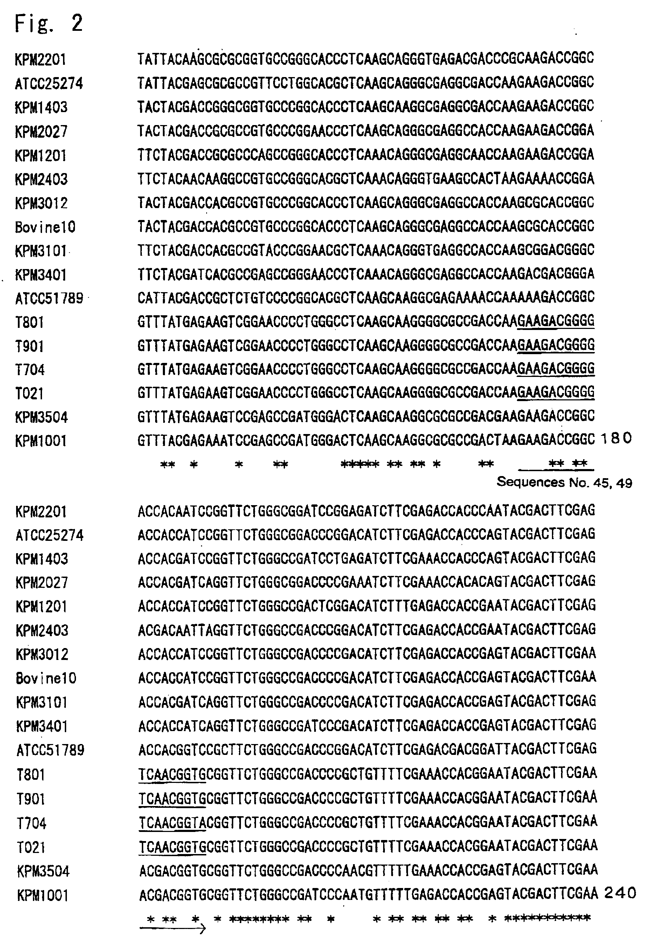

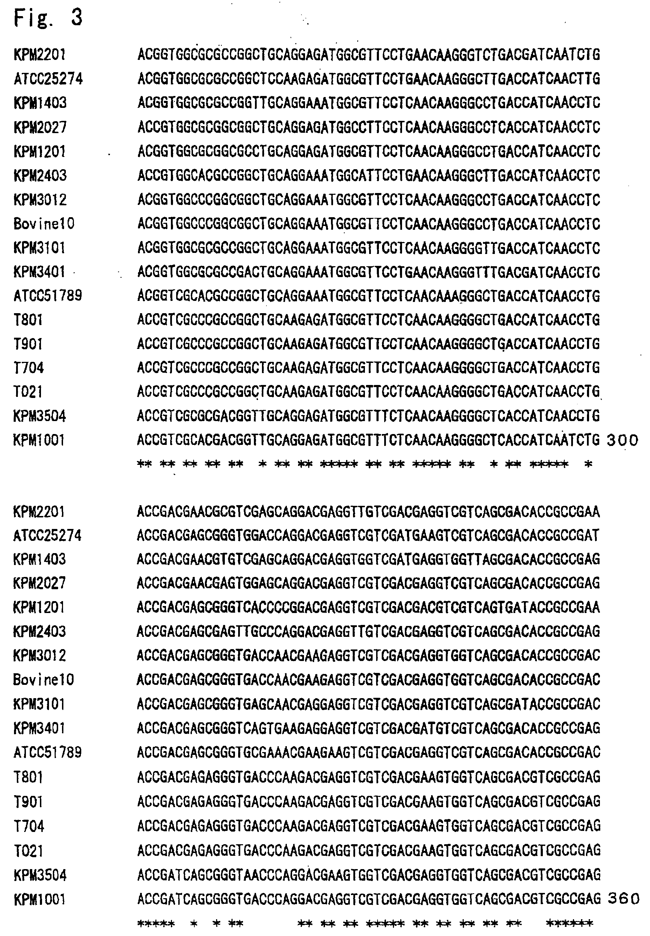

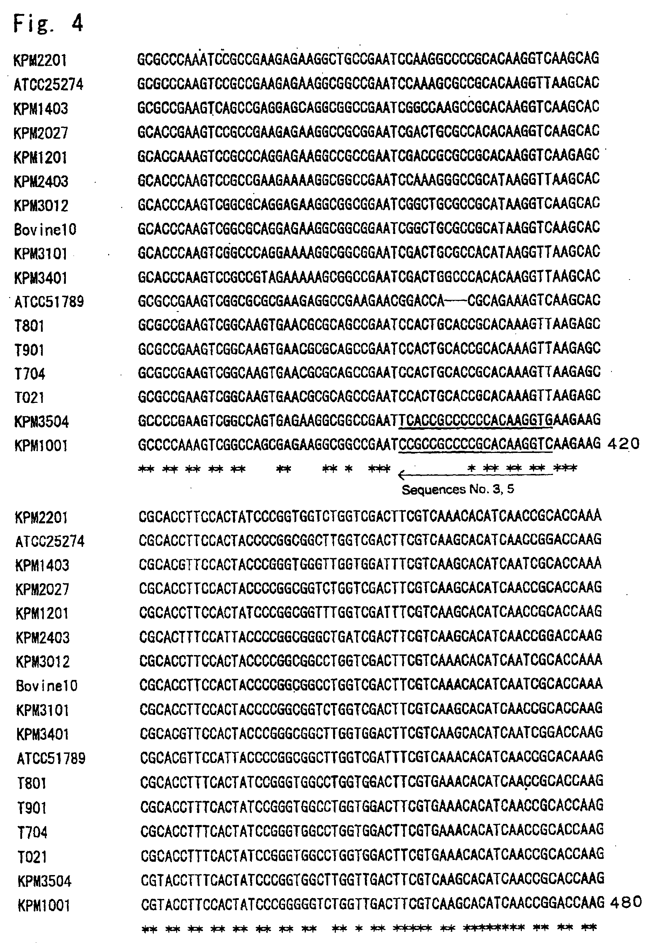

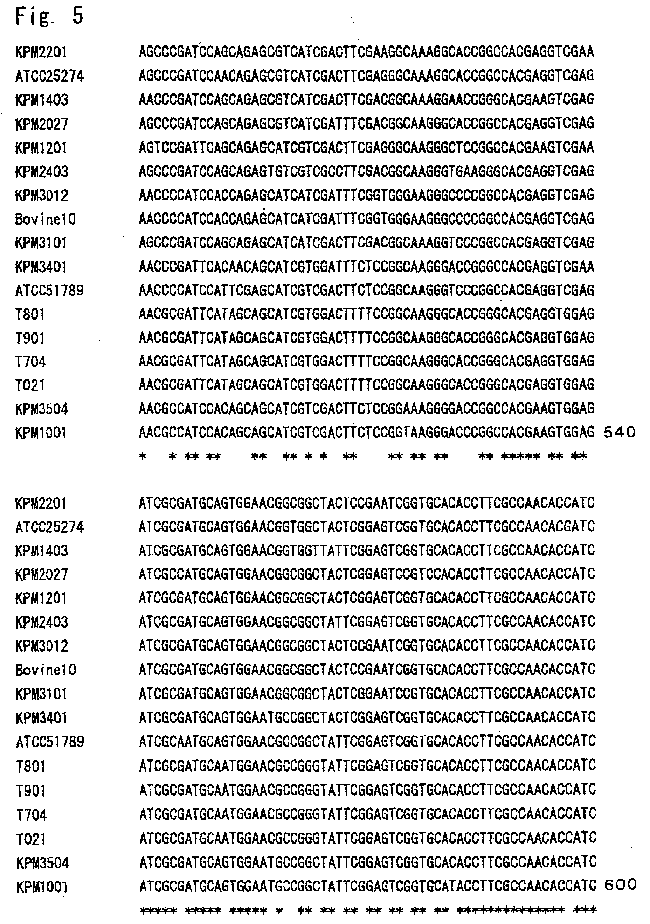

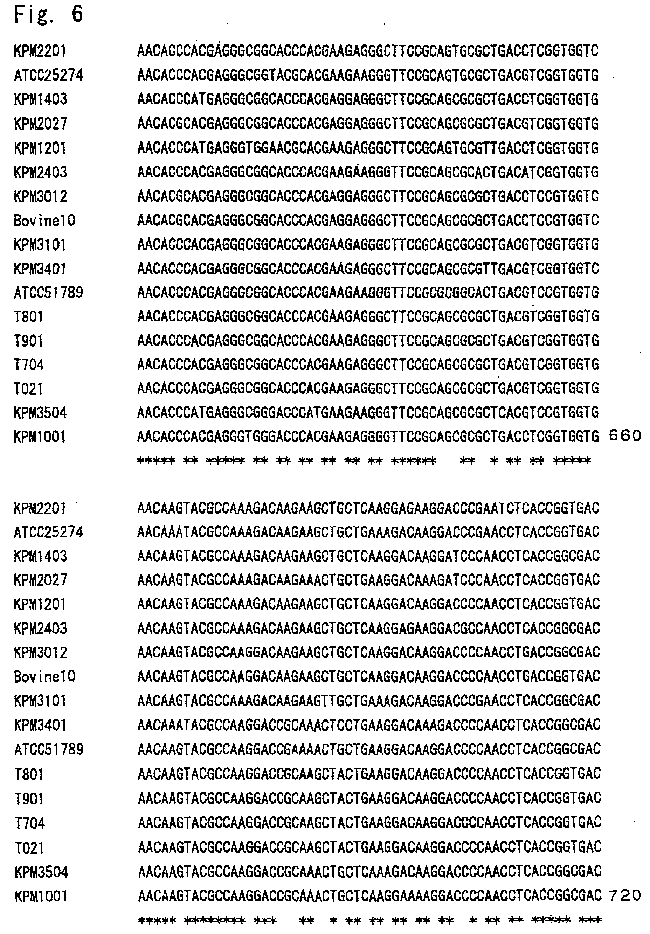

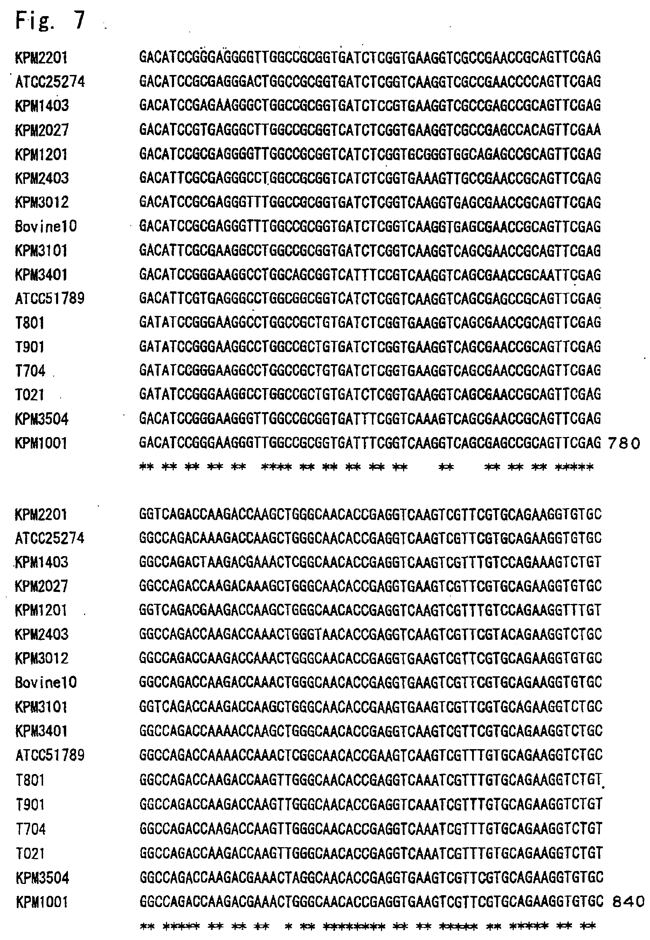

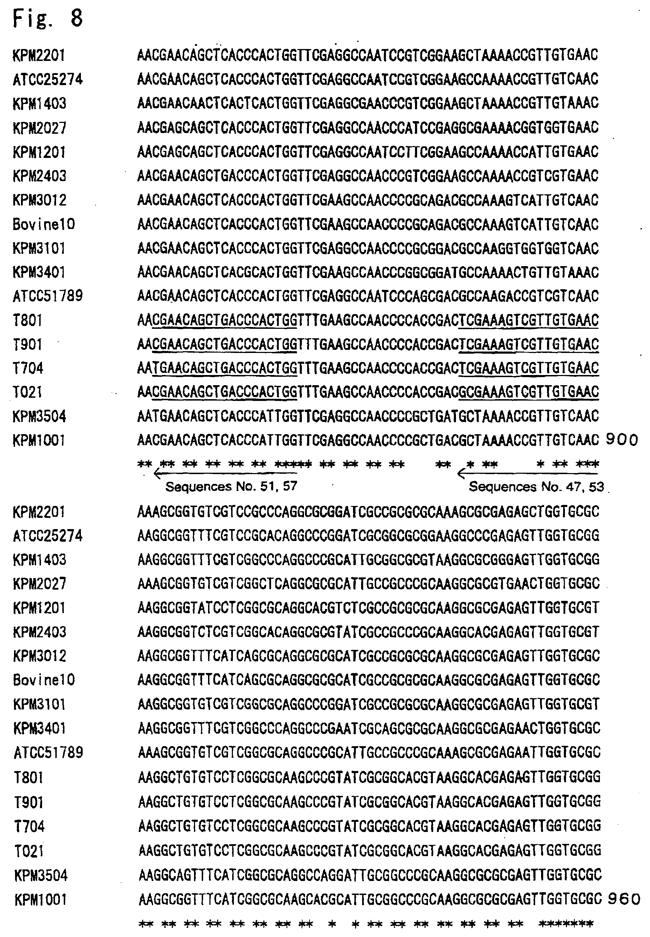

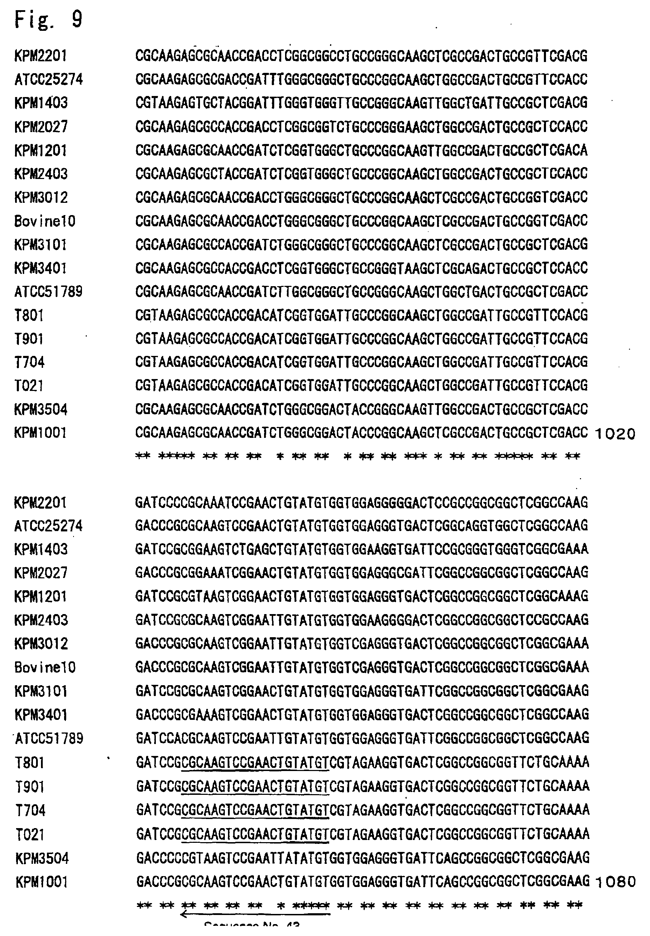

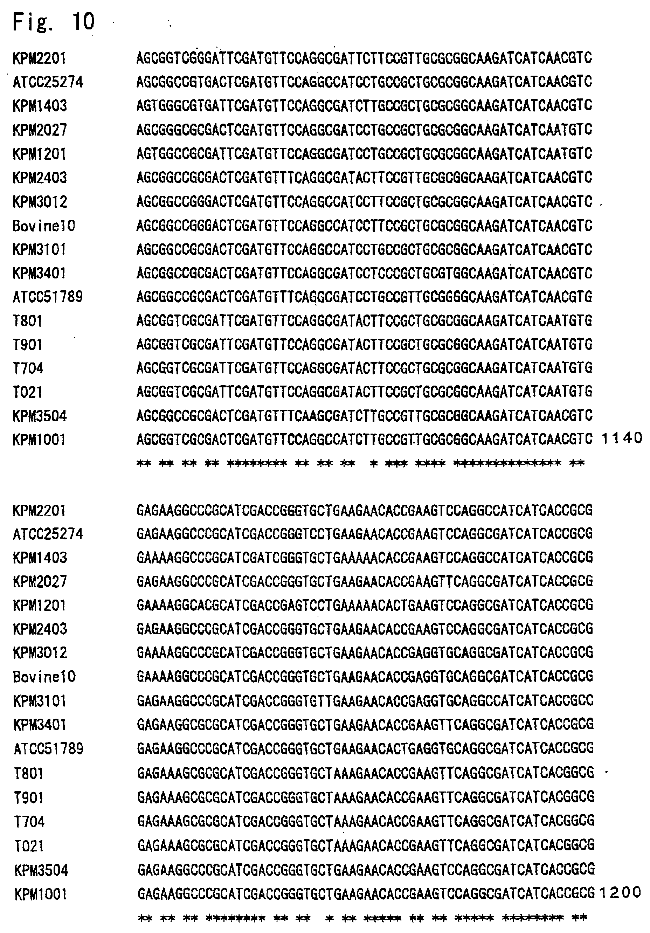

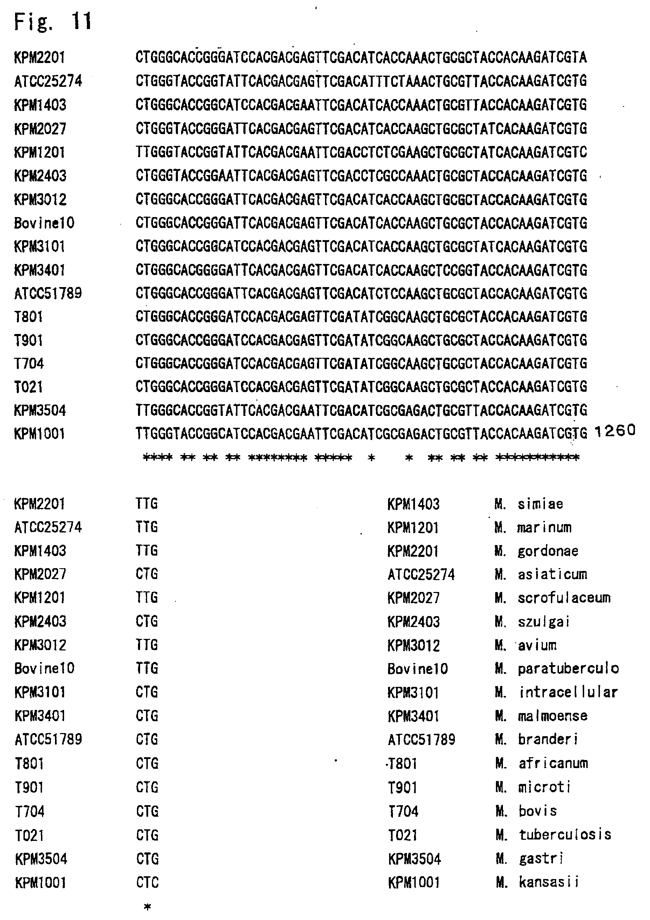

In addition, nucleotide sequences of *gyrB* fragments were determined by the PCR method by amplifying them from DNA samples of standard strains of atypical mycobacteria, *Mycobacterium gastri* and *Mycobacterium kansasii*, which cannot be distinguished by the nucleotide sequence of 16S rRNA gene which is the most generally used gene sequence-aided detection method of bacteria. When the resulting sequences were compared, it was found that the 16S rRNA gene sequence was identical in both strains, but 66 positions in the 1,257 base *gyrB* gene nucleotide sequence were different in both strains (Figs. 1-11). The present inventors further found that the taxonomically near bacteria belonging to the slow growing mycobacteria can be distinguished by designing primers based on such difference in their sequences, which renders possible the PCR amplification specific for each of these strains. Thus, the present inventors found that it can determine accurate molecular phylogenic position of even a newly isolated strain and also can distinguish related species which cannot be distinguished by other genes, so that it is a method superior to methods by other genes.

The present invention has been accomplished based on the above knowledge.

### SUMMARY OF THE INVENTION

Thus, the present invention relates to a method for identifying slow growing mycobacteria, especially tubercle bacilli group bacteria, which comprises carrying out identification of bacteria using *gyrB* DNA as a marker. Also, the present invention relates to a method for detecting slow growing mycobacteria, especially tubercle bacilli group bacteria, which comprises carrying out detection of bacteria using *gyrB* DNA as a marker.

The present invention further relates to a method for identifying slow growing mycobacteria, which comprises amplifying the regions corresponding to SEQUENCE NO. 7, 9, 11, 13, 15, 17, 19, 21, 23, 25, 27, 29, 31, 33, 35, 37 and 39 in the *gyrB* of slow growing mycobacteria, comparing nucleotide sequences of the amplified fragments with the nucleotide sequences described in SEQUENCE NO. 7, 9, 11, 13, 15, 17, 19, 21, 23, 25, 27, 29, 31, 33, 35, 37 and 39, thereby calculating genetic distance from each sequence, and carrying out identification of the aforementioned slow growing mycobacteria based on the genetic distance.

Also the present invention relates to a method for detecting a specific bacterium belonging to the slow growing mycobacteria using a specific sequence in the *gyrB*. In particular, the present invention relates to a method for detecting *Mycobacterium kansasii*, which comprises detecting *Mycobacterium kansasii* using, as a primer or probe, an oligonucleotide that contains a sequence coding for a part of or the entire portion of the amino acid sequence described in SEQUENCE NO. 4, or its complementary sequence, and also substantially functions as a primer or probe, and to a *Mycobacterium kansasii* detection kit which comprises the just described oligonucleotide.

The present invention also relates to a method for detecting *Mycobacterium gastri*, which comprises detecting *Mycobacterium gastri* using, as a primer or probe, an oligonucleotide that contains a sequence coding for a part of or the entire portion of the amino acid sequence described in SEQUENCE NO. 6, or its complementary sequence, and also substantially functions as a primer or probe, and to a *Mycobacterium gastri* detection kit which comprises the just described oligonucleotide.

### BRIEF DESCRIPTION OF THE DRAWINGS

Figs. 1 through 11 show alignment of the nucleotide sequences of various slow growing mycobacteria. The symbols at the left side in the figure indicate the organisms shown below.
   - KPM1403: *Mycobacterium simiae*
   - KPM1201: *Mycobacterium marinum*
   - KPM2201: *Mycobacterium gordonae*
   - ATCC25274: *Mycobacterium asiaticum*
   - KPM2027: *Mycobacterium scrofulaceum*
   - KPM2403: *Mycobacterium szulgai*
   - KPM3012: *Mycobacterium avium*
   - Bovine10: *Mycobacterium paratuberculo*
   - KPM3101: *Mycobacterium intracellular*
   - KPM3401: *Mycobacterium malmoense*
   - ATCC51789: *Mycobacterium branderi*
   - T801: *Mycobacterium africanum*
   - T901: *Mycobacterium microti*
   - T704: *Mycobacterium bovis*
   - T021: *Mycobacterium tuberculosis*
   - KPM3504: *Mycobacterium gastri*
   - KPM1001: *Mycobacterium kansasii*
Fig. 12 shows a result of the identification using the primers based on SEQUENCE No. 1, SEQUENCE NO. 3, and SEQUENCE No. 5. Panel A shows amplified results using *Mycobacterium kansasii*-specific primers (SEQUENCE NO. 1 and SEQUENCE NO. 3), and panel B using *Mycobacterium gastri*-specific primers (SEQUENCE NO. 1 and SEQUENCE NO. 5). Lanes 1 and 12 are molecular weight markers. Lanes 2: strain KPM 1001T, 3: strain KPM 1004, 4: strain KPM 1007, 5: strain KPM KY256, 6: strain KPM KY761, 7: strain KPM KY798, 8: strain KPM 1998-1, 9: strain KPM 3504T, 10: strain KPM 3502 and 11: strain KPM 3503.
Fig. 13 shows a phylogenetic tree of slow glowing mycobacteria prepared by the molecular phylogenic analysis. This figure shows an example in which the presence of new species of slow growing mycobacteria was shown by *gyrB* sequence analysis. By carrying out molecular phylogenetic analysis and comparing the thus obtained *gyrB* sequences with already known *gyrB* sequences, it was shown that a group of strains KPM 2212, 2014, 1988-5, 2209 and 2013 are new species.
Fig. 14 is an electrophoresis photograph of products amplified by PCR using primers specific for bacteria which constitute the tubercle bacilli.
Fig. 15 is an electrophoresis photograph of products amplified by PCR using primers specific for each bacterium which constitutes the tubercle bacilli.
Fig. 16 is an electrophoresis photograph of fragments prepared by digesting PCR products with restriction enzymes.

### DETAILED DESCRIPTION OF THE INVENTION

The following describes the present invention in detail.

As the first step for the detection or identification of the slow growing mycobacteria (especially tubercle bacilli group bacteria), a sample for detection or identification is collected. Examples of the sample include a sample collected from organisms (human, animals, etc.) showing tuberculosis or tuberculosis-analogous symptoms as well as a strain isolated from the sample. Examples of tuberculosis or tuberculosis-analogous symptoms include pneumonia, empyema, cystitis, pyelonephritis, prostatitis, peritonitis, pericarditis, meningitis, encephalitis, etc. (Pocket guide to clinical microbiology 2nd edition, Oatrick R. Murray, ASM press). The collected sample may be cultured or the microorganism in the sample may be isolated and cultured for the use in the following steps. However, the present invention is advantageous in that the sample as it is can be used.

Then, a sample or isolated microorganism is usually subjected to a treatment to destroy the cell to extract DNA from the cell. The method for this treatment is not particularly limited and includes physically destroying method, chemically destroying method, etc.

The sequence of the DNA gyrase β subunit in the sample is determined by using the conventional way. Examples of the method for determining the DNA sequence include the dideoxy terminator method (Molecular Cloning: a laboratory manual 2nd edition, J. Sambrook, E. F. Rritsch, T. Maniatis, CSH press). The sequence determined is compared with the sequences of the DNA gyrase β subunit of the slow growing mycobacteria (Sequence No. 7, 9, 11, 13, 15, 17, 19, 21, 23, 25, 27, 29, 31, 33, 35, 37, 39) to determine whether the microorganism in the sample belongs to the one of these slow growing mycobacteria or is a new species. For the determination, 85% to 100% homology with respect to the 1200 bp sequence of the DNA gyrase β subunit means the same species, while the homology less than 85% means a new species. As a surprising matter, the identification of the slow glowing mycobacteria based on the DNA gyrase β subunit well matches with the identification of the slow glowing mycobacteria by the conventional method. Thus, the present invention makes it possible to identify the slow growing mycobacterial in an accurate way and also makes it possible to distinguish the related species, which are not possible by the conventional way.

Without determining the whole sequence of the DNA gyrase β subunit, identification and detection of the slow growing mycobacteria according to the present invention can be carried out by utilizing one or more unique partial sequence in the DNA gyrase β subunit which is characteristic to one or more of the microorganisms belonging to the slow growing mycobacteria or related species thereof. Example of the unique sequence is a sequence having 0 or more, preferably at least 1, more preferably at least 2, and most preferably at least 3 unique bases in the sequence having a length of 5-mer to 50-mer, preferably 10-mer to 40-mer, more preferably 15-mer to 30-mer. When the unique sequence does not have a unique base, at least one unique base should exist at the 3'-side or 5'-side nearest neighbor base to the unique sequence. The complementary sequence to the unique sequence can be also used.

The unique base means a base which can be found in only one or only several related species among the slow growing mycobacteria. The unique base may be located at arbitrary position in the unique sequence. When the unique sequence is utilized as a primer for the PCR, a unique base located near the 3'-end is preferable for the 5'-end primer and a unique base located near the 5'-end is preferable for the 3'-end primer. For the method utilizing the gel electrophoresis described below, the unique sequence may be designed so that the 3'-side or 5'-side nearest neighbor base to the unique sequence is the unique base (i.e., the unique base is not contained in the unique sequence). Even if a unique sequence which is unique to a certain one species among the slow growing mycobacteria is not found, identification or detection of the certain species is possible by using in combination two or more unique sequences which are respectively unique to several species among the slow growing mycobacteria. For example, four tubercle bacilli group bacteria can be identified or detected by using Sequence 41 shown in Fig. 1. By using Sequence 55 in combination of Sequence 41, it is possible to identify or detect *Mycobacterium microti*. *Mycobacterium kansasii* and *Mycobacterium gastri* can be identified or detected by using Sequence 1 shown in Fig. 1. By using Sequence 3 in combination of Sequence 1, it is possible to identify or detect *Mycobacterium kansasii*. According to the present invention, a sample obtained from human or animals showing tuberculosis or tuberculosis-analogous symptoms is used. Accordingly, it is possible to avoid pseudo positive reaction even if there are microorganisms other than slow growing mycobacteria that have the same unique sequence in the DNA gyrase β subunit.

Examples of the concrete methods for identifying or detecting the slow growing mycobacteria, which utilizes the unique sequence, a partial sequence in the unique sequence, or a sequence having a unique sequence, in the DNA gyrase β subunit include (1) DNA chip (DNA microarray) (Gingeras et al., 1998, Genome Res. 8: 435-448; Troesch et al. 1999 J. Clin. Microbiol. 37: 49-55), (2) PCR using the same as primers (Kasai, H., Ezaki, T., Harayama, S. 2000. J. Clin. Microbiol. 38: 301-308), (3) hybridization using the same as a probe (de los Reyes et al. 1997. Appl. Environ. Microbiol. 63: 11007-1117), (4) cleavage by the restriction enzyme that recognizes the unique sequence (Kasai H., Ezaki, T., Harayama, S. 2000. J. Clin. Microbiol. 38: 301-308), and the like. Examples of the method to confirm the result of these methods include a method to confirm the existence of the amplified or cleaved fragments by the gel electrophoresis, a method using DNA chip (DNA microarray), etc. The above-described methods can be carried out by the known way (cf. Molecular Cloning: a laboratory manual 2nd edition, J. Sambrook, E. F. Fritssh, T. Maniatis, CSH press; Current protocols of molecular biology edited by Ausubel et al. Wiley; PCR primer - A laboratory manual. edited by Diffenbach & Dveksler. SCH press, all herein incorporated by reference) The identification method and the detection method according to the present invention are further described below.

### (1) Identification method

The method of the present invention for identifying slow growing mycobacteria is characterized in that the regions corresponding to SEQUENCE NOS. 7, 9, 11, 13, 15, 17, 19, 21, 23, 25, 27, 29, 31, 33, 35, 37 and 39 in the *gyrB* of slow growing mycobacteria are amplified by PCR, nucleotide sequences of the amplified fragments are compared with the nucleotide sequences described in SEQUENCE NOS. 7, 9, 11, 13, 15, 17, 19, 21, 23, 25, 27, 29, 31, 33, 35, 37 and 39, thereby calculating genetic distance from each sequence, and then identification of the aforementioned slow growing mycobacteria is carried out based on the genetic distance.

The term "identification" as used herein means that taxonomic positions of bacteria are determined by a molecular phylogenic or the like means.

Though not particularly limited, the primers represented by SEQUENCE NO. 59 and SEQUENCE NO. 60 can be exemplified as the primers to be used in the amplification of the regions corresponding to SEQUENCE NOS. 7, 9, 11, 13, 15, 17, 19, 21, 23, 25, 27, 29, 31, 33, 35, 37 and 39 in the *gyrB*.

Relationship between the nucleotide sequences of SEQUENCE NOS. 7, 9, 11, 13, 15, 17, 19, 21, 23, 25, 27, 29, 31, 33, 35, 37 and 39 and their corresponding amino acid sequences and names of original microorganisms is shown in the following table.

**TABLE 1**

| Nucleotide sequence | Amino acid sequence | Name of original microorganisms |
|---|---|---|
| SEQUENCE NO. 7 | SEQUENCE NO. 8 | *Mycobacterium simiae* |
| SEQUENCE NO. 9 | SEQUENCE NO. 10 | *Mycobacterium bovis* |
| SEQUENCE NO. 11 | SEQUENCE NO. 12 | *Mycobacterium szulgai* |
| SEQUENCE NO. 13 | SEQUENCE NO. 14 | *Mycobacterium malmoense* |
| SEQUENCE NO. 15 | SEQUENCE No. 16 | *Mycobacterium intracellulare* |
| SEQUENCE NO. 17 | SEQUENCE NO. 18 | *Mycobacterium avium* |
| SEQUENCE NO. 19 | SEQUENCE NO. 20 | *Mycobacterium gordonae* |
| SEQUENCE NO. 21 | SEQUENCE NO. 22 | *Mycobacterium africanum* |
| SEQUENCE NO. 23 | SEQUENCE NO. 24 | *Mycobacterium tuberculosis* |
| SEQUENCE NO. 25 | SEQUENCE NO. 26 | *Mycobacterium gastri* |
| SEQUENCE NO. 27 | SEQUENCE NO. 28 | *Mycobacterium marinum* |
| SEQUENCE NO. 29 | SEQUENCE NO. 30 | *Mycobacterium microti* |
| SEQUENCE NO. 31 | SEQUENCE NO. 32 | *Mycobacterium asiaticum* |
| SEQUENCE NO. 33 | SEQUENCE NO. 34 | *Mycobacterium scrofulaceum* |
| SEQUENCE NO. 35 | SEQUENCE NO. 36 | *Mycobacterium branderi* |
| SEQUENCE NO. 37 | SEQUENCE NO. 38 | *Mycobacterium paratuberculosis* |
| SEQUENCE NO. 39 | SEQUENCE NO. 40 | *Mycobacterium kansasii* |

The genetic distance can be calculated in accordance, for example, with the method described by Felsenstein in the Phylip program (Felsenstein, J., 1993 PHYLIP (Phylogeny Inference Package) version 3.5c. Distributed by the author, Department of Genetics, University of Washington, Seattle, U.S.A.).

### (2) Specific detection

The method of the present invention for detecting *Mycobacterium kansasii* is characterized by the use, as a primer or probe, of an oligonucleotide which contains a sequence coding for a part of or the entire portion of the amino acid sequence described in SEQUENCE NO. 4, or its complementary sequence, and functions substantially as a primer or probe. Also, the *Mycobacterium kansasii* detection kit of the present invention is characterized in that it contains the just described oligonucleotide.

The method of the present invention for detecting *Mycobacterium gastri* is characterized by the use, as a primer or probe, of an oligonucleotide which contains a sequence coding for a part of or the entire portion of the amino acid sequence described in SEQUENCE NO. 6, or its complementary sequence, and also functions substantially as a primer or probe. Also, the *Mycobacterium gastri* detection kit of the present invention is characterized in that it contains the just described oligonucleotide.

In this connection, the term "substantially functions as a primer or probe" means that the oligonucleotide has such a length that a specific annealing or hybridization can be effected, and its gist is to exclude the oligonucleotide which has a sequence that can anneal to or hybridize with the DNA to be detected but cannot be used in specific detection, because it frequently causes nonspecific annealing or hybridization due to its short length. In order to confirm that a certain oligonucleotide can substantially functions as a primer for PCR, the PCR is carried out at a 3°C higher annealing temperature and a 3°C lower annealing temperature than the usually employed temperature for the PCR. If the PCR product is observed only at 3°C lower annealing temperature, there is a possibility of false-positive. In such a case, the nucleotide sequence of the amplified fragment is determined by the conventional way and compared with the known sequence to confirm whether the oligonucleotide used can substantially work as a primer. In order to confirm that a certain oligonucleotide can substantially function as a primer for PCR, it is preferable to perform PCR by using DNA of already known strain (for example, type strain) as a template for positive and negative controls.

Though not particularly limited, the oligonucleotide represented by SEQUENCE NO. 3 can be exemplified as an oligonucleotide which can be used in the detection of *Mycobacterium kansasii*, and the oligonucleotide represented by SEQUENCE NO. 5 can be exemplified as an oligonucleotide which can be used in the detection of *Mycobacterium gastri*.

Preparation of DNA to be tested, preparation of primers and PCR using the same, and preparation of probes and hybridization using the same can be carried out in the usual way without requiring special techniques.

Regarding the primers to be used in PCR, it is not always necessary that both of them can perform specific annealing, and one of them may perform nonspecific annealing. The primer represented by SEQUENCE NO. 1 can be cited as an example of such a primer which performs nonspecific annealing.

The methods of the present invention for identifying and detecting the slow growing mycobacteria (especially, tubercle bacilli group bacteria) are characterized in that *gyrB* DNA is used as a marker. Examples of the slow growing mycobacteria include those shown in Table 1. Examples of the tubercle bacilli group bacteria include *Mycobacterium tuberculosis*, *Mycobacterium bovis*, *Mycobacterium africanum* and *Mycobacterium microti*.

The following four methods can be exemplified as the identification and detection methods which use *gyrB* DNA as a marker.

### A) A method which employs PCR

This method is carried out as follows.
(1) An oligonucleotide which contains a region of *gyrB* DNA, a region that has different nucleotide sequence among tubercle bacilli group bacteria, is synthesized. Since the nucleotide sequence of *gyrB* DNA corresponding to each bacterium is already determined as shown in Figs. 1-11, the just described oligonucleotide can be synthesized based on these drawings. As the oligonucleotide, an oligonucleotide which encodes the amino acid sequence described in SEQUENCE NO. 46, SEQUENCE NO. 48, SEQUENCE NO. 50, SEQUENCE NO. 52, SEQUENCE NO. 54, SEQUENCE NO. 56 or SEQUENCE NO. 58 can be exemplified as a preferable oligonucleotide, and an oligonucleotide represented by SEQUENCE NO. 45, SEQUENCE NO. 47, SEQUENCE NO. 49, SEQUENCE NO. 51, SEQUENCE NO. 53, SEQUENCE NO. 55 or SEQUENCE NO. 57 can be exemplified as a particularly preferable oligonucleotide.
(2) A solution which contains the oligonucleotide synthesized in the above step, dNTP, DNA polymerase and a bacterial DNA to be used as a sample is prepared. Concentration of each component contained in the solution may be the same as that in the reaction solution used in general PCR. It is not necessary to purify the bacterial DNA to be used as a sample, and disrupted cells may be used as such for example.
(3) The solution prepared in the above step is repeatedly heated under such conditions that PCR can be generated. The heating temperature, cycle and the like conditions are not particularly limited, with the proviso that they are within such ranges that PCR can be effected, but, since the homology of *gyrB* DNA among tubercle bacilli group bacteria is high as shown in Figs. 1-11, it is desirable to set the temperature at the time of annealing to a fairly high level. Illustratively, it is desirable to set at 68°C or more. When the synthesized oligonucleotide can be hybridized with the added bacterial DNA, PCR occurs by the repetition of heating and amplified product is formed thereby. On the other hand, when the synthesized oligonucleotide cannot be hybridized with the added bacterial DNA, PCR does not occur and amplified product is not formed.
(4) Electrophoresis of the solution after the above treatment is carried out. When the amplified product is contained in the solution, its corresponding band is formed on the electrophoresis gel. In consequence, identification and detection of the bacterium of interest can be made based on the electrophoresis pattern.

### B) A method which uses restriction enzyme digestion fragments

This method is carried out as follows.
(1) An oligonucleotide which is identical to a part of *gyrB* DNA of tubercle bacilli group bacteria and a complementary oligonucleotide of the aforementioned part of DNA are synthesized. Since the nucleotide sequence of *gyrB* DNA corresponding to each bacterium is already determined as shown in Figs. 1-11, the bust described oligonucleotides can be synthesized based on these drawings. As preferred oligonucleotides, an oligonucleotide which encodes the amino acid sequence described in SEQUENCE NO. 42 and an oligonucleotide which encodes the amino acid sequence described in SEQUENCE NO. 44 can be exemplified, and the oligonucleotide represented by SEQUENCE NO. 41 and the oligonucleotide represented by SEQUENCE NO. 43 can be exemplified as particularly preferred oligonucleotide.
(2) PCR is carried out using the two oligonucleotides synthesized in the above step as primers, and a bacterial DNA sample as a template. It is not necessary to purify the bacterial DNA to be used as a sample, and disrupted cells may be used as such for example. PCR can be carried out in the usual way.
(3) The DNA fragment amplified in the above step is digested with restriction enzymes. The restriction enzymes to be used are not particularly limited, with the proviso that they can generate different fragments among corresponding bacteria which constitute tubercle bacilli. For example, *Rsa* I and *Taq* I can be exemplified as such restriction enzymes.
(4) Electrophoresis of the fragments digested in the above step is carried out. The digested DNA fragments appear at positions corresponding to their length. In consequence, identification and detection of the bacterium of interest can be made based on the electrophoresis pattern.

The aforementioned two methods can be cited as typical examples of the identification or detection method of the present invention, but other methods are also included in the identification or detection method of the present invention, with the proviso that they use *gyrB* DNA as a marker. Examples of these other identification or detection methods include a method in which *gyrB* DNA is amplified by PCR, and identification or detection of the bacterium of interest is carried out by determining nucleotide sequence of the amplified fragment and a method in which an oligonucleotide which contains a region of *gyrB* DNA, a region that has different nucleotide sequence among tubercle bacilli group bacteria, is synthesized, and the bacterium of interest is identified or detected by carrying out Southern blotting using the oligonucleotide as a probe.

### C) Method which employs the gel electrophoresis

According to this method, easy and qualitative analysis as well as a certain degree of quantitative analysis are possible for *M. tuberculosis, M. bovis, M. africanum, M. microti, M. kansasii, M. avium, M. intracellulare* and for the multiple infection found in the patient having lowered immunological competence.

Dideoxy nucleotides and primers that are unique to one or more of these species are used for this method. The oligonucleotide used as a primer is designed so that the 3'-side nearest neighbor base in DNA gyrase β subunit sequence to the primer is the unique base. The sequence of the primer itself may be common in all of the slow growing mycobacteria or may be common in one or more slow growing mycobacteria. In the latter case, an oligonucleotide mixture is preferably used in order to assure the reaction.

All of the 4 types of dideoxy nucleotides is labeled with a fluorescent substance or radioactive substance. By labeling each of the 4 dideoxy nucleotides with different types of substances, it is possible to obtain the necessary information from only one lane of the sequence gel. Labeling the 4 dideoxy nucleotides with the same fluorescent substance or radioactive substance requires to carry out electrophoresis using 4 different lanes.

The reaction mixture used for this method is the same with the reaction mixture for the usual sequence reaction except that dATP, dTTP, dGTP, and dCTP are not contained. In other words, the reaction mixture contains, as essential components, an appropriate buffer, a DNA polymerase, a labeled ddNTP, and the primer. A sample collected from the patient is mixed with this reaction mixture and then subjected to the reaction at an appropriate temperature at which the reaction can occur (for example, at 95°C for 10 seconds, 50°C for 5 seconds, and 60°C for 4 minutes; 25 cycles). Then, existence of the labeled primer is checked, for example, by subjecting the reaction product to the gel electrophoresis by the conventional way, or the like. The pattern which appears on the gel differs depending on the length of the primer used and the type of the 3'-side nearest neighbor based to the primer. The location of the primer sequence is not particularly limited as long as the length of the primer sequence is the same. However, for the quantitative analysis, primers having a quite high ΔTm (about 5°C or more) is not preferable. Examples of the primers include the nucleotides represented by Sequence 61 (5'-gacgcstaygcgatatc-3') based on the *M. tuberculosis* complex and *M. kansasii* and Sequence 62 (5'-agcggytacaacgtcag) based on *M. avium* and *M. intracellulare* in Fig. 1. At the position corresponding to the 18-base length, a signal of "T" is found in the case of *M. tuberculosis* complex, "G" for *M. kansasii*, and "C" for *M. intracellulare*. Detection of a plural number of signals at the position corresponding to the 18-base length means the multiple infection. Moreover, approximate amount of existence of each species can be estimated from the signal intensity detected. The sequence that is unique to 4 species of the tubercle bacilli group bacteria and the sequence which can distinguish *M. gastri* (a species which is near to *M. kansasii* but has only a few number of clinical cases of human infection) have been explained in the above-described methods. By combining this method which uses gel electophoresis with the above-described methods, further detailed identification or detection is possible.

### D) Method which employs the DNA chip

The detection or identification of the slow growing mycobacteria is also possible by utilizing the DNA chip. Examples of the method which employs the DNA chip is described below. First, the region in the DNA gyrase β subunit in one or more standard strains of the slow growing mycobacteria is amplified, for example, by the PCR. Then, the amplified product is labeled by Cy5 or the like, and the synthesized DNA oligo probe is fixed on a plate such as slide grass. A DNA in a sample is obtained and subjected to a hybridization reaction on the plate having a solid phrased probe, which is then subjected to washing and detection in the conventional way. The size of the region in the DNA gyrase β subunit is preferably 250 bp or less, more preferably 180 bp or less, and still more preferably 125 bp or less. The oligo probe size is preferably from 14 to 17 mer.

Known protocols for the method which employs the DNA chip can be employed for the above-described method (cf. Lemieux, B., Aharoni, A., and M. Schena (1998), Overview of DNA Chip Technology, Molecular Breeding, 4, 277-289; Schena, M., Heller, R.A., Theriault, T.P., Konrad, K., Lachenmeier, E., and R.W. Davis (1998), Microarrays: biotechnology's discovery platform and functional genomics, Trends in Biotechnology, 16, 301-306; and Heller, R.A., Schema, M., Chai, A., Shalom, D., Bedilion, T., Gilmore, J., Woolley, D.E., and Davis, R.W. (1997), Discovery and analysis of inflammatory disease-related genes using cDNA microarrays, Proceedings of the National Academy of Sciences USA, 94, 2150-2155), all herein incorporated by reference).

Illustrative but non-limiting example of the protocol for the DNA chip method is described below.

### (1) Labeling

PCR amplification product (125 bp) is obtained by 40-cycles treatment (each cycle: at 96°C for 1 minute, 55°C for 30 seconds, and 72°C, 2 minutes). The product is then subjected to the ethanol precipitation. Then, 10 µM Cy5-dCTP/100 µl is added instead of dCTP/100 µl and the 40-cycles treatment was carried out again (each cycle: at 96°C for 1 minute, 55°C for 30 seconds, and 72°C, 2 minutes). Then, the product is subjected to the ethanol precipitation.

### (2) Spotting

Spotting is carried out under the following conditions.
Slide: Silylated Slides
Spotting: Spotting by SPBIO (manufactured by HITACHI) using a 4 Pin head with a pitch of 1.0 mm. A 20 µl sample (10 µl of 200 µM probe + 10 µl of ×2 Spotting Solution (Arraylt™)) is used for each well of the plate (about 4 to 5 nl per 1 spot).
Time: about 10 minutes/slide (96 spots)
Oligo probe size: conc. probe 14 - 17 mer, final conc. 100 µM

### (3) Hybridization

Hybridization is carried out using UniHyb™ (Arraylt™). The labeled product is dissolved in 4 µl of sterilized water and 16 µl of ×1.25 UniHyb™ was added.

Then, 9.6 µl of the resulting mixture was dropped onto a cover slip (24 × 32 mm; 1.25 µl/cm²) and the cover slip was placed onto the microarray such that bubbles are not included between the cover slip and the microarray. Then, the microarray is incubated at 46°C for 4 hours.

### (4) Washing

Washing is carried out with 2 × SSC (+ 0.2% SDS) for 5 minutes at room temperature, with 0.1 × SSC (+ 0.2% SDS) for 5 minutes at room temperature, and then with 0.1 × SSC (+ 0.2% SDS) for 5 minutes at room temperature. The microarray is centrifuged and dried. At least 1 week storage at 4°C is possible.

### (5) Scanning

Scanning is carried out by using ScanArray 1000 (ScanArray Lite) manufactured by GSI LUMONICS.
Scanning software: ScanArray
Analyzing software: QuantArray

### EXAMPLE 1

Using the oligonucleotides represented by the nucleotide sequences described in SEQUENCE NO. 39 and SEQUENCE NO. 40, *gyrB* gene sequences of 8 acid-fast bacterial strains (KPM 2201T, KPM 2202, KPM 2203, KPM 2013, KPM 2014, KPM 1988-5, KPM 2209 and KPM 2212) isolated from clinical cases were determined. Using the thus obtained *gyrB* sequences and a *gyrB* sequence set (SEQUENCE NO. 7 to SEQUENCE NO. 40) for slow growing mycobacteria identification use, their phylogenic relationship was estimated by a molecular phylogenic analysis. The molecular phylogenic analysis was carried out in the following manner using general-purpose molecular phylogenic analysis programs Clustal W (Thompson, J.D., D.G. Higgins and T.J. Gibson, 1994, Clustal W: Improving the sensitivity of progressive multiple sequence alignment through sequence weighting, positions-specific gap penalties and weight matrix choice., *Nucleic Acids Res*., *22*: 4673 - 4680) or Phylip (Felsenstein, J., 1993 PHYLIP (Phylogeny Inference Package) version 3.5c. Distributed by the author, Department of Genetics, University of Washington, Seattle, U.S.A.), in accordance with the instructions for use of both programs. A multiple alignment file is prepared by the Clustal W program from the *gyrB* gene sequences obtained using the oligonucleotides represented by the nucleotide sequences described in SEQUENCE NO. 59 and SEQUENCE NO. 60 and the slow growing mycobacteria identification *gyrB* sequence set of SEQUENCE NO. 7 to SEQUENCE NO. 40. An example of the parameters to be used in making the multiple alignment is "Gap Open Penalty: 15.00; Gap Extension Penalty: 6.66; DNA weight matrix: IUB; DNA transition weight: 0.5". The thus obtained multiple alignment is compared with a multiple alignment file obtained from amino acid sequences, and questionable points are corrected. Next, the genetic distance between respective sequences is calculated based on the multiple alignment file. The dnadist program of Phylip is used for the calculation. The calculation is carried out in accordance with the Kimura 2-parameter model. A phylogenetic tree is prepared from the thus obtained genetic distances by a neighboring sequences binding method. Correctness of the phylogenetic tree is checked by calculating the bootstrap probability.

On the other hand, the aforementioned 8 strains were also identified by a 16S rRNA gene-aided method and a biochemical method. The above results are shown in Table 2.

**TABLE 2**

| Strain name | Biological test | 16S rRNA gene | DNA homology test |
|---|---|---|---|
| KPM 2201T | *M. gordonae* | *M. gordonae* | *M. gordonae* |
| KPM 2202 | *M. gastri* | *M. gordonae* | *M. gordonae* |
| KPM 2203 | *M. gastri* | *M. gordonae* | *M. gordonae* |
| KPM 2013 | *M. scrofulaceum* | *M. gordonae* | new species |
| KPM 2014 | *M. scrofulaceum* | *M. gordonae* | new species |
| KPM 1988-5 | *M. scrofulaceum* | *M. gordonae* | new species |
| KPM 2209 | *M. scrofulaceum* | *M. gordonae* | new species |
| KPM 2212 | no data | *M. gordonae* | new species |

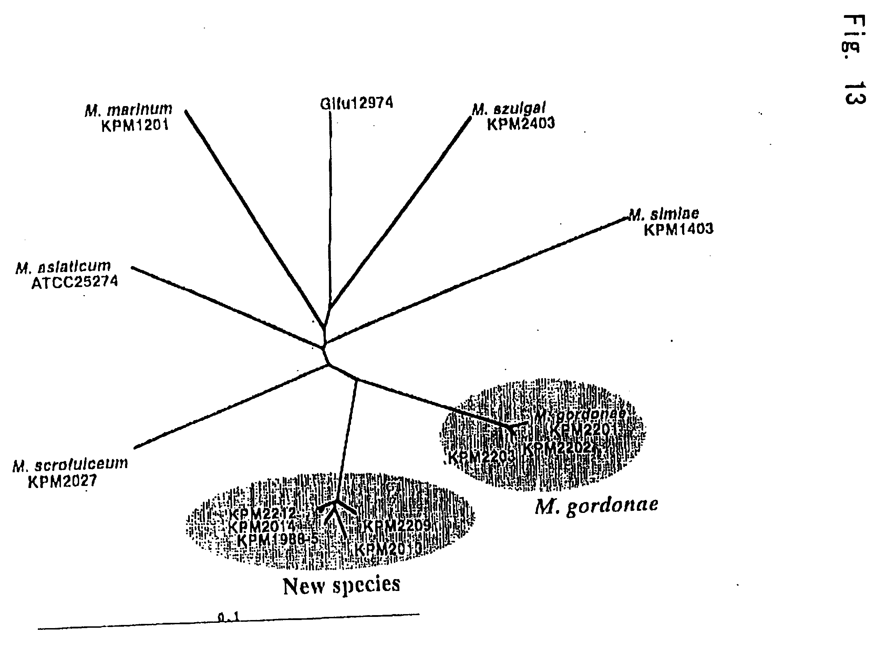

As shown in the table, 3 of the above 8 strains, namely KPM 2201T, KPM 2202 and KPM 2203, were identified as strains belonging to *Mycobacterium gordonae*, but the other 5 strains, KPM 2013, KPM 2014, KPM 1988-5, KPM 2209 and KPM 2212, were suggested to be sibling species of *Mycobacterium gordonae* but different species (new species) (Fig. 13). When a DNA-DNA hybridization test (Ezaki, T., Hashimoto, Y., Takeuchi, T., Yamamoto, H, Shu-Lin Liu, Matsui, K. & Yabuuchi, E (1988), *J. Clin. Microbiol*., 26, 1708 - 1713; Ezaki, T., Hashimoto, Y., Takeuchi & Yabuuchi, E (1989), *Int. J. Syst. Bacteriol*., 39, 224 - 229) was carried out in order to inspect this result, it was supported that they are new species. This result suggests that the *gyrB* sequence set for slow growing mycobacteria identification use gives highly reliable results for not only known strains but also strains of new species.

### EXAMPLE 2

Nucleotide sequences of the *gyrB* gene of *Mycobacterium kansasii* and *Mycobacterium gastri* (Figs. 1-11) were compared to prepare a primer which specifically anneals to the *gyrB* gene of *Mycobacterium kansasii* (SEQUENCE NO. 3) and a primer which specifically anneals to that of *Mycobacterium gastri* (SEQUENCE NO. 5). A primer which anneals to the *gyrB* gene of both strains (SEQUENCE NO. 1) was also prepared.

Using these primers, PCR was carried out on disrupted cell suspensions of strains KPM 1004, KPM 1007, KPM KY256, KPM KY761, KPM KY768, KPM 1998-1, KPM 3502 and KPM 3503 isolated from clinical cases.

The PCR amplification conditions are as follows.
At 95°C for 10 minutes; 1 cycle
At 95°C for 1 minute and 68°C for 1 minute and 30 seconds; 30 cycles
At 72°C for 10 minutes; 1 cycle
Primer concentration; 1 µM for each
dNTP: 100 µM for each
Ampli Taq GOLD™ and PCR buffer I attached thereto (Perkin Elmer, USA) were used.

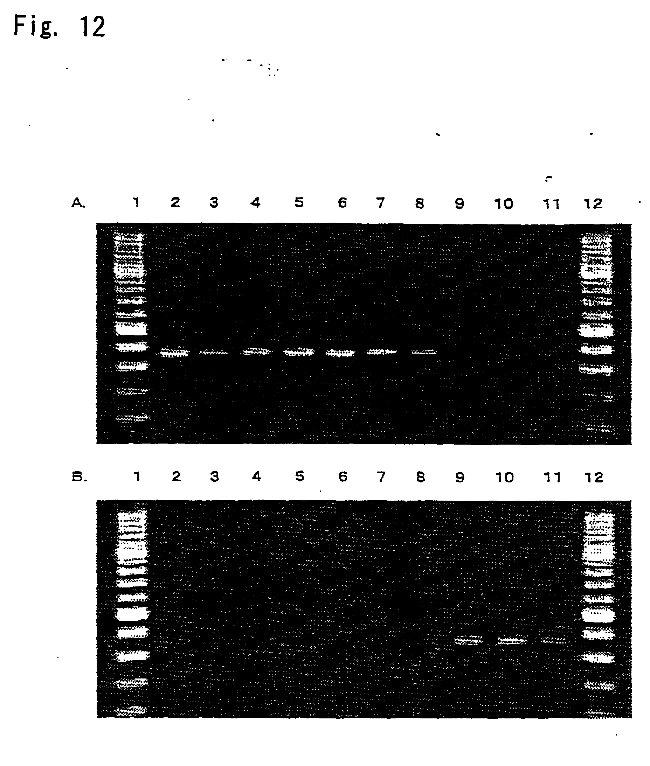

When the thus amplified DNA fragments were analyzed by an electrophoresis, amplified fragments were observed only by the combination of SEQUENCE NO. 1 and SEQUENCE NO. 3 in the case of KPM 1004, KPM 1007, KPM KY256, KPM KY761, KPM KY768 and KPM 1998-1 (Table 3), so that these strains were identified as *Mycobacterium kansasii*. In the case of KPM 3502 and KPM 3503, amplified fragments were observed only by the combination of SEQUENCE NO. 1 and SEQUENCE NO. 5 (Table 3), so that these strains were identified as *Mycobacterium gastri*. The electrophoresis patterns used in the judgment are as shown in Fig. 12. These identification results coincided with the identification results of the DNA-DNA hybridization method.

**TABLE 3**

| | *M. kansasii* | *M. gastri* |
|---|---|---|
| SEQUENCE NO. 1 | amplification was possible | No amplification |
| SEQUENCE NO. 3 | | |
| SEQUENCE NO. 1 | No amplification | amplification was possible |
| SEQUENCE NO. 5 | | |

### EXAMPLE 3

A 10 ng portion of purified DNA was prepared from each of 9 bacterial species including 4 tubercle bacilli group bacterial species and 5 other bacterial species belonging the genus *Mycobacterium*. PCR was carried out using these DNA samples as templates, and the oligonucleotides described in SEQUENCE NO. 41 and.SEQUENCE NO. 43 as primers. The PCR amplification conditions are as follows.
At 95°C for 10 minutes; 1 cycle
At 95°C for 1 minute, 68°C for 1 minute and 72°C for 1 minute; 30 cycles
At 72°C for 10 minutes; 1 cycle
Primer concentration; 1 µM for each
dNTP: 100 µM for each
Ampli Taq GOLD™ and PCR buffer 1 attached thereto (Perkin Elmer, USA) were used.

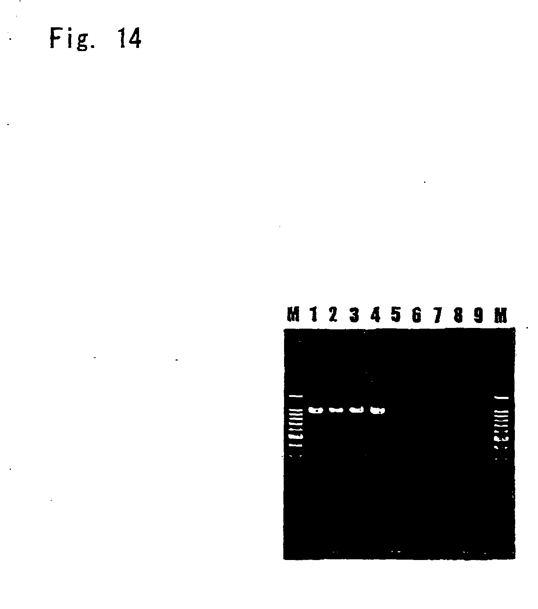

The products amplified by PCR were analyzed by an agarose gel electrophoresis. The results are shown in Fig. 14. In this connection, the relationship between lanes and bacterial species is as follows.
- Lane 1:: *Mycobacterium tuberculosis*
- Lane 2:: *Mycobacterium bovis*
- Lane 3:: *Mycobacterium africanum*
- Lane 4:: *Mycobacterium microti*
- Lane 5:: *Mycobacterium kansasii*
- Lane 6:: *Mycobacterium gastri*
- Lane 7:: *Mycobacterium abscessus*
- Lane 8:: *Mycobacterium chelonae*
- Lane 9:: *Mycobacterium trviale*

### EXAMPLE 4

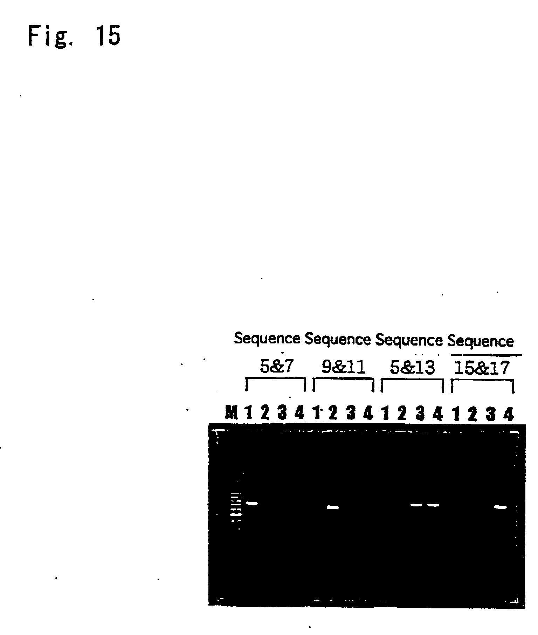

A 10 ng portion of purified DNA was prepared from each of 4 tubercle bacilli group bacterial species. PCR was carried out using these DNA samples as templates, and the oligonucleotides represented by SEQUENCE NO. 45, SEQUENCE NO. 47, SEQUENCE NO. 49, SEQUENCE NO. 51, SEQUENCE NO. 53, SEQUENCE NO. 55 and SEQUENCE NO. 57 as primers. The PCR amplification conditions are as described in Example 3. The products amplified by PCR were analyzed by an agarose gel electrophoresis. The results are shown in Fig. 15. In this case, the relationship between lanes and bacterial species is as follows.
- Lane 1:: *Mycobacterium tuberculosis*
- Lane 2:: *Mycobacterium bovis*
- Lane 3:: *Mycobacterium africanum*
- Lane 4:: *Mycobacterium microti*

As shown in Fig. 15, the amplified product was observed only by *Mycobacterium tuberculosis* when the oligonucleotides represented by SEQUENCE NO. 45 and SEQUENCE NO. 47 were used as primers, the amplified product was observed only by *Mycobacterium bovis* when the oligonucleotides represented by SEQUENCE NO. 49 and SEQUENCE NO. 51 were used as primers, the amplified product was observed by *Mycobacterium africanum* and *Mycobacterium microti* when the oligonucleotides represented by SEQUENCE NO. 45 and SEQUENCE NO. 53 were used as primers and the amplified product was observed by only *Mycobacterium microti* when the oligonucleotides represented by SEQUENCE NO. 55 and SEQUENCE NO. 57 were used as primers. Based on the above results, relationship between primers and bacterial species can be summarized as follows.

**TABLE 4**

| SEQUENCE No. | *Mycobacterium tuberculosis* | *Mycobacterium bovis* | *Mycobacterium africanum* | *Mycobacterium microti* |
|---|---|---|---|---|
| SEQ. NO. 41 | Amplification possible | Amplification possible | Amplification possible | Amplification possible |
| SEQ. NO. 43 | | | | |
| SEQ. NO. 45 | Amplification possible | No amplification | No amplification | No amplification |
| SEQ. NO. 47 | | | | |
| SEQ. NO. 49 | No amplification | Amplification possible | No amplification | No amplification |
| SEQ. NO. 51 | | | | |
| SEQ. NO. 45 | No amplification | No amplification | Amplification possible | Amplification possible |
| SEQ. NO. 53 | | | | |
| SEQ. NO. 55 | No amplification | no amplification | no amplification | Amplification possible |
| SEQ. NO. 57 | | | | |

### EXAMPLE 5

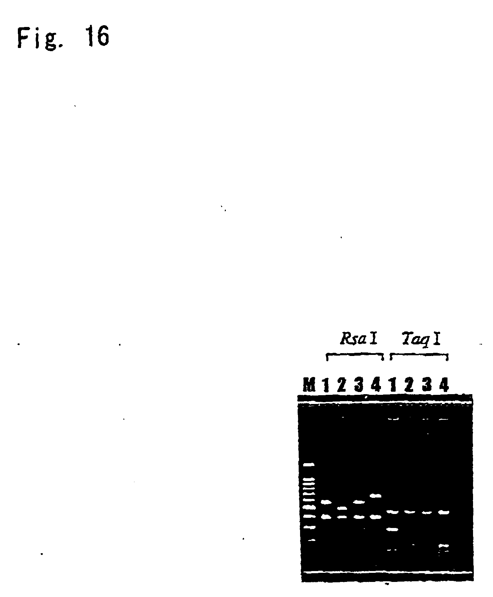

A 10 ng portion of purified DNA was prepared from each of 4 tubercle bacilli group bacterial species. PCR was carried out using these DNA samples as templates, and the oligonucleotides represented by SEQUENCE NO. 41 and SEQUENCE NO. 43 as primers. The PCR amplification conditions are as described in Example 3. The products amplified by PCR were digested with restriction enzymes *Rsa* I and *Taq* 1, and the thus formed DNA fragments were analyzed by an agarose gel electrophoresis. The results are shown in Fig. 16. In this connection, the relationship between lanes and bacterial species is as follows.
- Lane 1:: *Mycobacterium tuberculosis*
- Lane 2:: *Mycobacterium bovis*
- Lane 3:: *Mycobacterium africanum*
- Lane 4:: *Mycobacterium microti*

### EXAMPLE 6

Using the oligonucleotides represented by SEQUENCE NO. 1, SEQUENCE NO. 43, SEQUENCE NO. 45, SEQUENCE NO. 47, SEQUENCE NO. 49, SEQUENCE NO. 51, SEQUENCE NO. 53, SEQUENCE NO. 55 and SEQUENCE NO. 57 as primers, PCR was carried out on a solution of disrupted cells of a strain KPM KY631 isolated from a clinical patient of tuberculosis. When the product amplified by PCR was analyzed by an agarose gel electrophoresis, the amplified product was observed only by the combination of SEQUENCE NO. 1 and SEQUENCE NO. 43 and of SEQUENCE NO. 45 and SEQUENCE NO. 47, so that the strain KPM KY631 was identified as the tubercle *Mycobacterium tuberculosis* (Table 4 and Fig. 15).

### EXAMPLE 7

Using the oligonucleotides represented by SEQUENCE NO. 41 and SEQUENCE NO. 43 as primers, PCR was carried out on a solution of disrupted cells of a strain KPM KY590 isolated from a clinical patient of tuberculosis. When nucleotide sequence of the thus amplified DNA fragment was determined, the thus obtained nucleotide sequence coincided with the nucleotide sequence of the tubercle *Mycobacterium tuberculosis*, so that the strain KPM KY590 was identified as the tubercle *Mycobacterium tuberculosis* (Figs. 1-11).

### EXAMPLE 8

Using the oligonucleotides represented by SEQUENCE NO. 41 and SEQUENCE NO. 43 as primers, PCR was carried out on a solution of disrupted cells of a strain isolated from a bovine patient of tuberculosis. When the product amplified by PCR was digested with restriction enzymes *Rsa* I and *Taq* I and the thus formed DNA fragments were analyzed by an agarose gel electrophoresis, the result coincided with the pattern obtained from *Mycobacterium bovis*, so that this strain was identified as *Mycobacterium bovis* (Fig. 16).

The present invention realizes accurate classification and identification of slow growing mycobacteria which are difficult to identify by conventional methods. It also renders possible quick identification of certain species of atypical mycobacteria, such as *Mycobacterium kansasii* and *Mycobacterium gastri*, which are difficult to distinguish by the identification method based on 16S rRNA gene sequence. The present invention is useful in the fields of medical science, immunology, veterinary science, etc.

## Claims

1. A method for identifying a slow growing mycobacteria species, which comprises amplifying the regions corresponding to SEQUENCE NO. 7, 9, 11, 13, 15, 17, 19, 21, 23, 25, 27, 29, 31, 33, 35, 37 and 39 in the DNA which encodes DNA gyrase β subunit of a slow growing mycobacteria in a sample, determining and comparing the nucleotide sequence of the amplified fragment with the nucleotide sequences described in SEQUENCE NO. 7, 9, 11, 13, 15, 17, 19, 21, 23, 25, 27, 29, 31, 33, 35, 37 and 39, thereby calculating a genetic distance of the sequence of the amplified fragment from each sequence, and identifying the species of the slow growing mycobacteria in a sample based on the genetic distance.

2. A method for detecting *Mycobacterium kansasii*, which comprises detecting *Mycobacterium kansasii* using, as a primer or probe, an oligonucleotide which comprises a sequence coding for a part of or the entire portion of the amino acid sequence described in SEQUENCE NO. 4, or a complementary sequence thereof, and substantially functions as a primer or probe.

3. A *Mycobacterium kansasii* detection kit which comprises an oligonucleotide which comprises a sequence coding for a part of or the entire portion of the amino acid sequence described in SEQUENCE NO. 4, or a complementary sequence thereof, and substantially functions as a primer or probe.

4. A method for detecting *Mycobacterium gastri*, which comprises detecting *Mycobacterium gastri* using, as a primer or probe, an oligonucleotide which comprises a sequence coding for a part of or the entire portion of the amino acid sequence described in SEQUENCE NO. 6, or a complementary sequence thereof, and substantially functions as a primer or probe.

5. A *Mycobacterium gastri* detection kit which comprises an oligonucleotide which comprises a sequence coding for a part of or the entire portion of the amino acid sequence described in SEQUENCE NO. 6, or its complementary sequence, and substantially functions as a primer or probe.

6. A method for identifying a slow growing mycobacteria species, which comprises
checking, in DNA of a sample, the existence of a DNA comprising a unique region having a different nucleotide sequence in the DNA sequence coding for DNA gyrase β subunit among slow growing mycobacteria,
identifying a bacterium in a sample based on the existence of said DNA as a marker.

7. The method for identifying a slow growing mycobacteria species according to claim 6, wherein the slow growing mycobacteria are *Mycobacterium simiae*, *Mycobacterium bovis, Mycobacterium szulgai, Mycobacterium malmoense, Mycobacterium intracellulare, Mycobacterium* avium, *Mycobacterium gordonae, Mycobacterium africanum, Mycobacterium tuberculosis, Mycobacterium gastri, Mycobacterium marinum, Mycobacterium microti, Mycobacterium asiaticum, Mycobacterium scrofulaceum, Mycobacterium branderi, Mycobacterium paratuberculosis,* and *Mycobacterium kansasii*.

8. The method for identifying a slow growing mycobacteria species according to claim 6, which comprises the following steps (1) to (4):
(1) synthesizing an oligonucleotide which comprises a unique region having a different nucleotide sequence in the DNA sequence coding for DNA gyrase β subunit among slow growing mycobacteria,
(2) preparing a solution which comprises the oligonucleotide synthesized in the step (1), dNTP, DNA polymerase and a bacterial DNA in a sample,
(3) heating the solution prepared in the step (2) repeatedly under such conditions that polymerase chain reaction can occur, and
(4) subjecting the solution obtained in the step (3) to electrophoresis to identify the species of the bacterium in a sample based on the electrophoresis pattern.

9. The method for identifying a slow growing mycobacteria species according to claim 8, wherein the oligonucleotide is an oligonucleotide which encodes the amino acid sequence described in SEQUENCE NO. 46, SEQUENCE NO. 48, SEQUENCE NO. 50, SEQUENCE NO. 52, SEQUENCE NO. 54, SEQUENCE NO. 56 or SEQUENCE NO. 58.

10. The method for identifying a slow growing mycobacteria species according to claim 8, wherein the oligonucleotide is an oligonucleotide represented by SEQUENCE NO. 45, SEQUENCE NO. 47, SEQUENCE NO. 49, SEQUENCE NO. 51, SEQUENCE NO. 53, SEQUENCE NO. 55 or SEQUENCE NO. 57.

11. The method for identifying a slow growing mycobacteria species according to claim 6, which comprises the following steps (1) to (4):
(1) synthesizing a first oligonucleotide which is identical to a first partial sequence in the DNA sequence coding for the DNA gyrase β subunit of slow growing mycobacteria and a second oligonucleotide which is complementary to a second partial sequence in the DNA sequence coding for the DNA gyrase β subunit of slow growing mycobacteria, said first partial sequence and said second partial sequence being respectively conserved among slow growing mycobacteria,
(2) subjecting the two oligonucleotides synthesized in the step (1) as primers and a bacterial DNA sample as a template to the polymerase chain reaction,
(3) mixing the DNA fragment amplified in the step (2) with a restriction enzyme under the conditions at which the restriction enzyme is active, said restriction enzyme recognizing the sequence unique to one or more slow growing mycobacteria, and
(4) subjecting the mixture obtained in the step (3) to electrophoresis to identify the species of the bacterium in a sample based on the electrophoresis pattern.

12. The method for identifying a slow growing mycobacteria species according to claim 11, wherein the two oligonucleotides to be used as primers are oligonucleotides represented by SEQUENCE NO. 1 and SEQUENCE NO. 3, and the restriction enzymes to be used are *Rsa* I and *Taq* I.

13. An identification kit for a slow growing mycobacteria species, which comprises an oligonucleotide containing a region of DNA coding for DNA gyrase β subunit, a region having different nucleotide sequence among slow growing mycobacteria.

14. An identification kit for a slow growing mycobacteria species, which comprises
a first oligonucleotide which is identical to a first partial sequence in the DNA sequence coding for the DNA gyrase β subunit of slow growing mycobacteria,
a second oligonucleotide which is complementary to a second partial sequence in the DNA sequence coding for the DNA gyrase β subunit of slow growing mycobacteria, said first partial sequence and said second partial sequence being respectively conserved among slow growing mycobacteria, and
one or more restriction enzyme recognizing the sequence unique to one or more slow growing mycobacteria.

15. A method for detecting a slow growing mycobacteria species, which comprises
checking, in DNA of a sample, the existence of a DNA comprising a unique region having a different nucleotide sequence in the DNA sequence coding for DNA gyrase β subunit among slow growing mycobacteria,
detecting a bacterium in a sample based on the existence of said DNA as a marker.

16. The method for detecting a slow growing mycobacteria species according to claim 15, wherein the slow growing mycobacteria are *Mycobacterium simiae*, *Mycobacterium bovis*, *Mycobacterium szulgai*, *Mycobacterium malmoense, Mycobacterium intracellulare, Mycobacterium avium, Mycobacterium gordonae, Mycobacterium africanum, Mycobacterium tuberculosis, Mycobacterium gastri, Mycobacterium marinum, Mycobacterium microti, Mycobacterium asiaticum, Mycobacterium scrofulaceum, Mycobacterium branderi, Mycobacterium paratuberculosis,* and *Mycobacterium kansasii*.

17. The method for detecting a slow growing mycobacteria species according to claim 15, which comprises the following steps (1) to (4):
(1) synthesizing an oligonucleotide which comprises a unique region having a different nucleotide sequence in the DNA sequence coding for DNA gyrase β subunit among slow growing mycobacteria,
(2) preparing a solution which comprises the oligonucleotide synthesized in the step (1), dNTP, DNA polymerase and a bacterial DNA in a sample,
(3) heating the solution prepared in the step (2) repeatedly under such conditions that polymerase chain reaction can occur, and
(4) subjecting the solution obtained in the step (3) to electrophoresis to detecting the bacterium in a sample based on the electrophoresis pattern.

18. The method for detecting a slow growing mycobacteria species according to claim 17, wherein the oligonucleotide is an oligonucleotide which encodes the amino acid sequence described in SEQUENCE NO. 46, SEQUENCE NO. 48, SEQUENCE NO. 50, SEQUENCE NO. 52, SEQUENCE NO. 54, SEQUENCE NO. 56 or SEQUENCE NO. 58.

19. The method for detecting a slow growing mycobacteria species according to claim 17, wherein the oligonucleotide is an oligonucleotide represented by SEQUENCE NO. 45, SEQUENCE NO. 47, SEQUENCE NO. 49, SEQUENCE NO. 51, SEQUENCE NO. 53, SEQUENCE NO. 55 or SEQUENCE NO. 57.

20. The method for detecting a slow growing mycobacteria species according to claim 15, which comprises the following steps (1) to (4):
(1) synthesizing a first oligonucleotide which is identical to a first partial sequence in the DNA sequence coding for the DNA gyrase β subunit of slow growing mycobacteria and a second oligonucleotide which is complementary to a second partial sequence in the DNA sequence coding for the DNA gyrase β subunit of slow growing mycobacteria, said first partial sequence and said second partial sequence being respectively conserved among slow growing mycobacteria,
(2) subjecting the two oligonucleotides synthesized in the step (1) as primers and a bacterial DNA sample as a template to the polymerase chain reaction,
(3) mixing the DNA fragment amplified in the step (2) with a restriction enzyme under the conditions at which the restriction enzyme is active, said restriction enzyme recognizing the sequence unique to one or more slow growing mycobacteria, and
(4) subjecting the mixture obtained in the step (3) to electrophoresis to detect the bacterium in a sample based on the electrophoresis pattern.

21. The method for detecting a slow growing mycobacteria species according to claim 20, wherein the two oligonucleotides to be used as primers are oligonucleotides represented by SEQUENCE NO. 1 and SEQUENCE NO. 3, and the restriction enzymes to be used are *Rsa* I and *Taq* I.

22. A detection kit for a slow growing mycobacteria species, which comprises an oligonucleotide containing a region of DNA coding for DNA gyrase β subunit, a region having different nucleotide sequence among slow growing mycobacteria.

23. A detection kit for a slow growing mycobacteria species, which comprises
a first oligonucleotide which is identical to a first partial sequence in the DNA sequence coding for the DNA gyrase β subunit of slow growing mycobacteria,
a second oligonucleotide which is complementary to a second partial sequence in the DNA sequence coding for the DNA gyrase β subunit of slow growing mycobacteria, said first partial sequence and said second partial sequence being respectively conserved among slow growing mycobacteria, and
one or more restriction enzyme recognizing the sequence unique to one or more slow growing mycobacteria.

24. A method for identifying a slow growing mycobacteria species, which comprises the following steps (1) to (4):
(1) synthesizing an oligonucleotide which comprises a sequence corresponding to a region in the DNA gyrase β subunit wherein the 3'-side nearest neighbor base to said region in the DNA gyrase β subunit is a unique base among the slow glowing mycobacteria,
(2) preparing a solution which comprises the oligonucleotide synthesized in the step (1), a labeled ddNTP, DNA polymerase, and a bacterial DNA in a sample,
(3) heating the solution prepared in the step (2) under such conditions that reaction between the labeled ddNTP and the oligonucleotide occurs,
(4) checking the existence of the labeled oligonucleotide, and
(5) identifying a bacterium in a sample based on the existence of the labeled oligonucleotide.

25. A method for detecting a slow growing mycobacteria species, which comprises the following steps (1) to (4):
(1) synthesizing an oligonucleotide which comprises a sequence corresponding to a region in the DNA gyrase β subunit wherein the 3'-side nearest neighbor base to said region in the DNA gyrase β subunit is a unique base among the slow glowing mycobacteria,
(2) preparing a solution which comprises the oligonucleotide synthesized in the step (1), a labeled ddNTP, DNA polymerase, and a bacterial DNA in a sample,
(3) heating the solution prepared in the step (2) under such conditions that reaction between the labeled ddNTP and the oligonucleotide occurs,
(4) checking the existence of the labeled oligonucleotide, and
(5) detecting a bacterium in a sample based on the existence of the labeled oligonucleotide.
